Europäisches Patentamt

European Patent Office

Office européen des brevets

(19)

(11) Publication number : **0 577 243 A2**

(12) **EUROPEAN PATENT APPLICATION**

(21) Application number : **93302546.2**

(22) Date of filing : **31.03.93**

(51) Int. Cl.$^5$ : **C12N 15/13, C12P 21/08, C12N 5/10, A61K 39/395**

The applicant has subsequently filed a sequence listing and declared, that it includes no new matter.

(30) Priority : **01.04.92 US 861701**

(43) Date of publication of application :
**05.01.94 Bulletin 94/01**

(84) Designated Contracting States :
**AT BE CH DE DK ES FR GB GR IE IT LI LU NL PT SE**

(71) Applicant : **MERCK & CO. INC.**
**126, East Lincoln Avenue P.O. Box 2000 Rahway New Jersey 07065-0900 (US)**

(72) Inventor : **Emini, Emilio A.**
**6 Faggs Manor Lane**
**Paoli, PA 19301 (US)**
Inventor : **Conley, Anthony J.**
**231 Biddle Drive**
**Exton, PA 19341 (US)**
Inventor : **Mark, George E.**
**4 Richmond Court**
**Princeton Junction, NJ 08550 (US)**
Inventor : **Johnson, L. Syd**
**13545 Ambassador Drive**
**Gemantown, MD 20874 (US)**
Inventor : **Pfarr, David S.**
**21 Carriage Walk Court**
**Gaithersburg, MD 20879 (US)**

(74) Representative : **Quillin, Helen Kaye et al**
**European Patent Department, Merck & Co., Inc., Terlings Park, Eastwick Road Harlow, Essex CM20 2QR (GB)**

(54) **Recombinant human HIV-neutralizing monoclonal antibodies for prevention and treatment of HIV infection.**

(57)    Recombinant human immunoglobulin molecules neutralizing for HIV-1, methods of production of the immunoglobulins and methods of preventing HIV-1 infection using the immunoglobulins are disclosed. DNA constructs containing the complementarity determining regions (CDRs) and framework (FRs) of the native human antibody are combined with other constant regions, and expressed in recombinant host cells. These recombinant antibodies are useful for the treatment and prevention of HIV-1 infection in vivo.

EP 0 577 243 A2

FIG.8

INTRODUCTION

The gp120 V3 domain has been shown to be a disulfied-linked closed loop of approximately 30 amino acid residues [Leonard et al., (1990), J. Biol. Chem., 265, pp.10373-82]. The loop, either in the context of intact gp120 or as a synthetic peptide fragment, binds and elicits anti-HIV 1 type-specific virus-neutralizing antibodies [Goudsmit et al., (1988), AIDS, 2, pp.157-164; Goudsmit et al., (1988), Proc. Natl. Acad. Sci. USA, 85, pp.4478-4482; Ho et al., (1987), J. Virol., 61, pp.2024-2028; Javaherian et al., (1989), Proc. Natl. Acad. Sci. USA, 86, pp.6768-6772; Kenealy et al., (1989), AIDS Res., 5, pp.173-182; Rusche et al., (1988), Proc. Natl. Acad. Sci. USA, 85, pp.3198-3202]. Accordingly, the V3 domain has been termed the principal neutralization determinant. The in vitro characteristics of anti-V3 loop antibody include relatively potent virus-neutralizing activity, ability to neutralize following binding of the virus to the host cell CD4 receptor and ability to prevent fusion of virus-infected and uninfected cells [Linsley et al., (1988), J. Virol., 62, pp.3695-3702; Skinner et al., (1988), J. Virol., 62, pp.4195-4200]. Berman et al. immunized chimpanzees with recombinantly expressed mammalian cell-derived gp120 or its gp160 precursor [Berman et al., (1990), Nature, 345, pp.622-625]. Upon virus challenge of the animals, protection from infection was noted solely in those chimpanzees that had been inoculated with gp120. The only measured immune response that correlated with the protection was anti-V3 loop antibody. Girard et al. inoculated several chimpanzees with a series of immunogens, the last of which were V3 loop-specific synthetic immunogens [Girard et al., (1991), Proc. Natl. Acad. Sci. USA, 88, pp.542-546]. Significant virus-neutralizing activity was elicited only after this final inoculation. Upon challenge, the chimpanzees were either completely protected or exhibited delayed infection. Finally, Emini et al. reported an in vitro neutralization of chimpanzee infectivity study in which protection from or delay of infection also correlated with the presence of anti-V3 loop virus-neutralizing antibody [Emini et al., (1990), J. Virol., 64, pp.3647-3678].

Recently, Ward et al. showed that a chimeric molecule composed of the Fc region of human IgG and the viral binding domain of the CD4 virus receptor could also prevent HIV-1 infection of chimpanzees if administered prior to virus inoculation [Ward et al., (1991), Nature, 352, pp.434-436]. However, in contrast to anti-V3 loop antibody, agents that block virus-CD4 binding are weak neutralizers of in vitro HIV-1 infectivity and their effects are easily diminished by alterations of gp120-CD4 binding affinity [Layne et al., (1991), J. Virol., 65, pp.3293-3300; Kang et al., (1991), Proc. Natl. Acad. Sci. USA, 88, pp.6171-6175; Thali et al., (1991), J. Virol., 65, pp.5007-5012].

Emini et al. have recently shown that a mouse/human chimaeric monoclonal antibody specific for a single HIV-1 strain (IIIb) could prevent HIV-1 IIIb infection in chimpanzees both pre- and post-exposure to the virus [Emini et al., (1992), Nature, 355, pp.728-730].

It is a purpose of the present invention therefore, to provide novel recombinant human immunoglobulins which neutralize HIV-1 and to provide novel recombinant human FAb and Fv portions of HIV-1 neutralizing human immunoglobulin. The present invention also provides novel DNA sequences for human HIV-1 neutralizing immunoglobulins and provides expression vectors containing the novel immunoglobulin DNA sequences. Recombinant host cells containing the expression vectors with the novel immunoglobulin DNA sequences and recombinant HIV-1 neutralizing human immunoglobulin with modified human constant regions are provided. Also disclosed is a method of preventing HIV-1 infection by administration of recombinant HIV-1 neutralizing human immunoglobulins and methods of treatment of HIV-1 infection by administration of recombinant HIV-1 neutralizing human immunoglobulins.

SUMMARY OF THE INVENTION

Recombinant HIV-1-neutralizing human immunoglobulins and methods of cloning and expressing the recombinant immunoglobulins in host cells are disclosed. DNA encoding the HIV-1-neutralizing human immunoglobulins or complementarity determining regions (CDR) of the immunoglobulin recombinantly fused to human constant encoding DNA, is cloned and into expression vectors and expressed in recombinant host cells.

BRIEF DESCRIPTION OF THE DRAWINGS

Figure 1      shows the DNA oligonucleotide primers representing sequences within the DNA encoding the heavy chain and light chain of the antibody, used for recombinant cloning.

Figure 2A&B      displays the complete nucleotide sequence of the heavy chain of the 447-52D antibody, and 2B displays the complete nucleotide sequence of the light chain of the 447-52D antibody, as produced by the recombinant vectors.

Figure 3      shows the DNA oligonucleotide primers used to construct the variable domains for the heavy and light chains of the 447-52D antibody.

| Figure 4 | is a schematic diagram of the recombinant cloning procedure for the variable regions of the heavy and light chains of antibody 447-52D. |
| Figure 5 | is a schematic diagram of plasmid pHIV/447VH/Cγl containing the human gamma 1 heavy chain of the recombinant antibody. |
| Figure 6 | is a schematic diagram of the plasmid pHIV/447Vλ/Cλ containing the light chain of the recombinant antibody. |
| Figure 7 | is a schematic diagram of the plasmid p63.79r447 containing both the light chain and human gamma 1 heavy chain. |
| Figure 8 | is a schematic diagram of the cloning strategy used to produce p63.79r447. |

## DETAILED DESCRIPTION OF THE INVENTION

The present invention relates to HIV-1 neutralizing human immunoglobulins and the recombinant construction and expression of unique immunoglobulins. The unique recombinant immunoglobulins contain the HIV-1 neutralizing complementarity determining regions (CDR). The recombinant HIV-1 neutralizing CDR containing immunoglobulins can be recombinantly modified to contain heavy and/or light chain framework regions different from the original native immunoglobulin.

The recombinantly modified immunoglobulin can for example, be converted to the IgG1 isotype from any of the other IgG isotypes or other classes of immunoglobulin.

The present invention further comprises a method of preventing HIV infection by administration of the recombinant HIV-1 neutralizing immunoglobulin either prior to exposure to HIV-1 or after exposure to HIV-1. The invention further includes the treatment of HIV-1 infection by administration of the recombinant HIV-1 neutralizing immunoglobulin.

The present invention further comprises a method for constructing and expressing the altered antibody comprising: (i) mutagenesis and assembly of variable region domains including CDRs and framework (FRs) regions; (ii) preparation of an expression vector including at least one variable region which upon transfection into cells results in the secretion of protein sufficient for avidity and specificity determinations; and (iii) co-amplification of heavy and light chain expression vectors in appropriate cell lines.

The present invention also provides recombinant methods for incorporating CDRs from animal monoclonal antibodies into human immunoglobulin frameworks so that the resulting recombinant human antibody will be either weakly immunogenic or non-immunogenic when administered to humans. Preferrably the recombinant immunoglobulins will be recognized as self proteins when administered for threapeutic purposes. This method of "humanization" will render the recombinant antibodies useful as therapeutic agents because they will be either weakly immunogenic or non-immunogenic when administered to humans. The invention is further contemplated to include the recombinant conversion of any animal monoclonal antibody into a recombinant human monoclonal antibody providing that a suitable framework region can be identified (as described below). It is intended that the present invention include the nucleotide and amino acid sequences of human and animal CDR regions and the human framework regions either separately or combined as a light or heavy chain or an intact immunoglobulin and any conservatively modified variants thereof. The animal monoclonals may include, but are not limited to, those murine monoclonal antibodies which bind to HIV-1 and the appropriate mMAbs produced by hybridomas deposited in the Hybridoma Cell Bank maintained by the American Type Culture Collection (ATCC) and described in the ATCC Catalog of Cell Lines & Hybridomas, No. 6, 1988.

As used herein, all amino acid three letter and single letter designations conform to those designations which are standard in the art, and are listed as follows:

3

| Alanine | Ala | A | Leucine | Leu | L |
|---|---|---|---|---|---|
| Arginine | Arg | R | Lysine | Lys | K |
| Asparagine | Asn | N | Methionine | Met | M |
| Aspartic acid | Asp | D | Phenylalanine | Phe | F |
| Cysteine | Cys | C | Proline | Pro | P |
| Glutamic acid | Glu | E | Serine | Ser | S |
| Glutamine | Gln | Q | Threonine | Thr | T |
| Glycine | Gly | G | Tryptophan | Trp | W |
| Histidine | His | H | Tyrosine | Tyr | Y |
| Isoleucine | Ile | I | Valine | Val | V |

The present invention relates to methods and means for the construction and expression of a unique antibody capable of neutralizing HIV-1. Human blood specimens donated from HIV-1 positive individuals were the source of peripheral B cells expressing neutralizing antibodies. These cells were immortalized by Epstein-Barr virus (EBV) infection, then individual B cell clones were screened for their ability to secrete antibody which bound a peptide sequence representing the V3 loop of HIV-1 strain MN in a solid phase ELISA format. B cell clones positive in this assay were subsequently stabilized by their fusion to the SHM-D33 cell line (a murine x human heterohybridoma, ATCC CRL 1668). Resultant B cell-heterohybridoma clones were screened for their production of antibody which recognizes the MN V3 loop peptide in a solid-phase ELISA. These procedures establish the criteria for identification and isolation of stable human antibody-producing cells wherein the antibody produced is potentially useful for development into a substance for treatment prophylatically in cases of suspected HIV-1 exposure, and therapeutically in HIV-1 positive individuals.

Any of a variety of procedures may be used to molecularly clone anti HIV-1 antibody encoding DNA. These methods include, but are not limited to, direct functional expression of the antibody gene following the construction of an antibody containing cDNA library in an appropriate expression vector system. Another method is to screen an antibody-containing cDNA library constructed in a bacteriophage or plasmid shuttle vector with a labelled oligonucleotide probe designed from the amino acid sequence of the antibody fragments.

It is readily apparent to those skilled in the art that other types of libraries, as well as libraries constructed from other cells or cell types, may be useful for isolating anti-HIV-1 antibody-encoding DNA. Other types of libraries include, but are not limited to, cDNA libraries derived from other B-cells or B-cell lines other than the 447 cells, and genomic DNA libraries.

It is readily apparent to those skilled in the art that suitable cDNA libraries may be prepared from cells or cell lines which produce anti-HIV-1 antibody. The selection of cells or cell lines for use in preparing a cDNA library to isolate anti-HIV-1 antibody done by first measuring cell produced anti-HIV-1 antibody using the procedures described fully above and below.

Preparation of cDNA libraries can be performed by standard techniques well known in the art. Well known cDNA library construction techniques can be found for example, in Maniatis, T., Fritsch, E.F., Sambrook, J., Molecular Cloning: A Laboratory Manual (Cold Spring Harbor Laboratory, Cold Spring Harbor, New York, 1982).

It is also readily apparent to those skilled in the art that DNA encoding anti-HIV-1 antibody may also be isolated from a suitable genomic DNA library.

Construction of genomic DNA libraries can be performed by standard techniques well known in the art. Well known genomic DNA library construction techniques can be found in Maniatis, T., Fritsch, E.F., Sambrook, J., Molecular Cloning: A Laboratory Manual (Cold Spring Harbor Laboratory, Cold Spring Harbor, New York, 1982).

The following procedures are preferred to prepare recombinant DNA sequences which incorporate the antibody variable regions, light chains and heavy chains obtained from human B cell lines described above, combined with human constant regions. These recombinant DNAs can be used to transfect mammalian cells for the expression of a recombinant human antibody which retains the antigen specificity of the human-donor B cell-derived antibody. Preferably, the recombinant immunoglobulins will be recognized as self proteins when administered for therapeutic purposes. Total RNA is extracted from the human heterohybridomas, for example the human heterohybridoma cells described, using standard methods, for example involving cellular solubilization with guanidinium isothiocyanate (Chirgwin et al., Biochem. 18: 5294-5299 [1979]).

It is readily apparent to those skilled in the art that other anti-HIV-1 antibody producing cells are suitable for the preparation of recombinant DNA molecules encoding part or all of the anti-HIV-1 antibody molucule. Such anti-HIV-1 producing cells include, but are not limited to those described in Gorney M.K. et al., Proc. Natl. Acad. Sci. (USA) 88:3238-3242 (1991); Robinson J.E. et al., AIDS Res. Hum. Retrovir. 6:567-579 (1990); Posner M.R. et al., J. Immunol 146:5325-4331 (1991); Ho D.D. et al., J. Virol. 65:489-493 (1991); Tilley, S.A. et al., Research Virol. 142:247-259 (1991) and in the ATCC Catalogue of Cell Lines And Hybridomas, 7th Edition, 1992. The 447-52D heterohybridoma described below will be used as the primary example of the unique process being disclosed.

It is further intended and readily apparent to those skilled in the art that human immunoglobulin (Ig) can contain either kappa or lambda light chains or be one of any of the following heavy chain isotypes: alpha (IgA), delta (IgD), epislon (IgE), gamma (IgG) and mu (IgM).

The 5 different antibody heavy chain isotypes impart different properties to the whole antibody molecules. For example, IgA is the major class of antibody in bodily secretions (milk, saliva, tears and respiratory and intestinal secretions) and is commonly found on the surface of epithelial cells lining the intestines, bronchi, reproductive tract, or the mammary, salivary, and tear ducts. IgM is the major class of antibody secreted into the blood during the early stages of a primary antibody response to antigen, and is multivalent usually having a total of 10 antigen binding sites. IgE has a high affinity for receptors on most cells and basophylic leukocytes, and is typically involved in allergic reactions. IgD is found on he surface of resting B cells, and its function is unclear. IgG constitutes the major class of immunoglobulin in the blood, are copiously produced in secondary immune responses, the Fc region can bind to phagocytic cells aiding phgocytosis, the Fc region can activate the compliment system (as can IgM), and is the only antibody which can traverse the placenta and enter the bloodstream of a fetus.

The normal human immunoglobulin class G contains 4 subclasses with different biochemical characteristics. Normal IgG consists of about 70% IgG1, 18% IgG2, 8% IgG3 and 3% IgG4. The route and duration of antigen exposure, type of antigen, and genetic background may all affect the subclass of IgG anibody produced. As the major class of immunoglobulin, the role of IgG and IgG subclasses both in immunity and in disease is of considerable importance.

It may be desirable to produce a recombinant anti-HIV antibody through recombinant DNA techniques which has a different heavy chain class or subclass than the native antibody. For example, the native antibody may be an IgM (or other immunoglobulin class) which is less desirable than an IgA for the purpose of producing a recombinant antibody molecule which will be secreted into the respiratory, intestinal or reproductive tract. Through recombinant DNA techniques that are known in the art the IgM heavy chain constant region may be replaced with an IgA heavy chain and recombinantly expressed. In addition an IgG3 native antibody may be recombinant altered to an IgG1 antibody where it is desirable to recombinantly produce an antibody with greater complimnt system activation properties.

The conversion of an antibody molecule from one IgG subclass to another IgG subclass through recombinant DNA techniques is demonstrated in the present invention using the anti-HIV antibody 447-52D. 447-52D in its native form is an IgG3, which was converted ino an IgG1 through the following recombinant DNA procedures. It is readily apparent to those of ordinary skill in the art that the recombinant DNA procedures used herein are applicable for converting other immunoglobulin heavy chain classes other han IgG, and for converting one IgG subclass other than IgG1.

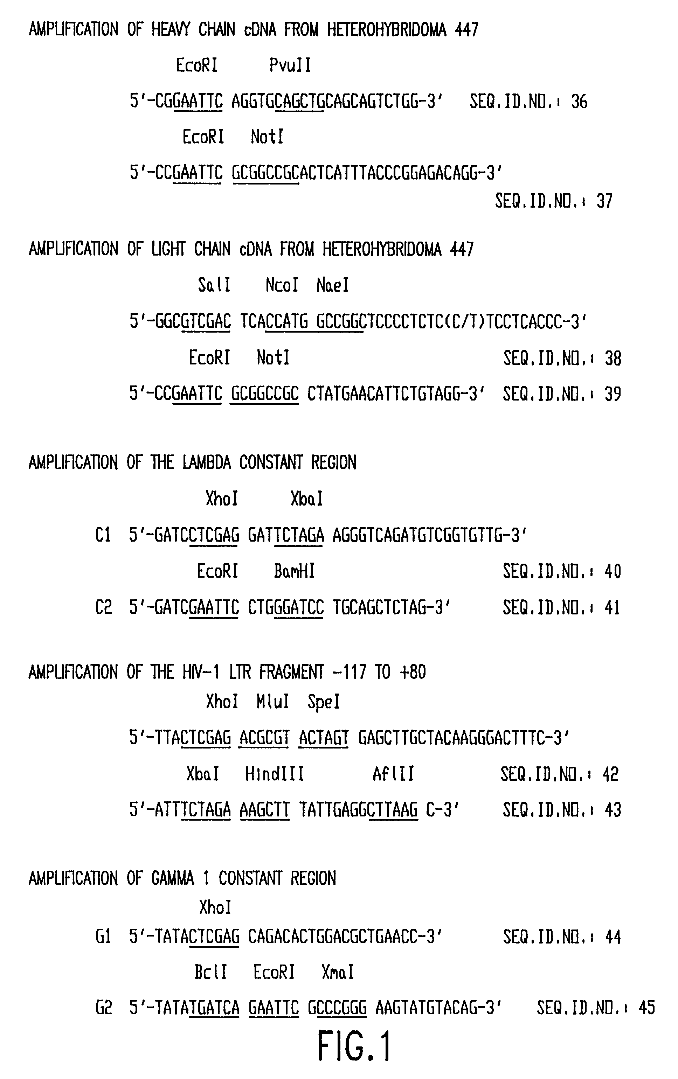

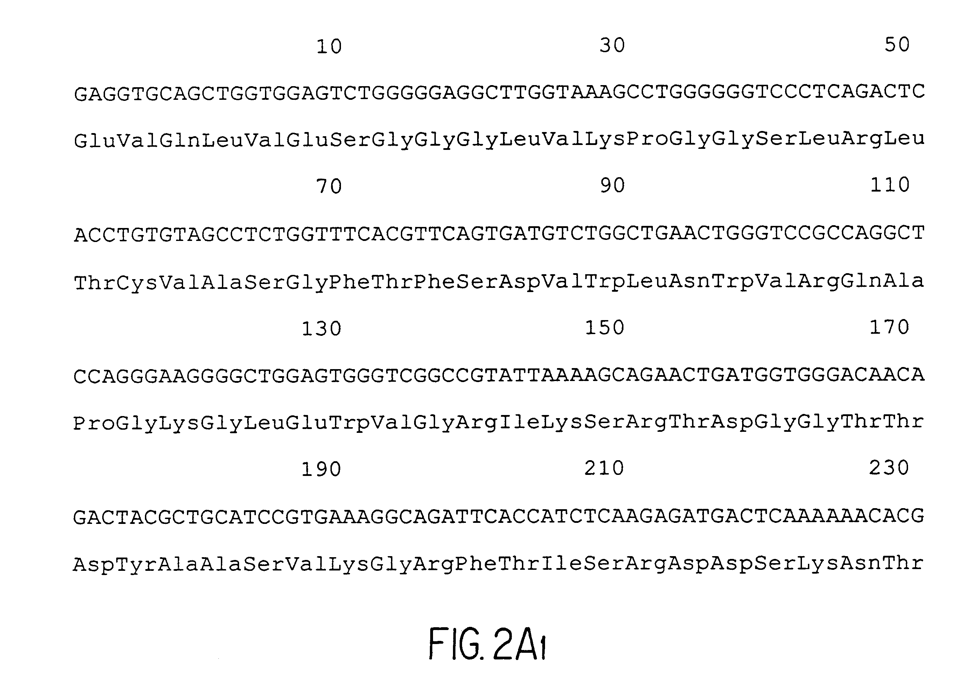

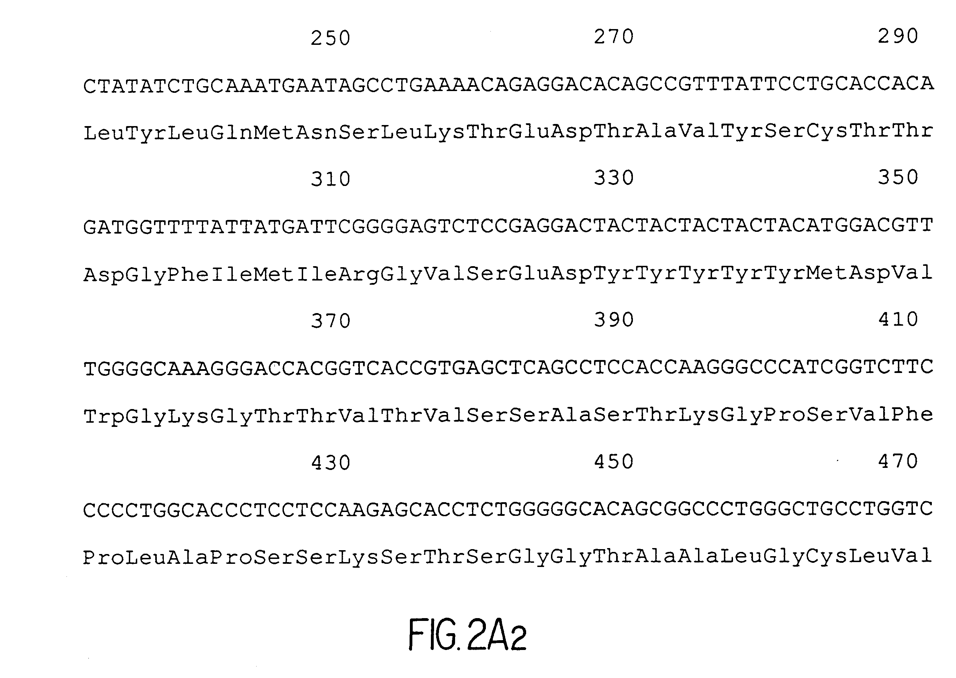

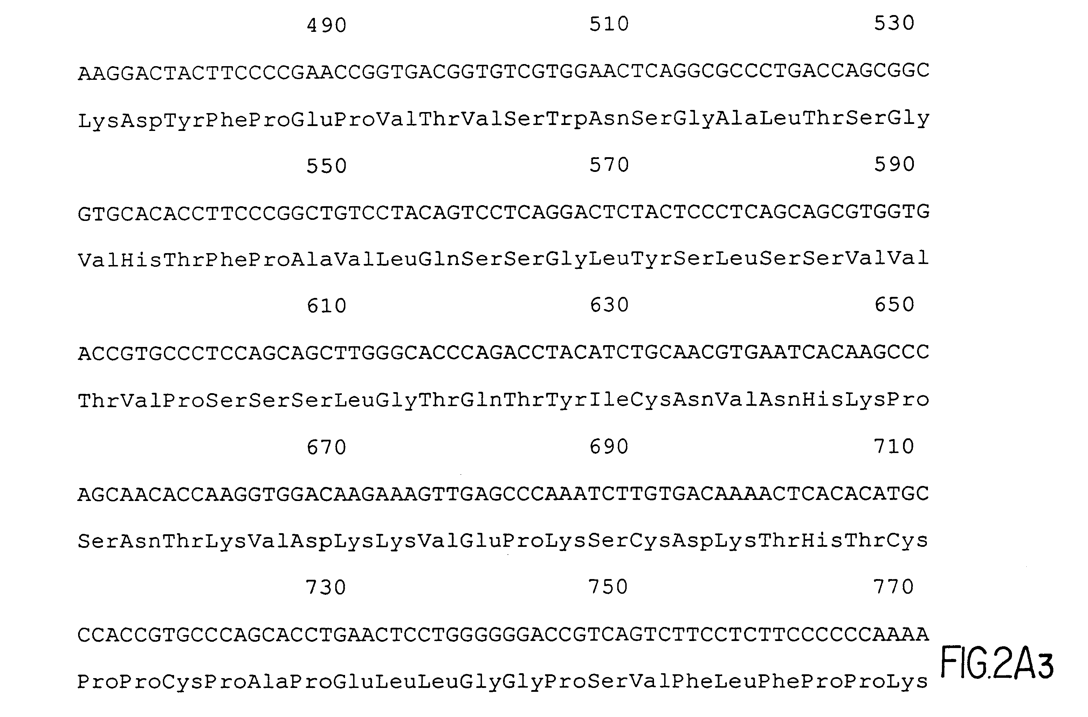

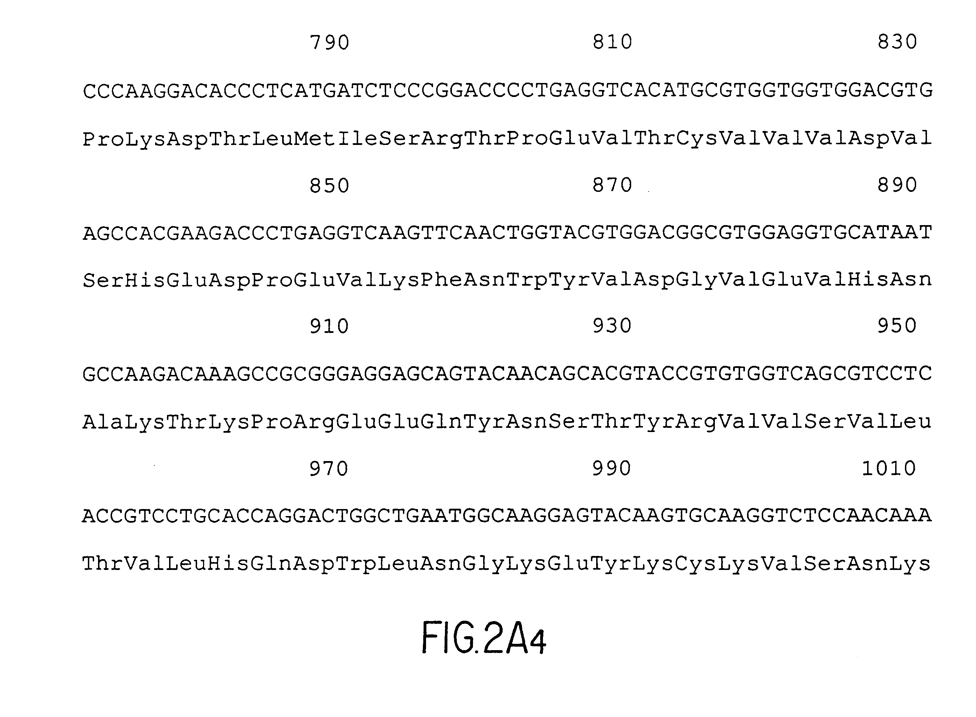

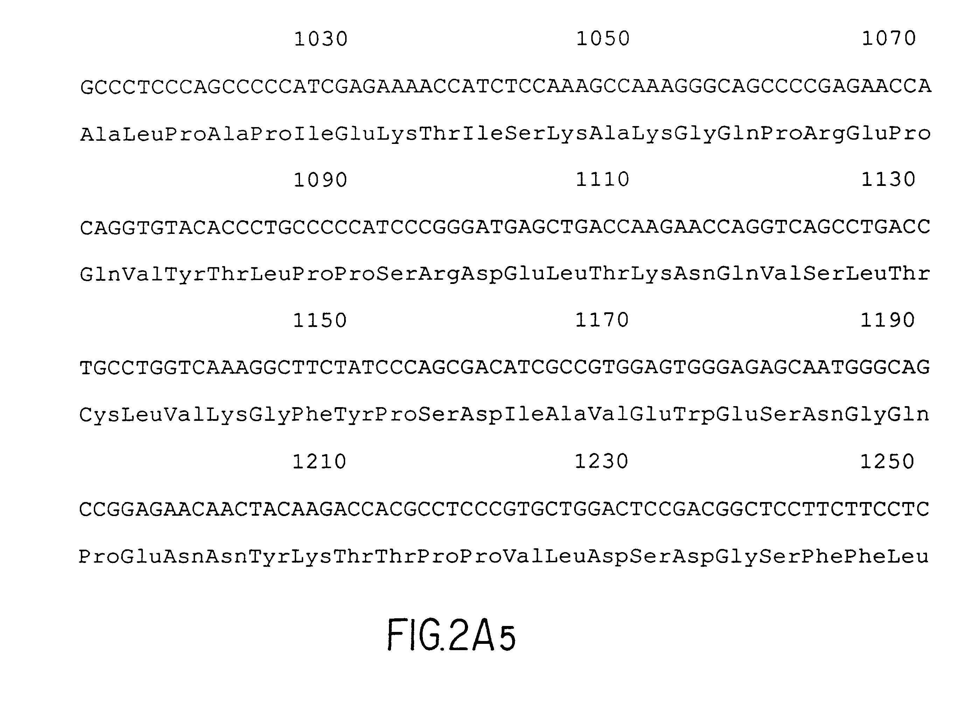

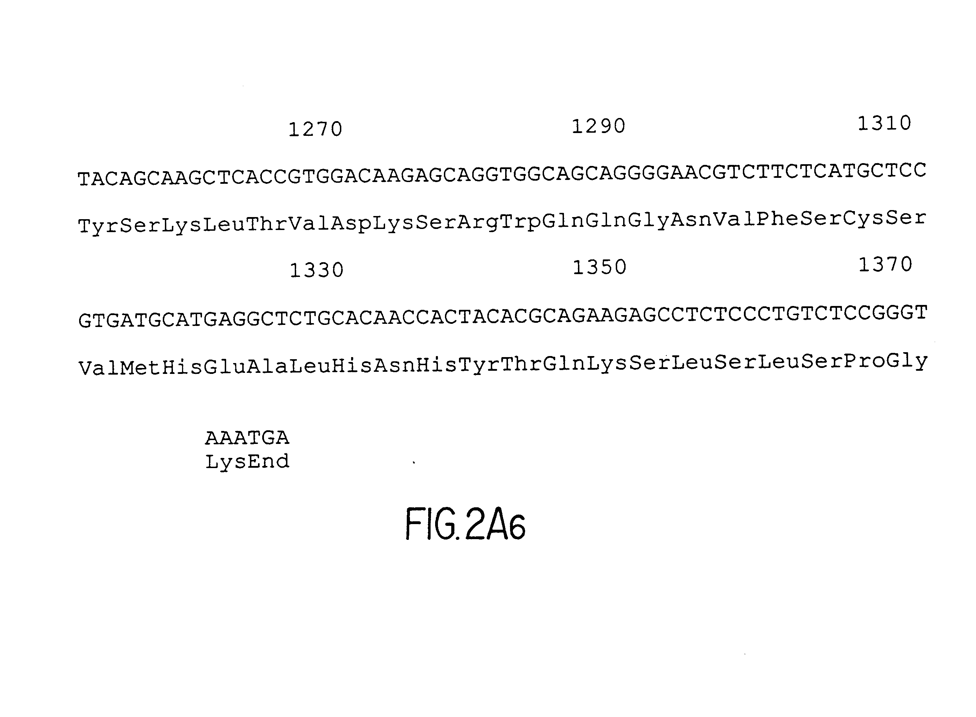

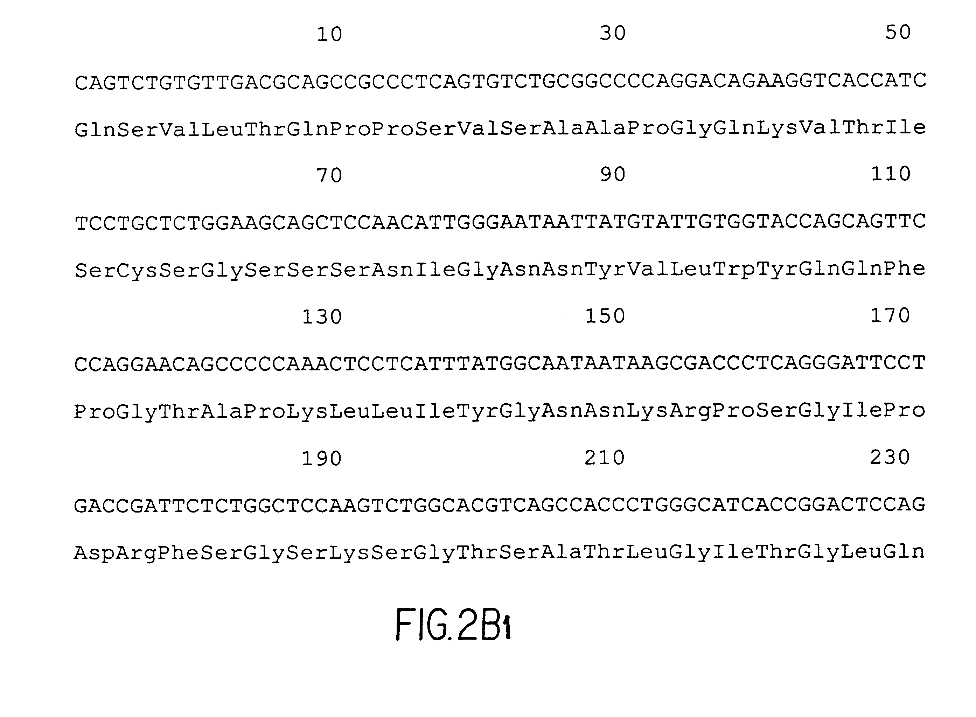

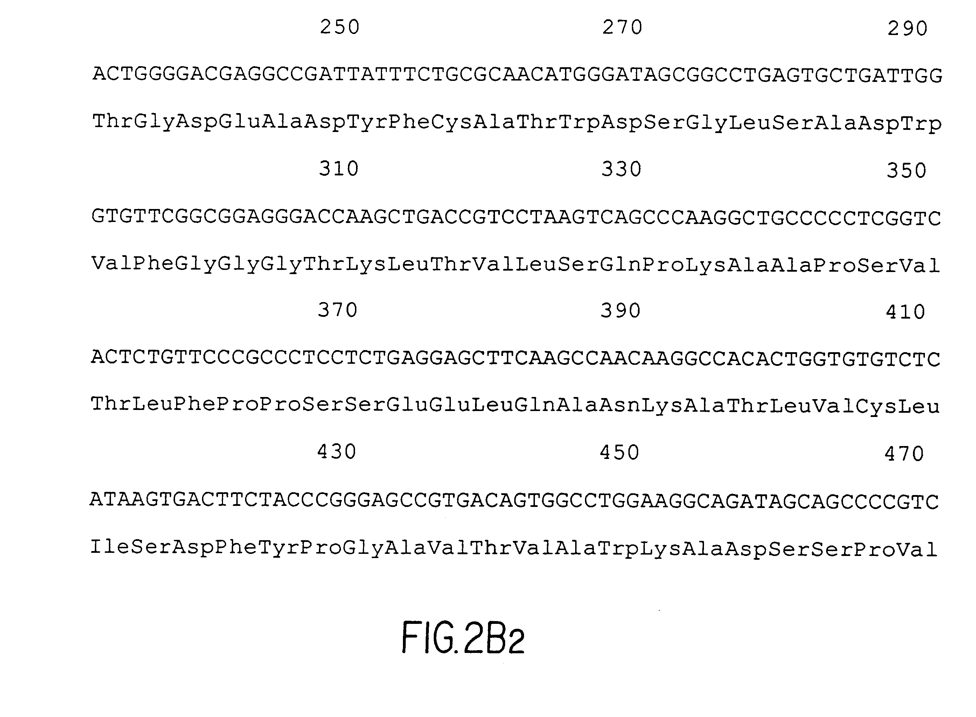

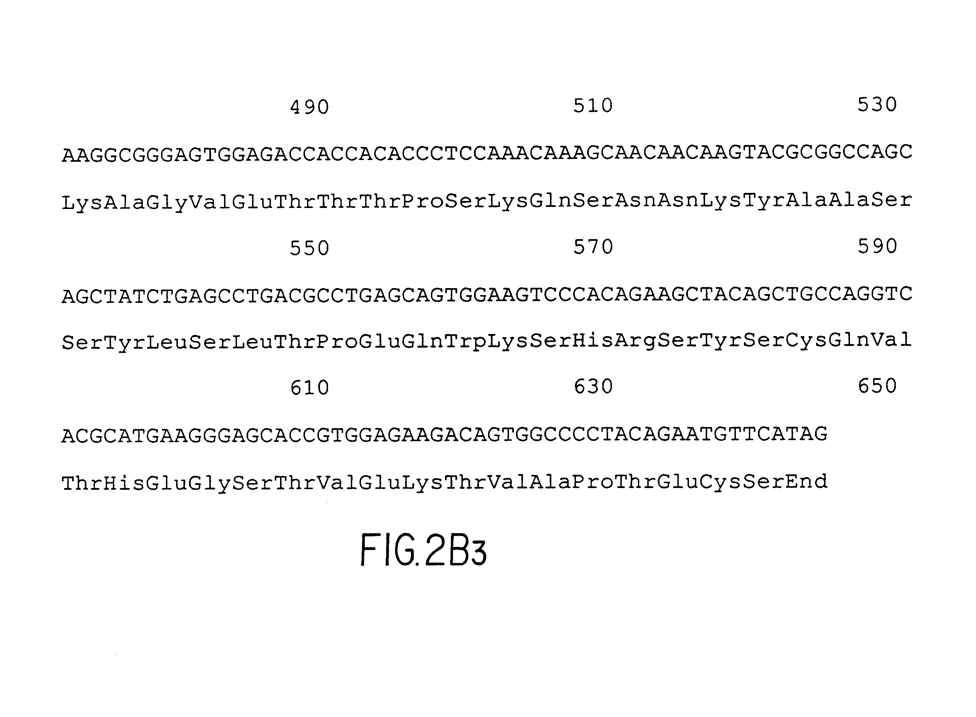

Pairs of oligodeoxynucleotide primers (Figure 1) representing sequences within the human VH signal sequence and the human C-gamma 3'-end and the signal sequences of human V-lambda antibodies and the 3'-end of human C-lambda sequences are synthesized onan Applied Biosystem 381A DNA synthesizer, removed from the resin by treatment with concentrated $NH_4OH$, desalted on a NAP-5 column and eluted with $H_2O$, as are all oligodeoxynucleotide primers described herein. Total RNA, about 1 μg, is reverse transcribed for about 30 minutes at 42°C using AMV reverse transcriptase, about 200 units (Boehringer Mannheim Biochemicals), and about 10 pmoles of the complementary strand primers for either the heavy or light chain. The reverse transcriptase is heat inactivated at about 95°C for about 5 minutes, and the reactions are made to contain in about 100 μl of PCR buffer, about 50 pmoles of each of the paired primers and 25 units of Taq polymerase. About 45 cycles of amplification (1', 94°C; 2', 55°C; 2' 72°C) are followed by gel purification of the anticipated DNA fragments (approximately 1400 or 700 base pair [bp] DNA fragments for heavy and light chains, respectively). Prior to subcloning into intermediate plasmids, these DNAs are digested with either EcoRI (the heavy chain) or EcoRI and Sall (the light chain) and then purified by agarose gel electrophoresis. The heavy chain cDNA is cloned into a derivative of plasmid pUCl8 into which Ncol and Notl sites are engineered between the pre-existing Kpnl and BamHI sites, while the light chain was cloned into plasmid pUCl8. Multiple clones representing these PCR amplified sequences are grown and submitted to DNA sequence determinations. A unique DNA sequence representing a human heavy chain variable region is obtained by analysis of the predicted amino acid sequence, and a human lambda light chain variable reqion is similarly obtained. The sequences of the cloned 447-52D cDNAs described below indicated that the heavy chain most closely resembles a subgroup III heavy chain variable region attached to a gamma 3 constant region, while the light chain variable reqion resembles a subgroup I variable region attached to a lambda constant region. The recombinant 447-52D antibody described herein is assembled to contain V-regions found as part of the 447-52D heterohybridoma and the human gamma 1 (in place of the native gamma 3) and lambda 2 constant regions (sequence of the mature recombinant 447-52D immunoglobulin is shown in Figures 2a and 2b).

The cloned antibody cDNA obtained through the methods described above may be recombinantly expressed by molecular cloning into an expression vector containing a suitable promoter and other appropriate transcription regulatory elements, and transferred into prokaryotic or eukaryotic host cells to produce recombinant antibodies. Techniques for such manipulations are fully described in Maniatis, T. et al., supra and are well known in the art.

Eukaryotic expression vectors are constructed as described below. Expression vectors are defined herein as DNA sequences that are required for the transcription of cloned copies of genes and the translation of their mRNAs in an appropriate host. Such vectors can be used to express eukaryotic genes in a variety of hosts such as bacteria, blue-green algae, plant cells, yeast cells, insect cells and animal cells. The immunoglobulins may also be expressed using a variety of virus-based systems.

Specifically designed vectors allow the shuttling of DNA between hosts such as bacteria-yeast or bacteria-animal cells. An appropriately constructed expression vector should contain: an origin of replication for autonomous replication in host cells, selectable markers, a limited number of useful restriction enzyme sites, a potential for high copy number, and strong promoters. A promoter is defined as a DNA sequence that directs RNA polymerase to bind to DNA and initiate RNA synthesis. A strong promoter is one which causes mRNAs to be initiated at high frequency. Expression vectors may include, but are not limited to, cloning vectors, modified cloning vectors, specifically designed plasmids or viruses.

A variety of mammalian expression vectors may be used to express recombinant anti-HIV-1 antibody in mammalian cells. Commercially available mammalian expression vectors which may be suitable for recombinant antibody expression, include but are not limited to, pMClneo (Stratagene), pXTI (Stratagene), pSG5 (Stratagene), EBO-pSV2-neo (ATCC 37593) pBPV-1(8-2) (ATCC 37110), pdBPV-MMTneo(342-12) ATCC 37224), pRSVgpt (ATCC 37199), pRSVneo (ATCC 37198), pSV2-dhfr (ATCC 37146), pUTCag (ATCC 37460), and λZD35 (ATCC 37565).

DNA encoding the anti-HIV-1 antibody may also be cloned into an expression vector for expression in a recombinant host cell. Recombinant host cells may be prokaryotic or eukaryotic, including but not limited to bacteria, yeast, mammalian cells including but not limited to bacteria, cell lines of human, bovine, procine, monkey and rodent origin, and insect cells including but not limited to drosophila derived cell lines. Cell lines derived from mammalian species which may be suitable and which are commercially available, include but are not limited to, CV-1 (ATCC CCL 70), COS-1 (ATCC CRL 1650), COS-7 (ATCC CRL 1651), CHO-KI (ATCC CCL 61), 3T3 (ATCC CCL 92), NIH/3T3 (ATCC CRL 1658), HeLa (ATCC CCL 2), Cl27I (ATCC CRL 1616), BS-Cl (ATCC CCL 26) AND MRC-5 (ATCC CCL 171).

The expression vector may be introduced into host cells via any one of a number of techniques including but not limited to transformation, transfection, protoplast fusion, and electroporation. The expression vector-containing cells are clonally propagated and individually analyzed to determined whether they produce anti-HIV-1 antibody protein. Identification of antibody expressing host cell clones may be done by several means, including but not limited to immunological reactivity with anti-antibody antibodies, and the presence of host cell-associated anti-HIV-1 antibody.

Expression of antibody DNA may also be performed using in vitro produced synthetic mRNA. Synthetic mRNA can be efficiently translated in various cell-free systems, including but not limited to wheat germ extracts and reticulocyte extracts, as well as efficiently translated in cell bases systems, including but no limited to microinjection into frog oocytes, with microinjection into frog oocytes being preferred.

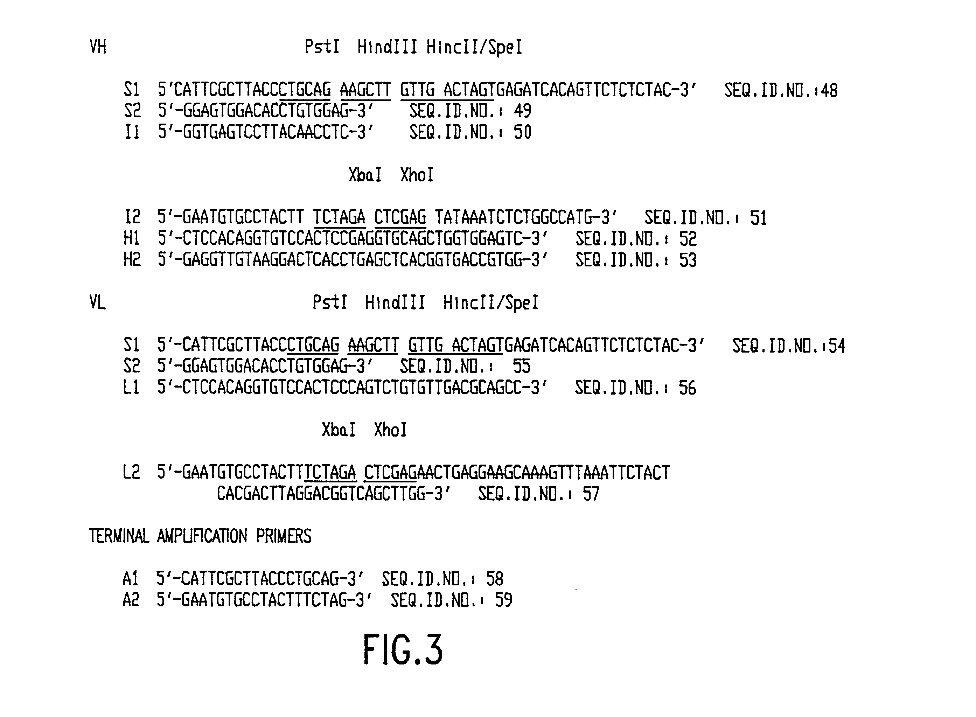

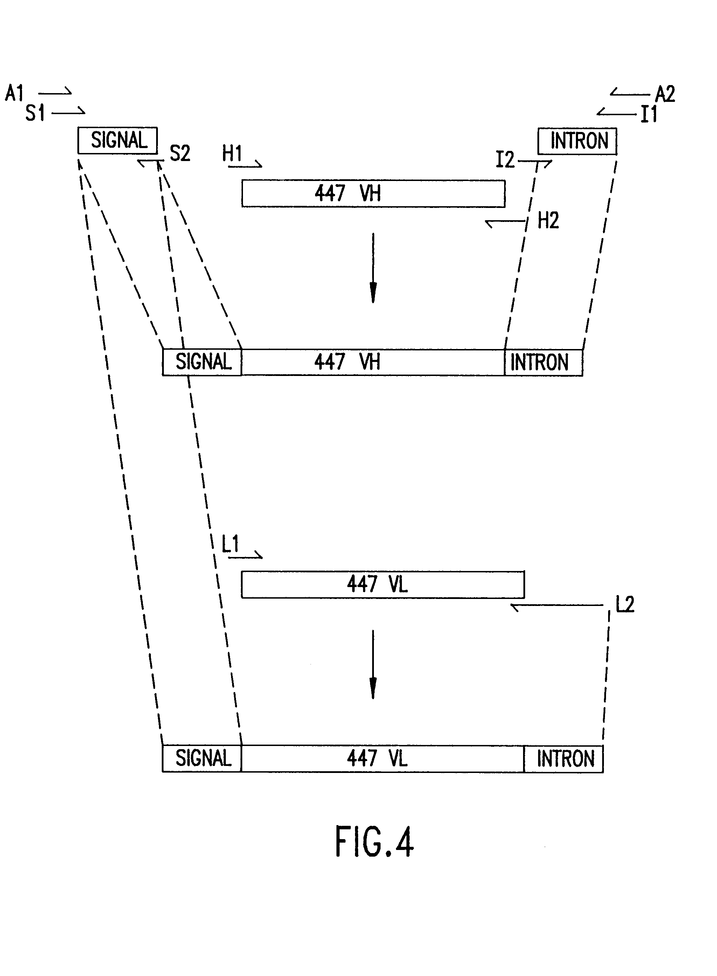

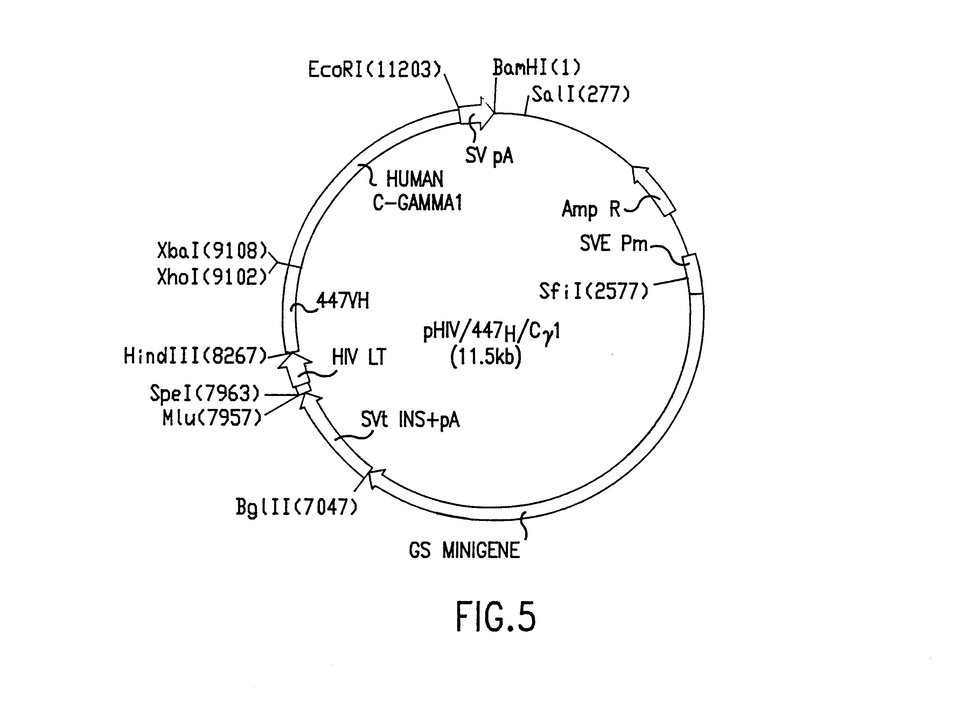

About eight oligodeoxynucleotide primers (Figure 3) are synthesized representing the primers necessary to generate by polymerase chain reaction (PCR) amplification five DNA fragments. Incorporated into all but the terminal oligodeoxynucleotide primers (Figure 3, A1, & A2) were those sequences corresponding to the heavy chain V-region of the 447-52D heterohybridoma, or sequences complementary to the signal and intronic fragments to be combined to the 447-52D V-region, and at least 15 bases of 5'-terminal complementarity to allow for the subsequent PCR-directed recombination of these three fragments (Daugherty, B.L. et al., DNA 9: 453-459, 1990). The appropriate primer pair (S1 & S2, H1 & H2, and I1 and I2), about 50 pmole each, was combined with about 10 ng of plasmid DNA representing the 447-52D heavy chain, about 2.5 units of Taq DNA polymerase and about twenty-five (25) cycles of PCR amplification performed (cycle periods: 1', 94°C; 1', 55°C; 2' 72°C). The products of the five reactions, purified by agarose gel electrophoresis, are combined, about 10 ng of each DNA fragment, along with terminal oligodeoxynucleotide primers (A1 & A2, Figure 3) and Taq DNA polymerase (see Figure 4). The combined fragments are PCR amplified (25 cycles of: 2', 94°C; 2', 55°C; 2' 72°C). Following restriction endonuclease digestion of the signal and intron sequence-appended heavy chain V-region DNAs with HindIII and XhoI the amplified DNA is purified by agarose gel electrophoresis and cloned into compatible sites of vector p9103 which contains the human gamma 1 heavy chain constant region (see Figure 5). This vector is constructed as follows. DNA is purified from the cosIg9 cosmid clone (Flanagan and Rabbits, Nature 300: 709-713, 1982) to act as template for PCR amplification of the human gamma 1 constant region. The appropriate primer pair (G1 & G2 , Figure 1), about 50 pmole each, is combined with about 10 ng of cosmid DNA containing the human gamma 1 constant region exons, about 2.5 units of Taq DNA polymerase and about twenty-five (25) cycles of PCR amplification are performed (cycle periods: 1', 94°C; 1', 55°C; 2' 72°C). The product of the reaction is digested with XhoI and EcoRI restriction enzymes, purified by agarose gel electrophoresis, and cloned into the intermediate vector (p9102) containing a fragment of the HIV-1 promoter (basepairs -117 to +80; pCD23 described in Siekevitz et al., Science 238: 1575, 1987). The signal peptide and intron appended light chain V-region is digested with HindIII and XbaI and the amplified DNA is purified by agarose gel electrophoresis and cloned into the FFFF vector (described below), which has been previously digested with HindIII and EcoRI, along with a DNA fragment representing a human light constant region exon. This latter fragment is obtained by PCR amplification of 10 ng of plasmid #208 DNA, containing an EcoRI/HindIII fragment encompassing the human lambda 2 constant region locus. The appropriate primer pair (C1 and C2, Figure 1), about 50 pmole each, was combined with the plasmid DNA, about 2.5 units of Taq DNA polymerase and about twenty-five (25) cycles of PCR amplification performed (cycle period: 1', 94°C; 2', 55°C; 2' 72°C). The 620 basepair product of the reaction is digested with XbaI and EcoRI restriction enzymes and purified by agarose gel electrophoresis prior to its inclusion in the three piece ligation described above. Analysis of the resultant expression clones reveals the expected about 1.2 kilobase light chain encoding insert following their digestion with HindIII and EcoRI and subsequent agarose gel electrophoresis.

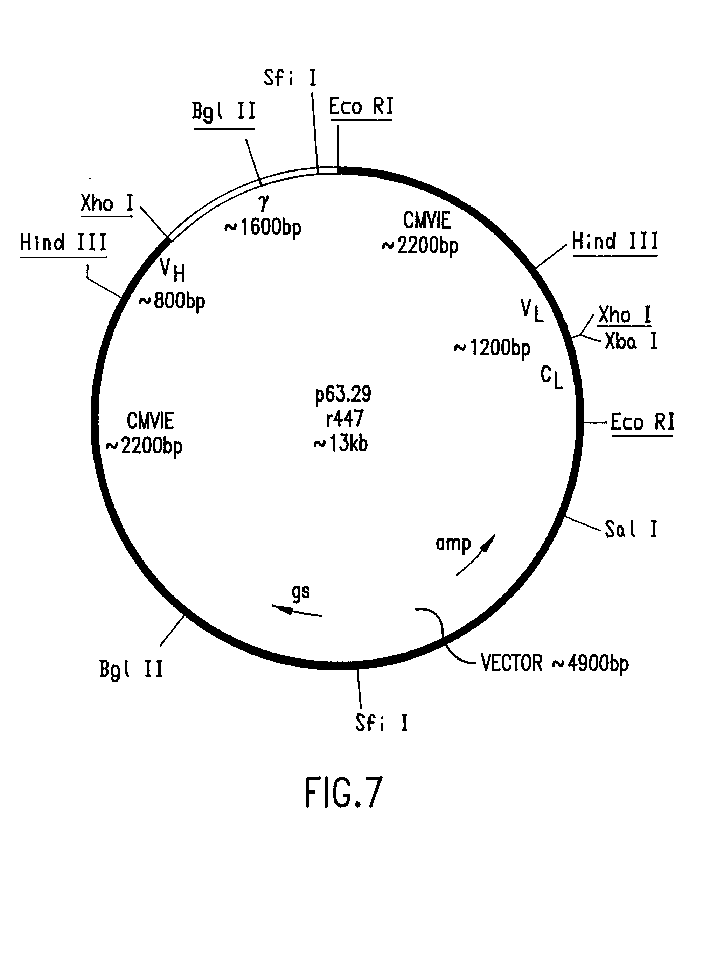

The heavy chain and light chain immunoglobulin molecules are transcribed from plasmids that are identical other than the fact that they contain different immunoglobulin encoding sequences. The preferred progenitor of the immunoglobulin expression vector is the one described above which contains a portion of the HIV-1 LTR promoter (-117 to +80, relative to the cap site), a multiple cloning site, and the SV40 late polyadenylation signal (Figure 7). Plasmid pEE14 (Celltech, Ltd.) is the origin of the majority of the DNA sequences within this vector, designated pSZ9015. The CMVIE promoter of the pEE14 vector was removed by digestion with MluI and HindIII. The 8 kb promoter-minus vector DNA is purified by agarose gel electrophoresis and ligated to an approximately 200 basepair PCR amplified DNA fragment containing 197 basepairs of the HIV LTR (-117 to +80) and MluI and HindIII termini (obtained using the primer pair described in Figure 1 and the pCD23CAT plasmid DNA template). The heavy chain gamma 1 constant region had been placed in the p9015 vector as a XbaI/EcoRI 2.0 kb fragment along with an unrelated heavy chain V-region, creating vector pSP72/58.2/L16VH/$\gamma$1MRC.

Plasmid DNAs containing the 447 heavy chain variable and IgG1 constant regions and the 447 light chain variable and lambda 2 constant regions are grown and purified for transfection into recipient mammalian cells. Equal amounts, about 10 μg, of the plasmids encoding the heavy chain and the lambda light chain are transfected by standard calcium phosphate precipitation procedures into the human embryonic kidney cell line 293 (ATCC CRL 1573). The culture supernants, as assayed by a solid-phase Elisa (described below), were found to contain a human lambda light chain / human IgG1 immunoglobulin. Immulon-2 (Dynatech Labs.) 96-well plates are coated overnight with about a 10 μg/ml solution of mouse anti-human lambda chain constant domain monoclonal antibody (cat. #05-4101, Zymed Laboratories, Inc.) in phosphate-buffered saline (PBS) at about 4°C, and blocked with about 1% bovine serum albumin (BSA) in PBS for about 1 hour at about 37°C. After repeated washing with PBS, samples (conditioned medium containing recombinant 447-52D antibody or human lambda/IgG1 from Sigma Chemical) diluted in PBS containing 1% BSA are added in duplicates and incubated for 1 hour at 37°C. Standard calibration curves are constructed using IgG1 concentrations ranging from about 7.8 ng/ml to about 500 ng/ml. Bound and fully assembled human IgG1 are detected with about 50 μl aliquots of a 1:400 dilution of mouse anti-human IgG1 Fc monoclonal antibody conjugated to horseradish peroxidase (cat #05-3320, Zymed Laboratories, Inc.) in PBS containing about 1% BSA. After incubation for about 1 hour at about 37°C and subsequent washing, the quantities of bound conjugate are detected by addition of about 1mM 2,2'-azino-bis(3-ethylbenzthiazoline-6-sulfonic acid) in about 0.1M sodium citrate, pH4.2, containing 0.03% hydrogen peroxide and incubation at room temperature for 20 min. The adsorbance of the wells is determined with a ELISA plate reader (Bio-Rad, Inc.) set at 415 nm. Alternatively, solid-phase ELISAs are carried out on plates coated with a 26-residue peptide based on the sequence of the MN isolate. The peptide (NIe-CysTyrAsnLysArgLysArgIleGlyProGlyArgAla PheTyrThrThrLysAsnIleIleGlyCys SEQ.ID.NO.:1) is synthesized by solid-phase Fmoc chemistry using preactivated pentafluorophenyl esters and hydroxybenzyltriazine acti-

EP 0 577 243 A2

vation. Immulon-2 plates are coated with about 1 µg/ml peptide overnight, and blocked with about 1% fetal bovine serum in PBS. Detection of bound 447-52D antibody is carried out as described above. The antibody secreted by the transfected human 293 cells following transient expression is purified by protein A chromatography. The concentration of recombinant 447-52D antibody is determined by the ELISAs described above, and tested for efficacy by demonstrating its capacity to neutralize-the infectivity of unique serotypes of HIV-1.

The following assay is performed to quantitate the neutralization of cell-free HIV-1 virus infection as well as the inhibition of cell-to-cell spread by measuring cell survival after exposure of cultures to antibody and virus for 7-8 days. Two fold serial dilutions of the antibody under test are made in cell growth medium (RPMI1640 + 10% fetal calf serum) and 100 µl volumes are placed in the wells of a 96-well dish (Costar, Corp.). 100 µl of virus stock (prepared from chronically infected H9 cells or from newly established chronically infected FDA/H9 cells; 3 day conditioned medium from the chronically infected cell population plated at a cell density of $2 \times 10^5$ cells/mL is clarified and 10 fold more than the last dilution of virus stock which kills all MT-4 cells in a 7 day assay is chosen as the challenge dose) is added to each well and the virus-antibody mixtures are incubated at 37°C for 1 hour. MT-4 cells [Harada et al., (1985), Science, 229, pp563-566] are added to each well ($1 \times 10^4$ cells/well) in 50 µl of culture medium and the dish is incubated for 7 days at 37°C, at which time the endpoint is determined. The concentration of the last antibody dilution which prevents MT-4 cell killing is reported as the neutralization endpoint.

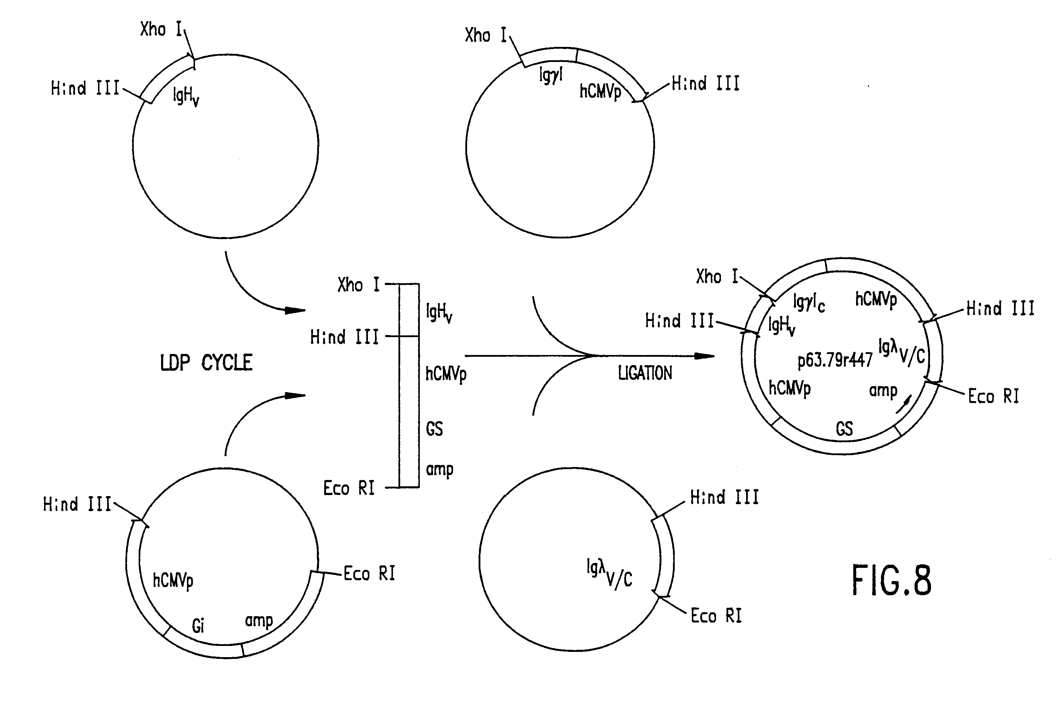

The results of the neutralization assays are shown in Figure 8 and indicate that the potency of the recombinant human 447-52D antibody is equal to that of the human heterohybridoma-derived 447-52D antibody for each HIV-1 serotype investigated. This result shows that the recombinantly constructed antibody, expressed with human lambda and gamma 1 constant domains, has not been modified in such a way as to alter its interactions with the V3 PND loop.

The construction of a complete recombinant 447-52D human HIV-1 neutralizing antibody, whose heavy and light chain variable domains contain the residues of the 447-52D human heterohybridoma antibody, with complete retention of the specificity and potency of the parent heterohybridoma antibody is disclosed.

It is readily apparent to those skilled in the art that the recombinant human anti-HIV-1 antibody disclosed herein possesses the same antigen specificity of the native antibody and also retains the broad spectrum of HIV-1 neutralizing activity. It is also readily apparent to those skilled in the art that other anti-HIV antibodies may be produced according to the methods disclosed in the present invention, to provide a recombinant HIV-1-specific antibody molecule.

Cell lines making human IgG mAb to a neutralizing epitope of HIV-1, such as epitopes associated with the gp120 glycoprotein, are produced by EBV transformation of human peripheral blood mononuclear cells followed by selection of cell lines making antibody of the desired specificity, followed by fusion of the selected EBV-transformed cells to a heteromyeloma cell line. The resultant heterohybridoma cells each make a human mAb having the epitope-specificity (e.g. for the gp120 epitope) of the antibodies produced by the selected parent EBV-transformed cells.

The gp120 glycoprotein, which contains one or more neutralizing epitopes recognized by the cells and antibodies of the present invention, may be derived from any of the known HIV-1 strains, such as the relatively common MN strain.

By the term "heteromyeloma" is intended a hybrid cell produced by fusion of a non-human myeloma cell line and a human myeloma cell line. Typically, a mouse myeloma or plasmacytoma cell is the fusion partner of the human myeloma cell. Such non-human and human myeloma and heteromyeloma cell lines are well-known in the art and are exemplified by cell lines reported in Teng, N.N. et al., Proc. Natl. Acad. Sci. (USA) 80:7308 (1983); Kozbor, D. et al., Hybridoma 2:7 (1983); and Grunow, R. et al., J. Immunol. Meth. 106:257-265 (1988).

As intended in the present invention, heteromyeloma cells are used as fusion partners for selected EBV-transformed human cells to produce the heterohybridomas of this invention.

In a preferred embodiment, the heteromyeloma SHM-D33 is used as a fusion partner. This cell line is available from the ATCC, under accession number ATCC CRL1668.

The term "heterohybridoma", as used herein, refers to a hybrid cell line produced by fusion of an antibody-producing cell of one species with a heteromyeloma. The term "heterohybridoma" has also been used elsewhere to refer to any interspecies hybridoma, such as one resulting from the fusion of an antibody-producing human lymphocytoid cell line cell and a murine myeloma cell. However, the term as used herein is more narrowly defined.

In one embodiment of this invention, a human antibody-producing cell is fused with a mouse-human heteromyeloma. In a preferred embodiment, the heterohybridoma is the result of fusing an EBV-transformed human lymphocyte which is producing an antibody to a neutralizing epitope of HIV, with a human-mouse heteromyeloma. In a more preferred embodiment, the human-mouse heteromyeloma is the cell line designated as SHM-D33.

By the term "neutralizing epitope" is intended an epitope which, when bound by an antibody specific for this epitope, results in neutralization of the virus. Neutralization of any biological activity of the virus, such as, for example, syncytium formation, falls within the scope of "neutralization", as used herein.

To generate human mAbs against a neutralizing epitope of HIV-1 gp120, human peripheral blood lymphocytes are transformed by EBV, as described, for example in Gorny, M.K. et al., Proc. Nat'l. Acad. Sci (USA) 86:1624-1628 (1989).

Preferably, the cells to be transformed are derived from the blood of an individual producing anti-HIV-1 antibodies.

The cultures of EBV-transformed cells are screened for antibody to the epitope of interest. In one embodiment the epitope is a neutralizing epitope of the gp120 protein and the screening is performed using purified gp120, a fragment thereof, or a synthetic peptide representing a portion thereof. In a preferred embodiment, cultures are screened for antibody to an epitope of the V3 loop of gp120 using a synthetic 23-mer peptide from the V3 loop representing amino acids 306-328 (see above for sequence). In addition to such a peptide, additional peptides having at least 6 amino acids are useful for screening the EBV-transformed cells in order to identify antibody producing cells of the desired epitope specificity.

Any of a number of immunoassays well known in the art can be used for this screening process. A preferred immunoassay is an Enzyme Linked Immunosorbent Assay, or ELISA. Using such an assay, the culture supernatants are tested for the presence of antibodies of desired specificity and isotype.

Positive EBV-transformed cultures are cloned repeatedly by any of a number of cloning methods known in the art, such as, for example, by doubling dilution. Cells from cultures found to be positive for the desired antibody specificity are also fused with cells of the heteromyeloma line to produce a heterohybridoma. Fused cells are subsequently cloned by culturing at a density of about 1-100 cells per well.

Specificity of the antibody produced by the heterohybridoma is determined by immunoassay methods which are well known in the art. In a preferred embodiment, ELISA and radioimmunoprecipitation (RIP) procedures are used. The antigen preparation comprises HIV-1 virions (such as strain MN), lysates of viruses or of infected cells, such as MH and HTLV-IIIB lysates, viral proteins such as gp120, or recombinant or synthetic viral peptides such as the 23-mer described above.

The mAbs of the present invention are of the IgG isotypes and may be recovered from the supernatants of the heterohybridoma cell cultures and purified by conventional methods known in the art for purification of IgG. Such methods include, but are not limited to, protein-A Sepharose affinity chromatography, a combination of Affigel Blue (BioRad, Richmond, CA) and Protein-A Sepharose chromatography, or High Performance Liquid Chromatography.

When administered to humans infected with HIV-1, or at risk for HIV infections, the antibodies of the present invention can provide therapeutic or prophylactic benefits. Such individuals particularly at risk are known in the art and include health care workers who have been exposed via a needle stick to HIV-1.

The antibodies of the present invention are also useful in diagnostic assays of the type used to determine if a patient has been exposed to, or infected with, HIV-1.

The antibodies are also useful for analyzing the expression of HIV proteins for which they are specific.

The HIV-specific human mAb of the present invention can be used to treat individuals infected by HIV or suffering from AIDS. The antibodies according to the invention are administered parenterally or enterally by any of a number of known routes. For example, administration may be subcutaneous, intravenous, intramuscular, intraperitoneal, transdermal, or intrathecal. Alternatively, or concurrently, administration may be by the oral, rectal or vaginal route. The antibodies may also be administered into the amniotic cavity for in utero treatment. The preferred routes are intravenous and intramuscular.

The dosage of antibody administered will be dependent upon the age, health, and weight of the recipient, kind of concurrent treatment, if any, frequency of treatment, and the nature of the effect desired. Effective amounts of the mAbs are from about 0.1 to about 500 mg per day, and preferably from about 3 to about 30 mg per day. Treatment may require infusion or injection of the antibody over a period of days, weeks, months, or even years, as would be readily ascertained by one of skill in the art.

A typical treatment regimen comprises administration of an effective amount of antibody administered over between one week and about six months. Duration of treatment required to achieve a therapeutic result will vary from patient to patient, depending upon the severity and stage of the illness and the individual characteristics of each patient.

The total dose required for each treatment may be administered by multiple doses or in a single dose. The mAbs may be administered alone or in conjunction with other therapeutics directed to HIV-1 infection, such as AZT, or directed to other disease symptoms.

The mAbs of the present invention can be administered to HIV-infected expectant mothers. Since the antibodies of the present invention are of the IgG isotype, they can cross the placenta and reach the fetus. This may prevent infection of the fetus or, alternatively, provide effective therapy for an infected fetus.

Pharmaceutical compositions comprising the antibodies of the invention include all compositions wherein the antibody is contained in an amount effective to achieve its intended purpose. In addition to the antibody, the pharmaceutical compositions may contain suitable pharmaceutically acceptable carriers comprising excipients and auxiliaries which facilitate processing of the active compounds into preparations which can be used pharmaceutically. An additional pharmaceutical composition within the scope of the present invention is a combination of the antibody of the invention with an intravenous immunoglobulin preparation as is known in the art.

Pharmaceutical compositions include suitable solutions for administration by injection or orally, and contain from about 0.01 to 99 percent, preferably from about 20 to 75 percent of active component (i.e. the antibody) together with the excipient. Pharmaceutical compositions for oral administration include tablets and capsules. Compositions which can be administered rectally, and vaginally include suppositories.

The mAbs of the present invention can be conjugated to cytotoxic agents and used as immunotoxins (see, for example, Vitetta et al., Science 238:1098-1104 (1987)), or incorporated onto the surface of liposomes containing anti-HIV drugs or toxins to specifically target such drugs or toxins to infected cells. As used herein, the term "immunotoxin" refers to a conjugate of an antibody with one or more toxins, drugs, radionuclides, or cytotoxic agents. A toxic moiety can either be chemically conjugated to the antibody of the present invention, or alternatively, can be ligated through recombinant DNA technology. In such a ligation, the DNA encoding the toxic protein or an active fragment thereof is ligated to the DNA encoding the entire, or a portion of, the mAb heavy chain, light chain, or both. Such genetic constructs and method for making them are known in the art. Among the toxins that may be conjugated to the antibodies of the present invention are ricin, diphtheria toxin, Pseudomonas toxin, tumor necrosis factor-alpha, and others known in the art.

In a typical treatment using the mAbs of the present invention as immunotoxins, the antibody is conjugated to a toxin such as ricin that, alone, is toxic to HIV-infected as well as uninfected cells. By coupling the cytotoxic agent to the antibody, a high level of toxic efficacy can be achieved in a highly localized manner, against the target cell to which the antibody has delivered the toxin, with a sparing of neighboring uninfected cells to which the antibody did not bind.

HIV antiviral therapy is based on a detailed understanding of the viral replication cycle, and almost every definable step of replication offers the possibility of interfering with replication of the virus. Compounds for therapy are available that interfere with HIV binding to the cell. Recombinant CD4 acts as a "molecular decoy" for the virus by competing with cellular receptors for binding viral gp120 envelope proteins. The most successful agents to date affect the next stage, formation of the DNA provirus, by inhibiting reverse transcriptase (RT). These agents include AZT and other nucleoside analogues such as dideoxycytosine (ddC) and dideoxyinosine (ddI) as well as non-nucleoside analogs. The next targets are the synthesis, maturation, and transport from the nucleus to cytoplasm of viral RNA from proviral DNA, which include inhibitors of tat and rev function. Viral mRNA must be translated, and on an experimental basis this step can be specifically inhibited with antisense oligonucleotides. Lastly, viral proteins must assemble to form new virus particles, and this depends on a specific viral proteinase. This proteinase has been crystallized, and inhibitors have been identified [Roberts et al. (1990), Science, 248, pp.358-362].

The present invention is also directed to combinations of the recombinant HIV-neutralizing antibody with one or more agents useful in the treatment or prevention of HIV infection and AIDS. For example, the recombinant antibody of this invention may be effectively administered, whether at periods of pre-exposure and/or post-exposure, in combination with effective amounts of other antiviral agents effective against HIV, immunomodulators, anti-infective agents, or vaccines.

The combination of the recombinant anti-HIV antibody of the present invention and other anti-HIV or AIDS treatment agents are useful in the prevention or treatment of infection by HIV and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymptomatic, and actual or potential exposure to HIV. For example, the combination of compounds of this invention are useful in treating or preventing infection by HIV after or before suspected exposure to HIV by, e.g., blood transfusion, use of blood-derived products, accidental needle stick, exposure to bodily fluids, or exposure to patient blood during surgery.

For these purposes, the recombinant anti-HIV antibody of the present invention and other anti-HIV or AIDS treatment compounds are administered in combination, either orally, parenterally (including subcutaneous injections or infusion techniques), by inhalation spray, rectally or vaginally, in dosage unit formations containing conventional non-toxic pharmaceutically-acceptable carriers, adjuvants and vehicles.

Thus, in accordance with the present invention there is further provided combination pharmaceutical compositions for the prevention and treatment of HIV infection and AIDS. The treatment involves administering to a patient in need of such treatment a pharmaceutical carrier and a combination of therapeutically-effective amounts of the recombinant anti-HIV antibody of the present invention and the anti-HIV or AIDS treatment compounds.

These combination pharmaceutical compositions may be in the form of orally-administrable suspensions or tablets nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate, lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, adsorption promoters to enhance bioavailability, fluorocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally or vaginally administered in the form of suppositories, these compositions may be prepared by mixing the drug with a suitable non-irritating excipient, such as coca butter, synthetic glyceride esters or polyethylene glycols, which are solid at ordinary temperatures, but liquify and/or dissolve at body temperature to release the drug.

The compounds of this invention can be administered orally to humans in a dosage range of 1 mg to 5 g/kg body weight in single or divided doses. It is understood, however, that the specific dose level, dosage ratio, and frequency of dosage for any particular patient may be varied and will depend upon a variety of factors including the activity or potency of the specific compound employed, the metabolic stability and length of action of the compound, the age of the patient, body weight, general health, sex, diet, mode and time of administration, rate of excretion, drug combination, the severity of the particular conditions, and the host undergoing therapy.

The novel combination pharmaceutical compositions of the present invention comprise the recombinant anti-HIV antibody disclosed herein, in combination with one or more anti-HIV agents which function to interrupt or inhibit HIV replication, and agents for treatment of AIDS related conditions. These anti-HIV agents include but are not limited to those which interfere with virus attachment to the cellular receptor (e.g. soluble CD4, and related peptides, dextran sulfate, N-Butyl DNJ); inhibitors of virus assembly (e.g. castanospermine, 6-0-butanoyl castanospermine, myristoylation inhibitors); reverse transcriptase inhibitors including but not limited to nucleoside analogs (e.g. AZT, ddC, ddl, d4T, AzdU, A-69992, IAF-BCHl89, carbovir, Fascarnet, Ganciclovir, and Acyclovir) and non-nucleoside analogs such as hydroxy pyridones (e.g.

3-{[(4,7-dichlorobenzoaxazol-2-yl)methyl]amino-5-ethyl-6-methyl-2-(1H)-pyridinone,

3-{[(4,7-dimethylbenzoxazol-2-yl)methyl]amino}-5-ethyl-6-methyl-2-(1H)-pyridinone,

3-{[(7-chlorobenzoxazol-2-yl)methyl]amino}-5-ethyl-6-methyl-2-(1H)-pyridinone,

3-{[(7-methylbenzoxazol-2-yl)methyl]amnio}-5-ethyl-6-methyl-2-(1H)-pyridinone,

3-{[(4-fluorobenzoxazol-2-yl)methyl]amino}-5-ethyl-6-methyl-2-(1H)-pyridinone,

3-{[(7-fluorobenzoxazol-2-yl)methyl]amino}-5-ethyl-6-methyl-2-(1H)-pyridinone,

3-{[(benzoxazol-2-yl)methyl]amino}5-ethyl-6-methyl-2-(1H)-pyridinone,

3-{[(4-chlorobenzoxazol-2-yl)methyl]amino}-5-ethyl-6-methyl-2-(1H)-pyridinone,

3-{[(4-fluoro-7-chlorobenzoxazol-2-yl)methyl]amino}5-ethyl-6-methyl-2-(1H)-pyridinone, and

3-[2-(benzoxazol-2-yl)ethyl]-5-ethyl-6-methyl-2-(1H)-pyridinone); inhibitors of transcription trans-activation (e.g. tat and rev protein inhibitors, such as 7-chloro-5-(2-pyrryl)-3H-1, 4-benzodiazepin-2(H)-one, or Ro5-3335); retroviral proteinase inhibitors (e.g. nonhyrolyzable chemical mimics of the structure of an enzymatic transition state, substrate, or reaction intermediate, R039-8959 [Hoffman La Roche]); and inhibition of viral genetic expression using antisense oligonucleotides. Agents useful for the treatment of AIDS-related conditions include, but are not limited to, agents to treat opportunistic infections, interferon, granulocyte macrophage colony stimulating factor, interleukin 2, tumor necrosis factor, and erythropoietin.

In a preferred embodiment of the present invention, the recombinant anti-HIV antibody is combined in a pharmaceutical composition with HIV reverse transcriptase inhibitors and/or HIV proteinase inhibitors. The preferred reverse transcriptase inhibitors are the amino pyridones listed above. The preferred proteinase inhibitors are the hydroxyethylamine transition state mimetics, for example R031-8959 (Hoffman LaRoche). Most preferred are the combinations of the recombinant anti-HIV antibody and one or more of the reverse transcriptase inhibiting amino pyridones listed above.

In addition, the recombinant anti-HIV antibody of this invention may be effectively administered, whether at periods of pre-exposure and/or post-exposure, in combination with effective amounts of the AIDS antivirals, immunomodulators, anti-infectives, such as those in the following Table.

## TABLE

### ANTIVIRALS

| Drug Name | Manufacturer | Indication |
|-----------|--------------|------------|
| AL-721 | Ethigen (Los Angeles, CA) | ARC, PGL HIV positive, AIDS |
| Recombinant Human Interferon Beta | Triton Biosciences (Almeda, CA) | AIDS, Kaposi's sarcoma, ARC |
| Acemannan | Carrington Labs (Irving, TX) | ARC (See also immunomodulators) |
| Cytovene Ganciclovir | Syntex (Palo Alto, CA) | sight threatening CMV peripheral CMV retinitis |
| d4T Didehydrodeoxy-thymidine | Bristol-Myers (New York, NY) | AIDS, ARC |
| ddI Dideoxyinosine | Bristol-Myers (New York, NY) | AIDS, ARC |
| EL10 | Elan Corp, PLC (Gainesville, GA) | HIV infection (See also immunomodulators) |

| Drug Name | Manufacturer | Indication |
|---|---|---|
| Trisodium Phosphonoformate | Astra Pharm. Products, Inc. (Westborough, MA) | CMV retinitis, HIV infection, other CMV infections |
| Dideoxycytidine; ddC | Hoffman-La Roche (Nutley, NJ) | AIDS, ARC |
| Novapren | Novaferon Labs, Inc. (Akron, OH) Diapren, Inc. (Roseville, MN, marketer) | HIV inhibitor |
| Peptide T Octapeptide Sequence | Peninsula Labs (Belmont, CA) | AIDS |
| Zidovudine; AZT | Burroughs Wellcome (Rsch. Triangle Park, NC) | AIDS, adv, ARC pediatric AIDS, Kaposi's sarcoma, asymptomatic HIV infection, less severe HIV disease, neurological involvement, in combination w/other therapies, post-exposure prophylaxis in health care workers |
| Ansamycin LM 427 | Adria Laboratories (Dublin, OH) Erbamont (Stamford, CT) | ARC |

13

| Drug Name | Manufacturer | Indication |
|---|---|---|
| Dextran Sulfate | Ueno Fine Chem. Ind. Ltd. (Osaka, Japan) | AIDS, ARC, HIV positive asymptomatic |
| Virazole Ribavirin | Viratek/ICN (Costa Mesa, CA) | asymptomatic HIV positive, LAS, ARC |
| Alpha Interferon | Burroughs Wellcome (Rsch. Triangle Park, NC) | Kaposi's sarcoma, HIV in combination w/Retrovir |
| Acyclovir | Burroughs Wellcome | AIDS, ARC, asymptomatic HIV positive, in combination with AZT. |

## IMMUNO-MODULATORS

| Drug Name | Manufacturer | Indication |
|---|---|---|
| Antibody which neutralizes pH labile alpha aberrant Interferon in an immunoadsorption column | Advanced Biotherapy Concepts (Rockville, MD) | AIDS, ARC |
| AS-101 | Wyeth-Ayerst Labs. (Philadelphia, PA) | AIDS |
| Bropirimine | Upjohn (Kalamazoo, MI) | advanced AIDS |

| Drug Name | Manufacturer | Indication |
|---|---|---|
| Acemannan | Carrington Labs, Inc. (Irving, TX) | AIDS, ARC (See also anti-virals) |
| CL246,738 | American Cyanamid (Pearl River, NY) Lederle Labs (Wayne, NJ) | AIDS, Kaposi's sarcoma |
| EL10 | Elan Corp, PLC (Gainesville, GA) | HIV infection (See also anti-virals) |
| Gamma Interferon | Genentech (S. San Francisco, CA) | ARC, in combination w/TNF (tumor necrosis factor) |
| Granulocyte Macrophage Colony Stimulating Factor | Genetics Institute (Cambridge, MA) Sandoz (East Hanover, NJ) | AIDS |
| Granulocyte Macrophage Colony Stimulating Factor | Hoeschst-Roussel (Somerville, NJ) Immunex (Seattle, WA) | AIDS |
| Granulocyte Macrophage Colony Stimulating Factor | Schering-Plough (Madison, NJ) | AIDS AIDS, in combination w/AZT |

| Drug Name | Manufacturer | Indication |
|---|---|---|
| HIV Core Particle Immunostimulant | Rorer (Ft. Washington, PA) | seropositive HIV |
| IL-2 Interleukin-2 | Cetus (Emeryville, CA) | AIDS, in combination w/AZT |
| IL-2 Interleukin-2 | Hoffman-La Roche (Nutley, NJ) Immunex | AIDS, ARC, HIV, in combination w/AZT |
| Immune Globulin Intravenous (human) | Cutter Biological (Berkeley, CA) | pediatric AIDS, in combination w/AZT |
| IMREG-1 | Imreg (New Orleans, LA) | AIDS, Kaposi's sarcoma, ARC, PGL |
| IMREG-2 | Imreg (New Orleans, LA) | AIDS, Kaposi's sarcoma, ARC, PGL |
| Imuthiol Diethyl Dithio Carbamate | Merieux Institute (Miami, FL) | AIDS, ARC |
| Alpha-2 Interferon | Schering Plough (Madison, NJ) | Kaposi's sarcoma w/AZT: AIDS |
| Methionine-Enkephalin | TNI Pharmaceutical (Chicago, IL) | AIDS, ARC |
| MTP-PE Muramyl-Tripeptide | Ciba-Geigy Corp. (Summit, NJ) | Kaposi's sarcoma |

16

| Drug Name | Manufacturer | Indication |
|---|---|---|
| Granulocyte Colony Stimulating Factor | Amgen (Thousand Oaks, CA) | AIDS, in combination w/AZT |
| rCD4 Recombinant Soluble Human CD4 | Genentech (S. San Francisco, CA) | AIDS, ARC |
| rCD4-IgG hybrids | | AIDS, ARC |
| Recombinant Soluble Human CD4 | Biogen (Cambridge, MA) | AIDS, ARC |
| Interferon Alfa 2a | Hoffman-La Roche (Nutley, NJ) | Kaposi's sarcoma AIDS, ARC, in combination w/AZT |
| SK&F106528 Soluble T4 | Smith, Kline & French Laboratories (Philadelphia, PA) | HIV infection |
| Thymopentin | Immunobiology Research Institute (Annandale, NJ) | HIV infection |
| Tumor Necrosis Factor; TNF | Genentech (S. San Francisco, CA) | ARC, in combination w/gamma Interferon |

## ANTI-INFECTIVES

| Drug Name | Manufacturer | Indication |
|---|---|---|
| Clindamycin with Primaquine | Upjohn (Kalamazoo, MI) | PCP |
| Fluconazole | Pfizer (New York, NY) | cryptococcal meningitis, candidiasis |
| Pastille Nystatin Pastille | Squibb Corp. (Princeton, NJ) | prevention of oral candidiasis |
| Ornidyl Eflornithine | Merrell Dow (Cincinnati, OH) | PCP |
| Pentamidine Isethionate (IM & IV) | LyphoMed (Rosemont, IL) | PCP treatment |
| Piritrexim | Burroughs Wellcome (Rsch. Triangle Park, NC) | PCP treatment |
| Pentamidine isethionate for inhalation | Fisons Corporation (Bedford, MA) | PCP prophylaxis |
| Spiramycin | Phone-Poulenc Pharmaceuticals (Princeton, NJ) | cryptosporidial diarrhea |

| Drug Name | Manufacturer | Indication |
|---|---|---|
| Intraconazole-R51211 | Janssen Pharm. (Piscataway, NJ) | histoplasmosis; cryptococcal meningitis |
| Trimetrexate | Warner-Lambert | PCP |

### OTHER

| | | |
|---|---|---|
| Recombinant Human Erythropoietin | Ortho Pharm. Corp. (Raritan, NJ) | severe anemia assoc. and AZT therapy |
| Megestrol Acetate | Bristol-Myers (New York, NY) | treatment of anorexia assoc. w/AIDS |
| Total Enteral Nutrition | Norwich Eaton Pharmaceuticals (Norwich, NY) | diarrhea and malabsorption related |

It will be understood that the scope of combinations of the recombinant anti-HIV antibody of this invention with AIDS antivirals, immunomodulators, anti-infectives or vaccines is not limited to the list in the above Table, but includes in principle any combination with any pharmaceutical composition useful for the treatment of AIDS.

The following Examples are provided to illustrate the present invention without, however, limiting the same thereto.

EXAMPLE 1

OPTIMIZING PRODUCTION OF HUMAN MONOCLONAL ANTIBODIES TO HIV-1

The objective of this study was to examine the optimal conditions for establishing cell lines producing human mAbs against HIV-1.

METHODS

Peripheral blood lymphocytes derived from 74 HIV seropositive individuals were transformed with EBV. Cultures producing antibodies to HIV were expanded and cloned several times on irradiated GK5 feeder cells by doubling dilution (5000 to 10 cells/well). Five of the 74 specimens could be processed by both cloning and fusions. Simultaneous with the first cloning (i.e. 5 to 7 weeks after initiation of culture), the lymphoblastoid cells from expanded cultures were fused with heteromyeloma SHM-D33 cells. Anti-HIV positive hybrids were cloned at 100 to 1 cell/well. The specificity of the mAbs was tested by ELISA, Western blot and RIP.

RESULTS

The immortalization of PBL by EBV alone gave rise to 2 cell lines synthesizing human mAbs. However

19

when EBV-transformed cells were fused to SHM-D33, 5 hybrid lines making human mAb were obtained. All cell lines have been in culture for 6-12 months.

Table I

| Patient Code | Stable EBV Lines | Stable Hybrids | Isotype | Specificity |
|---|---|---|---|---|
| 167 | None | 167-7-D | IgG1,lambda | gp41 |
| 181 | None | 181-1-D | IgG2,kappa | gp41 |
| 240 | None | 240-1-D | IgG1,kappa | gp41 |
| 238 | 238-2 | 238-2-D | IgG1,lambda | p24 |
| 241 | 241-1 | 241-1-D | IgG1,lambda | p24 |

EBV transformation of blood cells followed by fusion to a heteromyeloma appears to be the most effective method for the generation of human mAb to HIV-1 and is more efficient than EBV transformation alone.

EXAMPLE 2

Subjects

A group of 41 asymptomatic HIV-seropositive individuals participated in the study. The presence of serum antibodies to HIV-I was tested by commercial ELISA (Genetic Systems) and confirmed by Western blot using Novapath Immunoblot Assay (BioRad). The CD4 and CD8 phenotype of lymphocytes from each subject was determined using Leu 3a and Leu2a antibodies (supplied by Becton-Dickinson) by flow cytometry using a Cytofluorograf II (Ortho). Peripheral blood white blood cell counts were processed by a Coulter Counter and differential counts were performed manually.

Patients were classified as to disease progression using an immunologic staging system such that patients were divided into four categories on the basis of the following, previously described criteria (Zolla-Pazner, S. et al, Proc. Natl. Acad. Sci. (USA) 84:5404 (1987)):

| Scale Score | CD4:CD8 Ratio | CD4 cells/mm$^3$ | Lymphocytes/mm$^3$ |
|---|---|---|---|
| 0 | >1.0 | >500 | >1500 |
| 1 | <1.0 | >500 | >1500 |
| 2 | <1.0 | <500 | >1500 |
| 3 | <1.0 | <500 | <1500 |

Synthetic Peptide Used for Screening

A peptide which spans 23 amino acids of the gp120 V3 loop of the MN strain of HIV-1 (23-mer peptide) was synthesized by solid-phase methodology (Peninsula Laboratories, Inc. Belmont, CA). The peptide has the following sequence:

```
TyrAsnLysArgLysArgIleHisIleGlyProGlyArgAlaPheTyrThrThr
LysAsnIleIleGly (SEQ.ID.NO.: 2)
```

This peptide was used in an ELISA assay to screen for antibodies reactive to it.

Establishment of EBV-Transformed Cell Lines

The method for producing human cell lines synthesizing mAbs to HIV-1 was described by Gorny et al., 1989, supra. Peripheral blood mononuclear cells were incubated with Epstein-Barr virus (EBV) and cultured for 3-4 weeks in 96-well microplates. After screening for antibodies in the culture supernatants using the 23-mer peptide in an ELISA, cells from cultures having supernatants positive for antibodies were expanded, subcultured several times, and finally further expanded into flasks. These cells are called lymphoblastoid cells.

## Cell Fusion

The heteromyeloma (mouse-human hybrid) SHM-D33 (Teng N.H. et al., Proc. Natl. Acad., Sci. USA. 80:7308 (1983)) was grown in Iscove's modified Dulbecco's medium supplemented with 15% fetal bovine serum, 2 mM L-glutamine, penicillin (100 units/ml), and streptomycin (100 µg/ml) (complete medium). Periodically, heteromyeloma cells were cultured with the antibiotic G418 at 200 µg/ml to eliminate neomycin-sensitive variants.

Two days prior to fusion, the SHM-D33 cells were cultured at a concentration of $1\text{-}2 \times 10^5$ cells/ml (log phase growth). The viability of the cells, as determined by erythrosin B dye exclusion, exceeded 95%.

The SHM-D33 cells were washed twice in phosphate-buffered saline and then mixed with the lymphoblastoid cells which had been expanded from initial culture but had not yet been cloned. The cells were mixed at a ratio of 1:3 and centrifuged. Then, 1 ml of 50% polyethylene glycol 1300-1600 (Sigma Chemicals) was added dropwise to the pellet over a period of one minute with constant agitation that was continued for another one minute. During the next five minutes, the cells were slowly diluted with Iscove's medium and, after pelleting by centrifugation at 200 Xg, the cells were gently resuspended in complete medium and plated in 96-well microplates at a concentration of $8 \times 10^4$ cells/100 µl/well. The next day, $1 \times 10^4$ mouse peritoneal cells were added per well as feeder cells, and the cultures were continued in the presence of 0.5 mM hypoxanthine, 0.2 µM aminopterin, 16 µM thymidine (HAT) and 1 µM ouabain (Sigma Chemicals). Feeding was repeated twice weekly with fresh complete medium supplemented with HAT. After two to three weeks all culture wells were screened for antibody production against the aforementioned peptide and heterohybrids producing antibodies reactive with the 23-mer peptide were expanded in 24-well plates. Hybrids that produced the highest level of antibodies (and IgG) measured by ELISA were cloned at concentrations of 100, 25, and (at least twice at) 1 cell per well.

## Antibody Detection and Characterization

Culture supernatants were screened against the 23-mer by ELISA. Immulon 2 plates (Dynatech) were coated overnight at 4°C with the synthetic peptide (1 µg/ml), diluted in sodium carbonate buffer, pH 9.6. Plates were washed three times and culture supernatants were added to each well and incubated for 90 minutes at 37°C, then washed. Goat anti-human IgG (gamma chain-specific) conjugated to alkaline phosphatase (Zymed Laboratories) was added and incubated for another 90 minutes at 37°C and washed as above. The substrate, p-nitrophenyl phosphate (Sigma Chemicals), was added for 30 minutes and the absorbance was read at 405 nm on a MR 700 Microplate Reader (Dynatech).

The specificity of antibody binding was assessed by radioimmunoprecipitation (RIP). RIP assays were carried out by the method of Pinter et al., (J. Immunol. Meth. 112:735 (1988)) with 30 µg of HTLV-IIIB lysate (Organon Teknika) and/or MN lysate (Advanced Biotechnologies, Inc.) labelled with $^{125}$I using the Bolton-Hunter reagent (New England Nuclear). Culture supernatants were incubated with viral lysate and further processed as described by Gorny et al., supra, and Pinter et al., supra.

## Analysis of Human Antibodies

Antibody isotypes were determined by ELISA. Immulon 2 plates were coated with 1 µg/ml of the 23-mer and incubated with culture supernatants. The subtype of the IgG mAb was detected by alkaline phosphatase-labelled mouse mAbs against the four subclasses of human IgG (Zymed Laboratories).

The light chain of mAb was analyzed by ELISA using microplates coated with rabbit antibodies to human kappa chain or lambda chain (Dakopatts). The developing antibodies used were alkaline phosphatase-coupled goat anti-human kappa chain and goat anti-human lambda chain (Sigma Chemicals), respectively.

IgG quantitation was also performed by ELISA. Plates were coated with goat anti-human IgG (gamma chain-specific) and incubated with serially diluted culture supernatants. Bound IgG was detected with alkaline phosphatase-labelled goat anti-human IgG (gamma chain-specific). Affinity-purified human IgG (Organon Teknika-Cappel) was used as a standard. Plates were read and standard curves were generated using an automated MR-700 Microplate Reader (Dynatech Laboratories).

Epitope Mapping

The fine specificity of the mAb was determined using the Epitope Mapping Kit (Cambridge Research Biochemicals, Valley Stream, New York) which utilizes the method developed by Geysen et al., (Geysen, H. M. et al. Proc. Natl. Acad Sci. (USA) 81:3998-4002 (1984)) to synthesize hexapeptides on plastic pins. Eighteen sequential, overlapping hexapeptides which spanned the 23-mer were synthesized in situ on plastic pins with two additional control peptides. The peptides were deprotected, then washed and dried according to the manufacturer's instructions. Because the configuration of pins fit to 96-well microplate, the ELISA assays were carried out in standard microplates as recommended by the manufacturer. Thus, all peptide-containing pins were allowed to react with culture supernatants from the cell lines being tested at a 1:10 dilution in 0.1% Tween-20 in PBS containing 1% ovalbumin and 1% bovine serum albumin.

Thereafter, the pins were washed and reacted with horseradish peroxidase-conjugated goat anti-human IgG. The color reaction was read in a Dynatech MR-700 plate reader as absorbance at 405 nM.

RESULTS

A total of 46 blood specimens derived from 41 HIV-seropositive individuals were processed and transformed with EBV. After 3 to 4 weeks of culture, an average of 2.9% of the wells were positive for antibody against the 23-mer of the V3 loop as revealed by ELISA. Table II shows that the percentage of positive wells was slightly increased in the group of subjects with a scale score of 1, but that there was no significant difference in the yield of positive cultures from patients with different levels of severity of the disease.

TABLE II

| PRODUCTION OF ANTIBODIES SPECIFIC FOR A 23-mer OF THE MN STRAIN gp120 V3 LOOP BY EBV-TRANSFORMED HUMAN LYMPHOCYTES | | | |
|---|---|---|---|
| Scale Score | # of Cultures | # of Wells | # (%) Positive Wells |
| 0 | 1 | 610 | 16 (2.6%) |
| 1 | 17 | 4363 | 176 (4.0%) |
| 2 | 21 | 7340 | 171 (2.3%) |
| 3 | 7 | 2880 | 81 (2.8%) |

Lymphoblastoid cells from positive wells were further expanded in 24-well plates and, once per week, fresh culture supernatants were tested for antibody specificity by ELISA using the 23-mer peptide. Two lymphoblastoid cell lines, 257-2 (ATCC #CRL10483) and 268-11 (ATCC #CRL10482), that were producing high levels of specific antibody against the 23-mer were cloned by doubling dilution (from 10,000 to 10 cells per well). Cells from wells plated at the lowest cell density that continued to produce antibodies were further cloned three times at 100 to 10 cells/well.

Simultaneously with the original cloning, both lymphoblastoid cell lines (257-2, 268-11) were fused with heteromyeloma SHM D-33. All wells showed growth of hybrid cells. Three weeks after fusion, 50 of 183 wells (29%) plated with 257-2 heterohybrids and 43 out of 48 wells (90%) plated with 268-11 heterohybrids were found to contain antibody against the 23-mer. From each fusion, the eighteen clones producing the highest concentration of antibody (based on absorbance in ELISA), were expanded in 24-well plates. The production of antibodies was monitored weekly, and cells producing supernatant yielding the highest specific antibody and IgG concentrations were selected for cloning. The heterohybridomas were cloned at 100 and 25 cells/well and subsequently twice at 1 cell/well.

While the lymphoblastoid cell lines 257-2 (ATCC #CRL10483) and 268-11 (ATCC #CRL10482) produced 6.4 and 3.8 $\mu$g IgG/ml/$10^6$ cells/24 hr, respectively, the related heterohybridomas, 257-2D (ATCC #HB 10480) and 268-11D (ATCC #10481) produced 20.5 and 11.3 $\mu$g IgG/ml/$10^6$ cells/24 hr, respectively. The mAbs were shown to react in ELISA with the 23-mer when the latter was bound to the wells of microtiter plates at concentrations as low as 1 ng/ml (Figure 1).

The specificity of these mAbs was further defined by RIP, the results of which are shown in Figure 2. Both mAbs react with the env-encoded protein gp120 of HIV$_{MN}$ but not with the gp120 derived from HTLV-III$_B$, revealing the type-specificity of these mAbs.

The mAbs were found to be of the IgG isotype with lambda light chains. Table III shows some characteristics of the two EBV-transformed parent lines and the two related heterohybridomas. The heterohybridomas produce three times as much IgG in 24 hours as the EBV-transformed lines, even though the EBV-transformed lines produce considerably more than most EBV-transformed lines described in the literature (Kozbor, D. et al., Immunol. Today 4:72 (1983); Casali, P. et al., Science 234:476 (1986); and Steinitz, M. et al., Nature 269:420 (1977)).

---

### TABLE III

### CHARACTERISTICS OF CELL LINES PRODUCING HUMAN MONOCLONAL ANTIBODIES SPECIFIC FOR A 23-mer OF THE MN gp120 V3 LOOP

| Cell Line | Immortalization | Isotype | IgG Concentration* |
|---|---|---|---|
| 257-2 | EBV | IgG1 | 6.4 |
| 268-11 | EBV | IgG1 | 3.8 |
| 257-2D | EBV + fusion | IgG1 | 20.5 |
| 268-11D | EBV + fusion | IgG1 | 11.3 |

* $\mu g / 10^6$ cells/ml/24h

---

In order to define the fine specificity for each antibody, epitope mapping was performed using overlapping hexapeptides which represent sequential hexapeptides overlapping by five amino acids. Each peptide was synthesized in quadruplicate, so that it was possible to test four samples on one microplate simultaneously. The overlapping antigenic regions and the results of these experiments are shown in Table IV.

A pool of seronegative sera was not reactive. A seropositive serum sample (serum from HIV-seropositive individual at a dilution of 1:1000) reacted above background levels with all pins, giving peak reactions with three pins spanning the region P G R A F Y T T (SEQ.ID.NO.: 3) at the tip and right side of the V3 loop.

MAb 257-2D, at a dilution of 1:10 (3.7 μg/ml), bound strongly to two adjacent hexapeptides representing amino acid 309-315 to the left of the top of the loop R-K-R-I-H-I-G (SEQ.ID.NO.: 4). MAb 268-11D (5.4 μg/ml) bound to one hexapeptide covering the amino ac id sequence H-I-G-P-G-R (SEQ.ID.NO.: 5). Table IV shows the overlapping antigenic regions recognized by the two mAbs.

TABLE IV

| REACTIVITY OF HUMAN SERA AND HUMAN MONOCLONAL ANTIBODIES WITH HEXAPEPTIDES OF THE MN gp120 V3 LOOP | | | | | | |
|---|---|---|---|---|---|---|
| | | | ELISA REACTIVITY (Absorbance units) | | | |
| PIN# | SEQ.ID.NO. | Hexapeptide | HIV+ Serum | HIV- Serum | 257-2D | 268-11D |
| 3 | 6 | YNKRKR | .338 | .195 | .256 | .243 |
| 4 | 7 | NKRKRI | .339 | .200 | .288 | .283 |
| 5 | 8 | KRKRIH | .251 | .184 | .259 | .239 |
| 6 | 9 | RKRIHI | .308 | .1831 | .781 | .252 |
| 7 | 10 | KRIHIG | .286 | .1701 | .592 | .179 |
| 8 | 11 | RIHIGP | .302 | .177 | .276 | .135 |
| 9 | 12 | IHIGPG | .267 | .197 | .130 | .132 |
| 10 | 13 | HIGPGR | .269 | .185 | .1561 | .011 |
| 11 | 14 | IGPGRA | .361 | .191 | .163 | .164 |
| 12 | 15 | GPGRAF | .243 | .103 | .208 | .132 |
| 13 | 16 | PGRAFY | .586 | .237 | .456 | .346 |
| 14 | 17 | GRAFYT | .582 | .239 | .272 | .288 |
| 15 | 18 | RAFYTT | .658 | .239 | .333 | .350 |
| 16 | 19 | AFYTTK | .257 | .197 | .233 | .222 |
| 17 | 20 | FYTTKN | .384 | .233 | .273 | .274 |
| 18 | 21 | YTTKNI | .362 | .171 | .259 | .259 |
| 19 | 22 | TTKNII | .284 | .191 | .219 | .212 |
| 20 | 23 | TKNIIG | .305 | .198 | .164 | .141 |

These results indicate that the smallest reactive peptide (core of the epitope) that 257-2D recognizes is K R I H I (SEQ.ID.NO.: 23), located to the left of the conserved tip of the V3 loop. The flanking N-and C-terminal arginine and glycine residues may also contribute to the binding of this mAb. The mAb 268-11D bound to a single hexapeptide consisting of H I G P G R (SEQ.ID.NO.: 5) which spans the tip of the loop and the two adjacent N-terminal amino acids.

EXAMPLE 3

HUMAN HETEROHYBRIDOMA PRODUCTION WITHOUT PROLONGED EXPANSION OF EBV-TRANSFORMED LYMPHOCYTES

METHOD

The fusion of EBV-transformed cells and heteromyeloma SHM-D33 is usually performed after 2-3 weeks of expansion of EBV immortalized cells in 24-well microplates. This is equivalent to 5-7 weeks after culture initiation. However, the expansion period is very critical for production of the mAb because the majority (at least 90%) of culture wells become negative for mAb production during this period. We therefore tested an alternative method in which the expansion period was excluded and fusion took place 3-4 weeks after culture initiation. Thus, 96-well plates with EBV-transformed cells were screened for the presence of an antibody to HIV-1, 3-4 weeks after culture initiation. Cells from all wells producing anti-HIV antibody were pooled and immediately fused with heteromyeloma SHM-D33 as described in Example 2.

## RESULTS

PBL derived from 4 HIV-seropositive individuals produced 8 cell lines secreting mAb to HIV-1 after EBV transformation followed by early fusion. Six mAb were directed against p24 and 2 mAbs to gp41 of HIV-1 as determined by RIP and ELISA. 21 stable lines from 300 patients were obtained by EBV-transformation or by EBV-transformation and fusion as described in Examples 1 and 2. This is is equivalent to 6-7 lines per 100 patient specimens when 20-40 ml of patient's blood are used. Using the "early fusion method" described in this example, 8 lines were obtained from 4 patient specimens, a significant increase in the efficiency of obtaining stable antibody-producing lines.

## DISCUSSION

The early fusion of EBV-transformed cells without expansion of positive wells is a more efficient method than our previous techniques for the generation of human mAbs to HIV-1.

## EXAMPLE 4

## DETERMINATION OF MONOCLONAL ANTIBODY AFFINITY

Determination of the dissociation constants ($K_d$) of human mAbs was performed using an ELISA methodology as described by B. Friguet et al., J. Immunol. Methods 77:305-319 (1985). Briefly, the culture supernatants of 257-2D and 268-11D were tested at concentrations of 0.5 and 0.6 µg/ml, respectively. The 20-mer (MW=2702 Da), described above, was dissolved in distilled water to a concentration of 1 mg/ml ($3.7 \times 10^{-4}$ M), diluted in phosphate-buffered saline, pH 7.2, and used at concentrations ranging from $10^{-5}$ to $10^{-8}$ M. Supernatant and peptide were mixed in equal volumes and after 16 hours, the mixture was added to plates coated with the 23-mer (1 µg/ml) and the amount of unbound mAb was measured by ELISA. Data were plotted according to the Friguet modification of the Klotz method (Friguet et al., supra) to determine the $K_d$.

The $K_d$ of mAbs 257-2D and 268-11D were found to be $2.3 \times 10^{-7}$ and $5.9 \times 10^{-7}$ M, respectively. These values are in the range of those reported by others for IgG mAbs (Friguet et al., supra; Larsson, A. et al., Molec. Immunol. 24:569-576 (1987)). The $K_d$ of the mAbs produced by the human lymphoblastoid cell lines (257-2 and 268-11) were similar to those of the mAbs produced by related heterohybridomas (257-2D and 268-11D). The $K_d$ described above are for the binding of the mAbs to the 20-mer peptide. The $K_d$ of these mAbs for native gp120 molecules may be lower due to the contributions of the conformation of the whole protein molecule to the epitopes to which the mAbs react.

## EXAMPLE 5

## NEUTRALIZATION OF HIV INFECTIVITY

A plaque assay which measures the inhibition of HIV infection of MT-2 cells was used to detect the neutralizing activity of the mAbs of the present invention in the presence or absence of human complement (C.V. Hanson et al., J. Clin. Micro. 128: (1990); Harada et al., (1985), Science, 229, pp.563-566). Thus, mAbs were serially diluted in 50% assay medium (Hanson et al., supra) and 50% of a normal human plasma pool. The plasma pool served as the source of human complement; for studies in the presence of complement, mAb and virus were incubated for 18 hours at 37°C. For tests in the absence of complement, the plasma pool was heat-inactivated and the mAb and virus were incubated under these conditions for 1 hour at 37°C. The dilution at which 50% of the input virus was neutralized on the basis of plaque counts was calculated by interpolation using third order regression analysis of the mean plaque count at each dilution.

## RESULTS

When supernatant fluids from 257-2D and 268-11D were incubated with $HIV_{MN}$ for 1 hour (no complement) prior to addition to permissive MT-2 cells, 50% neutralization was achieved at dilutions of 1:4,700 and 1:2,000, corresponding to mAb concentrations of 3.0 and 23.0 ng/ml, respectively (Table 5). No neutralization of HTLV-IIIB was observed.

When the mAbs from 257-2D and 268-11D were tested in a more sensitive assay, wherein antibodies were incubated with virus for 18 hours in the presence of human complement, neutralization was achieved at dilutions of 1:44,000 and 1:41,000, corresponding to mAb concentrations of 0.3 and 1.1 ng/ml, respectively. Again, no neutralization of HTLV-IIIB occurred under these conditions.

Human mAb 50-69, specific for the HIV transmembrane protein, gp41, and mAb 71-31, specific for the core protein, p24, previously described by Gorny, M.K. et al., Proc. Natl. Açad. Sci. (USA) 86:1624-1628 (1989)), were tested in parallel and displayed essentially no neutralizing activity for either strain of HIV.

TABLE V

| NEUTRALIZING ACTIVITY OF HUMAN MONOCLONAL ANTIBODIES AGAINST HIV | | | | | |
|---|---|---|---|---|---|
| | | Neutralizing Antibody Titers (ng/ml) | | | |
| | | 1 hr, no C' | | 18 hr + C' | |
| mAb | Specificity | MN | IIIB | MN | IIIB |
| 257-2D | gp120 | 1:4700 (3) | neg | 1:44000 (0.3) | neg |
| 268-11D | gp120 | 1:2000 (23) | neg | 1:41000 (1.1) | neg |
| 50-69 | gp41 | 1:3 | neg | 1:3 | neg |
| 71-31 | p24 | neg | neg | neg | neg |

EXAMPLE 6

HUMAN mABS TO HIV-1 V3 LOOP REACT WITH DIVERGENT VIRUS STRAINS

Using the methods described above, additional EBV-transformed cell lines and heterohybridomas producing human mAbs specific for the V3 loop of HIV$_{MN}$ gp120 were produced. Several of the heteromyelomas were designated 386-D, 391-D, 419-D, 447-52D, 477-D, 311-11D, 391-95D and 412-D. The reactivity patterns of some of these mAbs are compared to those of 257-2D and 268-11D (described above) are shown in Table 6, below.

### TABLE VI

**HIV-Type Specificity and Neutralization Activity of Six Human Monoclonal Antibodies Specific for gp120**

| mAb | Core Epitope | Reactive in ELISA to synthetic V3 | | | | Neutralization | | | |
|-----|--------------|------|----|------|----|------|----|------|----|
| | | IIIB | MN | SF-2 | RF | IIIB | MN | SF-2 | RF |
| 257-2D | KRIHI (SEQ.ID.NO.24) | − | + | + | + | − | + | + | − |
| 268-11D | HIGPGR (SEQ.ID.NO.:5) | − | + | + | + | − | + | + | − |
| 386-D | HIGPGR (SEQ.ID.NO.:5) | − | + | + | + | − | + | | |
| 391-95D | * | − | + | + | − | − | + | | |
| 419-D | * | − | + | + | − | − | + | | |
| 447-52D | GPGR (SEQ.ID.NO.:25) | + | + | + | + | | + | | |
| 311-11D | RKRIHIGPGRAFYTT (SEQ.ID.NO.:26) | − | + | + | − | − | + | | |
| 412-D | (conformational) | + | + | + | − | − | − | | |

\* Not determined

These results lead to the following conclusions. (1) The tip of the V3 loop constitutes a cluster of epitopes recognized by human mAbs; (2) the human mAbs cross-react in ELISA with some or all synthetic V3 peptides from divergent HIV-1 strains; (3) all tested anti-V3 (MN) human mAbs neutralize the MN virus, including one mAb (257-2D) directed primarily to a region N-terminal to the most conserved region of the loop ; (4) cross-neutralization can occur even when 2 out of 5 amino acids in the core epitope are changed (e.g., 257-2D reacting with MN and SF-2) but some changes in the core epitope abrogate neutralizing activity (e.g., 268-11D reacting with MN and not with IIIB); and (5) cross-reactivity as detected with ELISA is much less stringent than cross-reactivity measured in a biologic assay.

In order to further identify the epitope to which HuMoAb 447-52D reacts, the HuMoAb was tested for reactivity with a set of 18 hexapeptides spanning the region of V3$_{MN}$ represented by the 23-mer used for screening; each hexapaptide overlaps with its neighbor by five amino acids. The hexapeptides, synthesized in situ on polyethylene pins using the method of Geysen et al. (14), were reacted with culture supernatants containing 29 μg/ml of HuMoAb. Figure 1 shows that the HuMoAb reacted with three hexapaptides, HIGPGR SEQ.ID.NO.: 13, IGPGRA SEQ.ID.NO.: 14 and GPGRAF SEQ.ID.NO.: 15. These data suggest that the epitope to which HuMoAb 447-52D is directed is hiGPGRaf, where the captial letters of the amino acid code represent the core of the epitope and the lower case letters represent flanking amino acids which may also contribute to the binding of the epitope.

To determine which amino acids within the region were critical to the binding of the HuMoAb, a series of hexapeptides were synthesized on polyethylene pins such that each residue of the HIGPGR hexapeptide was substituted with each of 19 amino acids. Thus, 115 hexapeptides were synthesized and each was reacted with HuMoAb 447-52D at a concentration of mg/ml. The results of this experiment are shown in Figure 7 and suggest that the first, second and fifth residues of HIGPGR can be varied considerably while still allowing detectable reactivity. The substitutions not permitted, e.g., C, D, and E for I at the second position, are never or only rarely seen in virus isolates sequenced to date (Meyers et al., (1990), Theoretical Biology and Biophysics, Los Alamos; LaRosa et al., (1990), Science, 249, pp932). However, the third, fourth and sixth residues of this hexapaptide cannot be changed without abrogating the ability for the peptide to react with the HuMoAb. These data suggest that the core epitope of HuMoAb 447-52D is most correctly represented as GPXR and that the amino acid sequences flanking this epitope do not contribute substantially to antibody recognition of this core epitope.

## EXAMPLE 7

### RELATION BETWEEN CROSS-REACTIVITY AND AFFINITY OF HUMAN mABS TO SYNTHETIC PEPTIDES OF THE V3 LOOP OF DIVERGENT HIV STRAINS

The reactivity of human mAbs, described above, was measured by direct ELISA against diverse synthetic peptides (19-mers through 23-mers) which had been coated on plates at concentrations of 1 μg/ml. Antibody affinity, measured as dissociation constants ($K_d$) of binding to these peptides, were determined as described in Example 4, above.

The results are shown in Table 7. Anti-V3 human mAbs at 2-4 μg/ml cross-reacted with synthetic peptides of the V3 region of HIV-1 strains MN, SF-2 and RF, in an ELISA. No reaction was detected with the V3 peptide derived from HIV-1 strain IIIB. Kd's for binding to these peptides ranged from $\geqq 10^{-6}$M (268-11D binding to RF) to $10^{-7}$M (268-11D binding to MN).

### TABLE VII

| mAb | Core Epitope | Relative Binding Affinity | ELISA Reactivity to V3 from: | | | |
|---|---|---|---|---|---|---|
| | | | MN | SF-2 | RF | IIIB |
| 257-2D | KRIHI (SEQ.ID.NO.24) | MN = SF2 >> RF, IIIB=0 | 1.8* | 1.6 | 1.6 | 0.3 |
| 268-11D | HIGPGR (SEQ.ID.NO.5) | MN > SF2 > RF, IIIB=0 | 1.9 | 1.9 | 1.3 | 0.2 |
| 386-D | HIGPGR (SEQ.ID.NO.5) | MN > SF2 >> RF, IIIB=0 | | | | |

\* Absorbance units at 410 nm

These results lead to the following conclusions: (1) Human mAbs specific for the HIV-1 V3 region display cross-reactivity to V3 regions of divergent virus strains, as measured by ELISA or as antibody affinity; (2) affinities of human mAbs to different V3 peptides may vary by about one order of magnitude or more; (3) affinity differences cannot be explained simply by the amino acid sequence in the relevant epitope; and (4) human mAbs with identical epitope-specificity vary in their affinities for different V3 peptides.

## EXAMPLE 8

A number of additional human mAbs were produced and tested using the methods described above. These antibodies and their specificity and affinity characteristics are described in Table 8, below.

The correlation between the dissociation constants and the neutralization capacity of five human mAbs was analyzed . The results indicate a direct relationship between these two properties, suggesting that the affinity of binding to the 23-mer peptide from the V3 loop is a good predictor of efficacy for virus neutralization.

Table VIII

Characteristics of Human Monoclonal Antibodies Specific for HIV-1 gp120 Epitopes

| | | | ELISA Reactivity* | | | | | |
|---|---|---|---|---|---|---|---|---|
| Human mAb | Isotype | Epitope | MN | SF-2 | RF | NY5 | IIIB | ELI |
| 257-2D | IgG1,lambda | KRIHI | + | + | + | + | − | − |
| 268-11D | IgG1,lambda | HIGPGR | + | + | + | + | − | − |
| 386-D | IgG,lambda | HIGPGR | + | + | + | + | − | − |
| 447-52D | IgG3,lambda | GPGR | + | + | + | + | + | − |
| 447-D | IgG1,lambda | HIGP | + | + | − | − | − | − |
| 311-11D | IgG1,lambda | ** | + | + | − | + | − | − |
| 391-95D | IgG1,Kappa | ** | + | + | − | + | − | − |
| 419-D | IgG1,lambda | ** | + | + | − | + | − | − |
| 412-D | IgG1,lambda | ** | + | − | − | + | − | − |

TABLE VIII (continued)

| | Affinity (Kd in mM) | | | | IgG 50% Titer |
|---|---|---|---|---|---|
| Human mAb | MN | SF-2 | RF | IIIB | ng/ml |
| 257-2D | .23 | .22 | 1.7 | NT | 1.0 |
| 268-11D | .59 | 2.3 | 5.3 | NT | 1.0 |
| 386-D | .18 | .85 | 1.7 | NT | 1.2 |
| 447-52D | .56 | .32 | 96 | 43 | NT |
| 447-D | NT | NT | NT | NT | NT |
| 311-11D | 7.4 | 6.0 | NT | NT | 392.0 |
| 391-95D | .9 | 14.4 | NT | NT | 4.9 |
| 419-D | 3.8 | .23 | NT | NT | 12.0 |
| 412-D | 27 | NT | NT | NT | Neg |

TABLE VIII (continued)

```
NT:  not tested
 * :  tested with mAbs at 10 mg/ml except for 477-D which was tested at <5 mg/ml
 **:  not determined by Epitope Mapping Kit but shown to be reactive with the
peptide RKRIHIGPGRAFYTT
IgG 50% Titer refers to the neutralization titer, indicating the concentration
in ng/ml at which the antibody gave 50% neutralization at 18 hr with no
complement (except for 257-2D and 268-1D, which were tested at 1hr without
complement.
```

Relative affinity of human mAbs (from low to high)

```
HIV
Antigen  ......................1.0.........1.0........................(Kd in mM)


MN       412 <  311 <  419 <  391 <  268=447 <  257 <  386


SF-2     311 <  391 <  268 <  386 < 447~419~257
```

## EXAMPLE 9

### Preparation of a Recombinant 447-52D Antibody

An antibody was produced in which the variable domain of the light chain contains a signal sequence and light chain intronic sequence-appended version of the heterohybridoma 447-52D light chain variable region fused to a DNA fragment containing a short intronic segment of the human lambda 2 constant region, and the lambda 2 constant encoding domain. The variable domain of the heavy chain is similarly derived from the heterohybridoma 447-52D heavy chain V-region to which the same signal sequence and a heavy chain intronic sequence are appended, fused to a fragment containing a short intronic segment of the human gamma 1 constant region, and the human gamma 1 encoding domain in its genomic form.

Total RNA was extracted from the 447-52D heterohybridoma cells using standard methods involving cellular solubilization with guanidinium isothiocyanate (Chirgwin et al., Biochem. 18:5294-5299 [1979]). Sets of oligodeoxynucleotide primers (Figure 1) representing sequences within the signal peptide of the human lambda light chain variable region and lambda light chain constant domain, or those within framework 1 of the human heavy chain variable region and the heavy chain gamma 3 constant domain, were synthesized by standard phosphoramidite procedures on an Applied Biosystem 381A DNA synthesizer. Removal of the oligodeoxynucleotides (oligos) from the resin was accomplished by treatment with concentrated $NH_4OH$ followed by desalting on a NAP-5 column (Pharmacia) with $H_2O$ elution (when the oligos were <45 bases in length), or by use of an OPC column (Applied Biosystems Inc.) with 20% acetonitrile elution (when the oligos were >45 bases in length), as recommended by the manufacturers. Total RNA (2µg) was reversed transcribed for 30 minutes at 42°C using AMV reverse transcriptase (200 units, BRL) and 10 pmoles of the constant region complementary strand primers representing either heavy or light chain in a buffer (final volume of 20 µl) containing 50 mM TRIS HCl, pH 8.3, 75 mM KCl, 3 mM $MgCl_2$, 10 mM DTT, and 20 units of RNAsin (Pharmacia). The reverse transcriptase was heat inactivated (95°C, 5 minutes) and the reactions were made to contain in 100µl of PCR buffer (10 mM TRIS HCl, pH 8.3, 50 mM KCl, 1.5 mM $MgCl_2$, 0.01% gelatin, 200 µM each dNTP), 50 pmoles of each of the paired primers, and 2.5 units of Taq polymerase (Perkin Elmer/Cetus). Polymerase chain reaction (PCR) amplification was carried out essentially as described by Saiki et al., Science 230:1350-1354 (1985) and others (Mullis et al., Cold Spring Harbor Symp. Quant. Biol. 51:263:273 [1986]; Dawasaki and Wang, PCR Technology, Principles and Applications for DNA Amplification, Erlich, Ed., Stockton Press, NY, pp. 89-97 [1989]; Tung et al., ibid. pp. 99-104 [1989]). Forty-five cycles of amplification by a DNA Thermal Cycler (Perkin Elmer Cetus Instruments; 1', 94°C; 2', 55°C; 2' 72°C) were followed by gel purification of the anticipated 1400 and 700 base pair (bp) DNA fragments. Prior to subcloning these DNAs into intermediate plasmids the DNAs were digested with either EcoRI (the heavy chain) or EcoRI and SalI (the light chain) and then purified by agarose gel electrophoresis. The heavy chain cDNA was cloned into a derivative of pUCl8 into which NcoI and NotI sites were placed between the pre-existing KpnI and BamHI sites, while the light chain was cloned into pUCl8. Multiple clones representing these PCR amplified sequences were isolated from DH5 transformed E. Coli plated on LB agar plates containing 50 μg/ml ampicillin, grown by described procedures (Maniatis et al., Molecular Cloning, A Laboratory Manual, Cold Spring Harbor Laboratory, Cold Spring Harbor, NY, 1982). Plasmid DNAs were extracted from the bacteria using the DNA preparation procedures of Birnboin and Doly, Nucleic Acid Res. 7: 1515(1979), and the double-stranded plasmid DNAs were subjected to DNA sequence determinations using Sequenase® (United States Biochemicals) and, initially, T7 and SP6 specific sequencing primers (Boehringer Mannheim) using the protocols recommended by the manufacturer. A unique DNA sequence representing a human gamma 3 heavy chain was obtained, as was a human lambda light chain sequence.

Eight oligodeoxynucleotides (Figure 3) were synthesized representing the primers necessary to generate, by PCR amplification, five DNA fragments. Incorporated into all but the terminal oligodeoxynucleotides were those sequences corresponding to the heavy chain V-region or light chain V-region of the 447-52D heterohybridoma, or sequences complementary to the signal and intronic fragments to be combined to the 447-52D V-regions, and at least 15 bases of 5'-terminal complementarity (see Figures 3 & 4). The appropriate primer pair (50 pmole each) was combined with 10 ng of plasmid DNA representing either the 447-52D heavy chain cDNA or the 447-52D light chain cDNA, 2.5 units of Taq DNA polymerase, PCR reaction components and buffer, and twenty-five (25) cycles of PCR amplification was performed (cycle period: 1', 94°C; 1', 55°C; 2' 72°C). The products of the five reactions, purified by agarose gel electrophoresis, were appropriately combined (10 ng of each DNA fragment) along with a terminal oligodeoxynucleotide primer pair (Figures 3 & 4), Taq DNA polymerase, PCR reaction components and buffer, and the subsequent recombined fragments were amplified by PCR, as described above, for twenty-five cycles. Following restriction endonuclease digestion of the signal and intron sequence-appended heavy chain V-region with HindIII and XhoI the amplified DNA was purified by agarose gel electrophoresis and cloned into compatible sites of a vector (p9103) which contained the human gamma 1 constant region, obtained as follows (shown in Figure 5). DNA was purified from the cosIg9 cosmid clone (Flanagan and Rabbits, Nature 300: 709-713, 1982) to act as template for PCR amplification of the human gamma 1 constant region. The appropriate primer pair (G1 & G2, Figure 1), 50 pmole each, was combined with 10 ng of cosmid DNA containing the human gamma 1 constant region exons, 2.5 units of Taq DNA polymerase, and twenty-five (25) cycles of PCR amplification was performed (cycle periods: 1', 94°C; 1', 55°C; 2' 72°C). The PCR product was digested with XhoI and EcoRI endonucleases, purified by agarose gel electrophoresis, and cloned into the p9102 vector previously digested with the aforementioned restriction enzymes. The individual clones representing the vector containing both the 447-52D heavy chain variable region derived as described above, and the human gamma 1 constant region, derived by PCR amplification of genomic DNA, were used to verify the DNA sequence of the recombinantly modified 447-52D encoding domain (Figure 2a).

The 447-52D human light chain recombinant vector was constructed following digestion of the PCR amplified signal and intron appended light chain V-region, described above, with HindIII and XbaI and purification of this DNA fragment by agarose gel electrophoresis. This V-region encoding DNA was cloned into pSP72/58.2/L16VH/γ1MRC (described below), which has been previously digested with HindIII and EcoRI, along with a DNA fragment representing a human light constant region exon. This latter fragment is obtained by PCR amplification of 10 ng of plasmid #208 DNA, containing and EcoRI/HindIII fragment encompassing the human lambda 2 constant region locus. The appropriate primer pair (C1 and C2, Figure 1), 50 pmole each, was combined with the plasmid DNA, 2.5 units of Taq DNA polymerase and twenty-five (25) cycles of PCR amplification was performed (cycle periods: 1', 94°C; 2', 55°C; 2', 72°C). The 620 basepair product of the reaction was digested with XbaI and EcoRI restriction enzymes and purified by agarose gel electrophoresis prior to its inclusion in the three piece ligation described above. Analysis of the resultant clones revealed the expected about 1.2 kilobase light chain encoding insert following their digestion with HindIII and EcoRI and subsequent agarose gel electrophoresis. DNA was obtained following growth of individual bacterial clones and submitted to DNA sequence determination using Sequenase® and, initially, T7 and SP6 specific sequencing primers in order to verify the sequence of the reconstructed variable region and its flanking lambda constant region domain (Figure 2b). Plasmid DNAs representing the 447-52D heavy and light chain expression vectors were grown (Maniatis et al., supra) and purified for transfection into recipient mammalian cells (Maniatis et al., supra; Birbion and Doly, supra.).

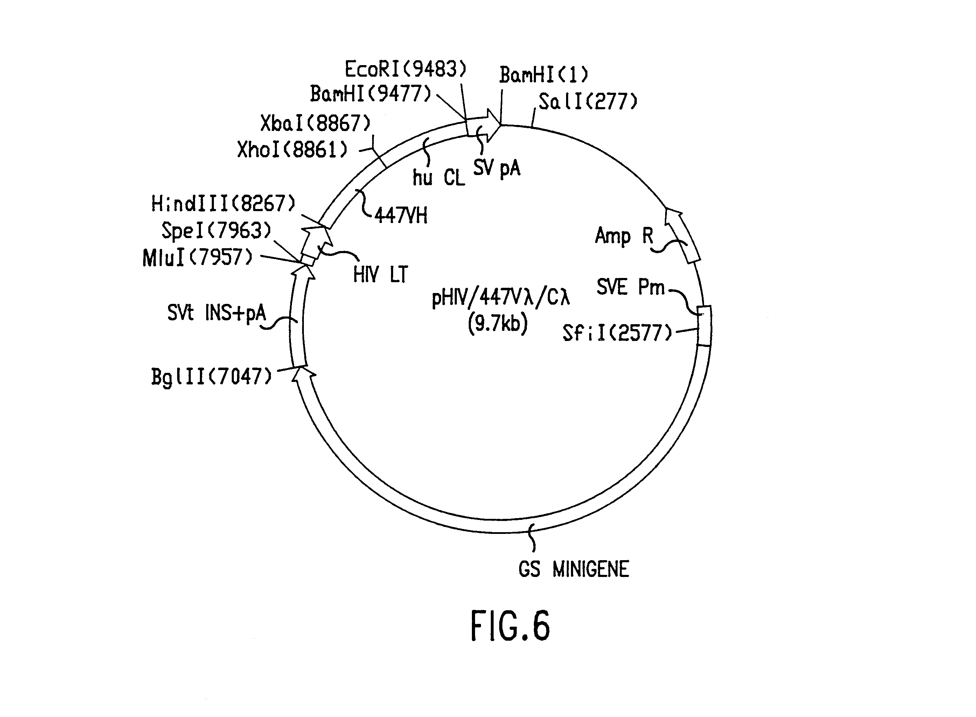

The heavy chain and light chain immunoglobulin molecules are transcribed from plasmids that are identical other than the fact that they contain different immunoglobulin encoding sequences. The preferred progenitor of the immunoglobulin expession vector is the one described above which contains a portion of the HIV-1 LTR promoter (-117 to +80, relative to the cap site), a multiple cloning site, and the SV40 late polyadenylation signal (Figure 6). Plasmid pEE14 (Celltech, Ltd.) is the origin of the majority of the DNA sequences within this vector, designated pSZ9015. The CMVIE promoter of the pEE14 vector was removed by digestion with MluI and HindIII. The 8 kb promoter-minus vector DNA was purified by agarose gel electrophoresis and ligated to an approximately 200 basepair PCR amplified DNA fragment containing 197 basepairs of the HIV LTR (-117 to +80) and MluI and HindIII termini (obtained using the primer pair described in Figure 1 and the REP3/HIV-LTR CDR-grafted 1B4-Vk Human-Ck/HygB plasmid DNA template described by DeMartino, J.A. et al., Antibody, Immunoconjugates, and Radiopharmaceuticals 4: 829-835, 1991). The heavy chain gamma 1 constant region had been placed in the p9015 vector as a XbaI/EcoRI 2.0 kb fragment along with an unrelated heavy chain V-region, creating vector pSP72/58.2/L16VH/γ1MRC . The plasmids were disignated pHIV447Vλ for the heavy chain-containing plasmid, and pHIV447Vλ for the light chain-containing plasmid. Both plasmids in their respective E. coli host have been deposited before the filing date of this application, with the American Type Culture Collection, 12301 Parklawn Drive, Rockville MD, without restriction as to availability and in accordance with the terms and requirements of the Budapest Treaty. The E. coli hosts containing the plasmids have been assigned the ATCC accession numbers 68945 for pHIV447VHCγ1 and 68943 for pHIV447Vλ/Cλ.

Equal amounts (10μg) of the plasmids encoding the heavy chain and the light chains were transfected by standard calcium phosphate precipitation procedures into human 293 cells (DeMartino, J.A. et al., supra.), except co-transfection with an HIV-1 TAT encoding plasmid was found not to be required for transactivation and resultant high level transcription of the HIV-1 LTR expression vector described herein. The culture supernatant fluids were assayed by trapping or solid-phase ELISAs (described below) for the secretion of a human lambda light chain containing IgG1 immunoglobulin.

ELISAs were developed for the quantitation of the amounts of 447-52D recombinant antibody expressed in conditioned mammalian cell growth medium. Immulon-2 (Dynatech Labs) 96-well plates are coated overnight with a 10μg/ml solution of mouse anti-human lambda chain constant domain monoclonal antibody (cat. #05-4101, Zymed Laboratories, Inc.) in phosphate-buffered saline (PBS) at 4°C, and blocked with 1% bovine serum albumin (BSA) in PBS for 1 hour at 37°C. After repeated washing with PBS, samples (conditioned medium containing recombinant 447-52D antibody or a human lambda/IgG1 standard antibody obtained from Sigma Chemical) diluted in PBS containing 1% BSA were added in duplicates and incubated for 1 hour at 37°C. Standard calibration curves were constructed using IgG1 concentrations ranging from 7.8 ng/ml to 500 ng/ml. Bound and fully assembled human IgG1 were detected with 50μl aliquots of a 1:400 dilution of mouse anti-human IgG1 Fc monoclonal antibody conjugated to horseradish peroxidase (cat #05-3320, Zymed Laboratories, Inc.) in (PBS) containing about 1% BSA. After incubation for 1 hour at 37°C and subsequent washing, the quantities of bound conjugate were detected by addition of 1mM 2,2'-azino-bis(3-ethylbenzthiazoline-6-sulfonic acid) in 0.1M sodium citrate, pH4.2, containing 0.03% hydrogen peroxide and incubation at room temperature for 20 minutes. The adsorbance of the wells was determined with a ELISA plate reader (Bio-Rad, Inc.) set at 415 nm. Alternatively, solid-phase ELISAs were carried out on plates coated with a 26-residue peptide based on the sequence of the MN isolate. The peptide (NleCSYNKRKRIHIGPGRAFYTTKNIIGCS SEQ.ID.NO.: 1) is synthesized by solid-phase Fmoc chemistry using preactivated pentafluorophenyl esters and hydroxybenzyltriazine activation. Immulon-2 plates were coated with 1 μg/ml peptide overnight, and blocked with 1% fetal bovine serum in PBS. Detection of bound 447-52D antibody is carried out as described above. The antibody secreted by the transfected human 293 cells following transient expression was subsequently purified by standard protein A chromatography (DeMartino, J.A. et al., supra.).

The concentration of recombinant 447-52D antibody was determined by the ELISAs described above, and tested for efficacy by demonstrating its capacity to neutralize the infectivity of unique serotypes of HIV-1.

The following assay was performed to quantitate the neutralization of cell-free HIV-1 virus infection as well as the inhibition of cell-to-cell spread by measuring cell survival after exposure of cultures to antibody and virus for 7-8 days. Two-fold serial dilutions of the antibody under test were made in cell growth medium (RPMI1640 + 10% fetal calf serum), and 100 μl volumes were placed in the wells of a 96-well dish (Costar, Corp.). 100 μl of virus stock (prepared from chronically infected H9 cells or from newly established chronically infected FDA/H9 cells; 3 day conditioned medium from the chronically infected cell population plated at a cell density of 2 x 10^5 cells/mL is clarified and 10 fold more than the last dilution of virus stock which kills all MT-4 cells in a 7 day assay is chosen as the challenge dose) was added to each well and the virus-antibody mixtures were incubated at 37°C for 1 hour. MT-4 cells were added to each well (1 x 10^4 cells/well) in 50 μl of culture medium and the dish was incubated for 7 days at 37°C at which time the endpoint was determined. The concentration of the last antibody dilution which prevented MT-4 cell killing is reported as the neutralization endpoint.

The results of the neutralization assays are shown in Table 9 and indicate that the potency of the recombinant human 447-52D antibody was equal to that of the human heterohybridoma-derived 447-52D antibody for each HIV-1 serotype investigated. This result shows that the recombinantly constructed antibody, expessed with human lambda and gamma 1 constant domains, has not been modified in such a way as to alter its interactions with the V3 PND loop and the recombinant antibody retains the biological activities of the native antibody.

## TABLE 9

### NEUTRALIZATION ENDPOINT (µg/mL) IN in vitro MT-4 CELL KILLING

| HIV-1 ISOLATE | RECOMBINANT 447 | HETEROHYBRIDOMA 447 |
|---|---|---|
| $III_B$ | 0.78 | 1.29* |
| MN | 0.19 | 0.37 |
| AL-1 | 0.09 | 0.15 |
| SF-2 | 0.04 | 0.04 |
| DU 6587-5 | 0.09 | 0.62 |
| DU 7887-7 | 0.37 | 0.78 |
| WMJ-2 | 0.78 | 1.35 |
| RF | nd | 0.62 |
| SF-162 | nd | 1.98+ |

* = Geometric mean titer
+ = Macrophage/monocyte primary cultures (2 x $10^5$ cells/microtiter well) were fed at days 5, 8, 12, 14, 21 with fresh medium. Conditioned medium was assayed for the presence of p24 virus core antigen by ELISA (Coulter Immunology, Hialiah, FL) and at day 24 the cells of each well were lysed and subjected to the same assay. The endpoint was determined to be the last dilution of antibody that prevented the appearance of p24 within the cell culture.
nd = not done.

EXAMPLE 10

An expression system was constructed and employed to produce large quantities of the recombinant 447 antibody. The expression system utilized the Cytomegalovirus immediate early (CMVIE) transcriptional promoter and the glutamine synthetase (GS) selection and amplification cassette, and is applicable to various different mammalian cell lines (Bebington et al., Biotechnology 10: 169-175, 1992). The basic vectors, pEE12 and pEE6, were obtained from Celltech, Ltd. and used to create a single plasmid, designated p63.79r447, which transcribed both the heavy and light chain immunoglobulin peptides, in addition to the GS gene product. The manner in which this r447 expression vector was constructed utilized existing basic vectors which contained other immunoglobulin sequences.

50 µg of DNA of the pHIV/447$V_H$/C$\gamma$1 plasmid (Figure 5), containing the V-region of the r447 antibody, was digested with the restriction endonucleases HindIII and XhoI. The approximately 800 basepair (bp) heavy chain 447 V-region fragment was purified by gel electrophoresis in 0.8% agarose (prepared in TAE; 40 mM TRIS base, 1 mM EDTA, pH 8.0 acetic acid to pH 7.4), the excised DNA fragment was placed in 3/4 inch diameter dialysis tubing (BRL) with a minimum volume of TAE, and the DNA was electroeluted by electrophoresis at 150 volts for 30 minutes. The resultant DNA solution was removed from the dialysis tubing and extracted twice with phenol/chloroform, then the DNA was concentrated by ethanol precipitation, and resuspended in TE (10 mM TRIS HCl, 1 mM EDTA, pH 8.0).

Similarly, 50 µg of DNA of the pEE12/58.2L16$V_H$/C$\gamma$1 plasmid, containing the CMVIE promoter, GS gene transcriptional unit, and ampicillin resistance marker, was digested with the restriction endonucleases HindIII and EcoRI. The 7087 bp pBR322-based vector DNA fragment was purified as described above. About 2 µg of this DNA fragment was combined with about 0.9 µg of the 800 bp DNA fragment from pHIV/447VH/C$\gamma$1 (described above), and one tenth volume of 10x ligation buffer (660 mM TRIS-HCl, 50 mM MgCl$_2$, 10 mM DTT, 10 mM ATP, pH 7.5) was added, plus about 25 units of $T_4$ DNA ligase (Boehringer Mannheim). The mixture (final DNA concentration of 28.9 ng/mL) was incubated at room temperature for 1 hour to allow for ligation of the DNA fragments, then following heat inactivation of the ligase (65°C for 5 minutes) the reaction was phenol/chloroform extracted and the DNA was concentrated by ethanol precipitation. The DNA was resuspended in $dH_2O$, and restriction endonucleases EcoRI and XhoI were added after adjusting the solution to the Manufacturer's recommended buffer concentration. Following incubation at 37°C for 1 hour, the digested DNA was electrophoresed on a 0.8% agarose gel, as described above. The desired 7887 bp ligated DNA fragment was excised from the gel and the DNA was electroeluted and concentrated by ethanol precipitation, as described above. The procedure, described as the LDP Cycle (Figure 8), in which DNA fragments are Ligated, restriction enzyme Digested, and Purified was employed to shorten the time normally required to construct complex plasmid vectors.

About 50 μg of each of two plasmid DNAs (pHIV/447$V_L$/Cλ and pEE12/58.2L16$V_H$/Cγ1), one containing the 1200 bp 447 lambda light chain sequence and the other containing the human immunoglobulin gamma 1 constant region and the CMVIE promoter within a 4158 bp DNA fragment, were digested with the restriction enzymes HindIII and EcoRI, or XhoI and HindIII, respectively. These two DNA fragments were purified following electrophoresis in a 0.8% agarose gel by electroelution as previously described. A three fragment DNA ligation was prepared using the above two DNA fragments (18.8 mn HindIII-EcoRI, respectively) and the 7887 bp DNA fragment obtained above (500 ng). The reaction, containing a final DNA concentration of 11.85 μg/mL and 5 units of $T_4$ DNA ligase in 1x ligation buffer (described above), was incubated at room temperature for 1 hour then diluted 1:5 prior to the use of 1 μl for the transformation of competent E. Coli strain HB101 (BRL), as described by the manufacturer. Transformants were selected from LB agar plates containing 50 μg/mL of ampicillin (Sigma) and DNA was extracted for evaluation of the presence of the desired recombinant plasmid sequences (see Figure 7). The expression plasmid designated p63.79r447 encoded the 447 gamma 1 heavy chain, 447 lambda light chain, GS selection cassette, and prokaryotic plasmid ampicillin selection and neighboring replication origin.

Vector pEE12/58.2L16$V_H$/Cγ1 (above) was constructed from the pEE12 vector described by Bebington et al. (Supra). Following digestion with HindIII and EcoRI the pEE12 vector and an intermediate vector (pSP72; Promega, Inc.) containing the heavy chain V-region of a humanized antibody 58.2, fused to a human gamma 1 constant region through an XbaI transform E. coli. Clones harboring the pEE12/58.2L16$V_H$/Cγ1t were identified. This plasmid DNA was subsequently cleaved with XhoI and EcoRI, separating the heavy chain Cγ1t-region from the heavy chain V-region with the bulk of the plasmid still attached. A shortened version of the human Cγ1 encoding domain was obtained by PCR amplification using the oligos G1 and G2, and the cosmid clone cosIg9 DNA as the template (Flanagan and Rabbits, Nature 300: 709-713, 1982). The appropriate primer pair (G1 & G2, Figure 1), 50 pmole each, was combined with 10 ng of cosmid DNA containing the human gamma 1 constant region exons, 2.5 units of Taq DNA polymerase and twenty-five (25) cycles of PCR amplification (cycle periods: 1', 94°C; 1', 55°C; 2' 72°C). The PCR product was digested with XhoI and EcoRI, purified by agarose gel electrophoresis, and cloned into the pEE12/58.2L16$V_H$ - containing portion of the vector. The individual clones representing the vector containing both the 447 heavy chain variable region derived as described above, and the shortened version of the human gamma 1 constant region, were identified and isolated.

The final plasmid p63.79r447 in its E. coli host, containing both the heavy chain and light chain coding regions of human monoclonal antibody 447, has been deposited before the filing date of this application, with the American Type Culture Collection, 12301 Parklawn Drive, Rockville, MD, without restriction as to availability and in accordance with the terms and requirements of the Budapest Treaty. The E. coli host containing plasmid p.63.79r447 has been assigned the ATCC accession number ATCC 68944.

EXAMPLE 11

Transfection of NS/0 Cells

NS/0 cells were maintained in exponental growth in the following medium: Iscove's Minimum essential medium supplemented with 10% heat inactivated Fetal Bovine Serum and 4 mM Glutamine; they were maintained at 37°C in a humidified incubator set at 5% to 6.5% $CO_2$.

The plasmid for transfection was linearized by digestion with a restriction enzyme at a unique site; the preferred unique site was one situated outside the foreign gene expression sequences, in the acterial sequences of the vector. After restriction, DNA was deproteinized by phenol extraction, phenol/chloroform (1:1) extraction and one final extraction with chloroform; it was then precipitated under sterile conditions in a biological safety cabinet, using a final concentration of 0.2-0.4 M sodium chloride and 70% ethanol. DNA was resuspended in sterile distilled water at a calculated 1 μg/1 μl concentration. DNA was either used immediately or frozen (-20°C) until use.

On the day of transfection, viable cell counts were taken for the stock NS/0 culture. A total of $1 \times 10^7$ viable cells were used per transfection cuvette. The cells were first collected by centrifugation at 3,000 x g for 5 minutes at room temperature; pelleted cells were then washed twice with sterile phosphate buffered saline (PBS) and resuspended in PBS at a concentration of $10^7$ cells per 800 μls. The cell suspension was maintained on ice from this point on. $10^7$ cells were transferred gently to a 0.4 cm (distance between electrodes) BioRad cuvette, under sterile conditions in a biological safety cabinet. 40 μg of linearized plasmid DNA in solution was mixed with the cells gently and the cuvette was kept on ice for 5 mintues. Before electroporation, the outside of the cuvette was wiped dry, then placed in the cuvette holder of a "BioRad Gene Pulser". The gene pulser was set to deliver 3 μF at 1500 volts per pulse. Two consecutive pulses were used. The cuvette was then placed on ice for 2-5 minutes and then the cells were transferred to 30 ml of modified growth medium containing 1 mM glutamine, rather than 4 mM glutamine, in a 50 ml disposable sterile tube. 10 ml of cell suspension out of 30 ml was distributed into one 96 well microtiter dish, approximately 100 μl per well; 10 ml of cell suspension, (from the remaining 20 ml) was diluted with 10 μl of modified growth medium and distributed to two 96 well microtiter dishes, approximately 100 μl per well; the final 10 ml of cell suspension was diluted with 30 ml of modified growth medium and distributed over four 96-well microtiter dishes at approximately 100 μl per well. These plates were incubated overnight in a humidified incubator set at 37°C with 5%-6.5% $CO_2$.

Selective Medium:

Selective medium was as follows:
Iscove's Minimum Essential Medium (Glutamine-free; Sigma)
10% Dialyzed Fetal Bovine Serum (from Hyclone)
1X Nucleosides∗
1X Asparagine∗∗

Selection:

24 hours post transfection each 96 well microtiter dish was fed with 100 μl of selective medium and incubated in a humidified incubator set at 37°C with 5% to 6.5% $CO_2$ until colonies come up, which takes approximately 3 to 3.5 weeks. No feeding was required unless wells begin to dry out; plates monitored at 3-4 day intervals. The wells with colonies growing eventually turn yellow and at this point those wells were assayed by removing 50 to 100 μl of supernatant and refeeding the wells with selective medium (to maintain viable clones).

EXAMPLE 12

In the first study, a purified preparation of HIV-1 neutralizing antibody was administered to a chimpanzee via the intravenous route, at a dose of 36 mg per kg. The animal was challenged 24 hours later with an intravenous administration of 75 chimpanzee infectious doses of the HIV-1 IIIb variant. The animal exhibited a circulating virus-neutralizing antibody titer of 1:320 at the time of challenge. An untreated control chimpanzee was simultaneously inoculated with virus.

Table 10 presents the results of the study. The control animal (x39) first presented signs of the virus infection at 3 weeks post-challenge, based on virus isolation from its peripheral blood mononuclear cells (PBMCs). This animal specifically seroconverted by 6 weeks post-challenge, and it has remained antibody and virus isolation positive during the 72 week observation period. In contrast, the antibody-treated animal (x289) has remained free from of signs of virus infection. Attempts to detect virus-specific nucleic acid in the animal's PBMCs by polymerase chain reaction (PCR) were negative.

∗50X Ribonucleosides Stock Solution:
　　35 mg adenosine
　　35 mg guanosine
　　35 mg cytidine
　　12 mg thymidine
(each from Sigma, cell culture grade) Make up to 100 ml with sterile distilled water. Filter sterilize through 0.1 μ filter unit and store frozen (-20°C) in 10 ml aliquots.
∗∗100X Asparagine: 600 mg per 100 ml of sterile distilled water, filter sterilize through an 0.1 μ filter unit and store at 4°C.

Table 10:    Protective Effect of Pre-Exposure Administration of V3
             Loop-Directed Monoclonal Antibody[a]

A. Control chimpanzee (x39)

| weeks. p.c.[b] | virus-neutralizing antibody titer[c] | anti-HIV 1 ELISA and western blot[d] | virus isolation[e] | virus specific PCR[f] |
|---|---|---|---|---|
| 0 | 10 | − | − | − |
| (1 day) | 10 | − | NDg | ND |
| 1 | 10 | − | − | − |
| 2 | 10 | − | − | − |
| 3 | 10 | − | + | + |
| 4 | 10 | − | + | + |
| 6 | 10 | + | + | + |
| 8 | 20 | + | + | + |
| 10 | 106 | + | + | + |
| 12 | 320 | + | + | + |
| 14 | 160 | + | + | + |
| 16 | ≥640 | + | + | + |
| 20 | ≥640 | + | + | + |
| 24 | ≥640 | + | + | + |
| 28 | ≥640 | + | + | + |
| 32 | ≥640 | + | + | + |
| 36 | ≥640 | + | + | + |
| 40 | ≥640 | + | + | + |
| 44 | ≥640 | + | + | + |
| 48 | ≥640 | + | + | + |
| 52 | ≥640 | + | + | + |
| 64 | ≥640 | + | + | + |
| 72 | ≥640 | + | + | + |

Table 10: (cont.)

B.  Pre-exposure antibody treated chimpanzee (x289)

| weeks. p.c.[b] | neutralizing antibody titer[c] | anti-HIV 1 ELISA and western blot[d] | virus isolation[e] | virus specific PCR[f] |
|---|---|---|---|---|
| 0 | <10 | – | – | – |
| (1 day) | 320 | +/– | ND[g] | ND |
| 1 | 320 | +/– | – | – |
| 2 | 80 | +/– | – | – |
| 3 | 20 | +/– | – | – |
| 4 | 10 | – | – | – |
| 6 | <100 | – | – | – |
| 8 | <10 | – | – | – |
| 10 | <10 | – | – | – |
| 12 | <10 | – | – | – |
| 14 | <10 | – | – | – |
| 16 | <10 | – | – | – |
| 20 | <10 | – | – | – |
| 24 | <10 | – | – | – |
| 28 | <10 | – | – | – |
| 32 | <10 | – | – | – |
| 36 | <10 | – | – | – |
| 40 | <10 | – | – | – |
| 44 | <10 | – | – | – |
| 48 | <10 | – | – | – |
| 52 | <10 | – | – | – |
| 64 | <10 | – | – | – |
| 72 | <10 | – | – | – |

Footnotes to Table 10

a

The Cβ1 antibody preparation was purified by protein A-affinity chromatography. The animals were challenged 1 day following adminsitration of the antibody to chimpanzee x289. The challenge virus was kindly provided by Larry Arthur and Peter Fischinger (National Cancer Institute) and was quantitated at the time of challenge as described by Emini et al.,

supra. The chimpanzees were maintained under conditions that met or exceeded the United States National Institutes of Health guidelines for the care of laboratory animals.

b

    p.c.: post-challenge

c

Virus-neutralizing antibody titer was measured in cell culture using the IIIb variant of HIV-1 as described by Robertson et al., [14]. The quantity of virus used in the assay was identical to the quantity used for chimpanzee challenge. The values represent the inverse of the highest serum dilution which yielded an effective virus neutralization as described.

d

Anti-HIV-1 ELISA and Western blot reactive antibodies were measured as described by Emini *et al.* (supra). (+) Positive ELISA reaction and multiple reactive Western blot bands. (+/-) Only gp120 reactive Western blot band, negative ELISA. (-) Negative ELISA and no Western blot reactive bands.

e

Virus isolations were performed by *in vitro* culture of chimpanzee PBMCs using four methods. (1) Chimpanzee PBMCs were co-cultured with an equal number of mitogen-activated human PBMCs in medium containing IL-2 and DEAE-dextran. (2) Chimpanzee PBMCs were activated by phytohemaglutinin (PHA) and then co-cultured per method 1. (3) Chimpanzee PBMCs were stimulated with PHA and were then cultured alone in IL-2 containing medium. (4) Chimpanzee PBMCs were co-cultured with an equal number of activated human PBMCs in medium supplemented with PHA and IL-2. In each case, cultures contained 1x10 chimpanzee cells in a volume of 10 ml. The cultures were incubated at 37°C in a 5% $CO_2$ atmosphere for a period of 4 weeks. Medium supernatants were assayed for HIV-1 p24 antigen production at weekly intervals. Virus growth by any of the methods was considered to be a positive virus isolation.

f

The presence of HIV-1 specific nucleic acid in chimpanzee PBMCs was assayed by polymerase chain reaction (PCR). DNA was obtained from PBMCs by incubation of cell samples in 10 mM Tris HCl, pH 8.3, 1.0 mM EDTA, pH 8.0, 0.5% SDS, 150 µg/ml proteinase K at 60° for 60 minutes. After phenol/chloroform extraction, the DNA was precipitated by ethanol. 1-3 µg of DNA was used for the PCR. Each reaction mixture contained, in addition to the DNA, 1.5 µM of DNA was used for the PCR. Each reaction mixture contained, in addition to the DNA, 1.5 mM $MgCL_2$, 10 mM Tris HCl, pH 8.3, 50 mM CKl, 0.032 mM of each dNTP, 50 ng of each primer and 1.0 unit Taq polymerase. The HIV-1 gag specific primer pair SK38/39 was used. 35 reaction cycles were performed; each cycle consisted of a 94°C incubation for 1.5 minutes and a 56°C incubation for 3.0 minutes. Following the reaction, the amplified product was detected by hybridization with SK19 probe. Sensitivity of the PCR assay was determined to be 10 HIV-1 specific copies per DNA sample.

g

ND: Not done.

EXAMPLE 13

Following this initial observation of protection (Example 10), a second study was done to assess the protective effect of an HIV-1 neutralizing antibody when administered after virus challenge. Two chimpanzees (x299 and x196) were each inoculated intravenously with 75 chimpanzee infectious doses of HIV-1 IIIb. Ten minutes post-inoculation, animal x299 was given an intravenous administration of the antibody at a dose of 36 mg/kg. The total volume (approx. 200 ml) was delivered in 30 mins. The circulating neutralizing antibody titer at the end of the administration was 1:640. Chimpanzee x196 was not treated.

The study's results are presented in table 11. The control chimpanzee (x196) first yielded a positive virus isolation from PBMCs at 6 weeks, post-challenge. The control animal seroconverted at 8 weeks and has remained virus isolation and antibody positive during the 48 week observation period. The antibody-treated chimpanzee (x299) has not presented any signs of persistent virus infection to date. PCR analyses for virus-specific nucleic acid in the animal's PBMCs have also been negative.

These observations provide the first direct evidence that anti-V3 loop, HIV-1 neutralizing antibody can, in the absence of other virus-specific immune responses, prevent the establishment of an HIV-1 persistent infection.

Table 11:     Protective Effect of Pre-Exposure Administration of
              V3 Loop Directed Monoclonal Antibody[a]

A.   Control chimpanzee (x196)

| weeks. p.c. | virus-neutralizing antibody titer | anti-HIV 1 ELISA and western blot | virus isolation | virus specific PCR |
|---|---|---|---|---|
| 0 | <10 | − | − | − |
| (1 day) | <10 | − | ND | ND |
| 1 | <10 | − | − | − |
| 2 | <10 | − | − | − |
| 3 | <10 | − | − | − |
| 4 | <10 | − | − | − |
| 6 | <10 | − | + | + |
| 8 | <10 | + | + | + |
| 10 | <10 | + | + | + |
| 12 | 10 | + | + | + |
| 14 | 20 | + | + | + |
| 16 | 80 | + | + | + |
| 18 | 80 | + | + | + |
| 20 | 160 | + | + | + |
| 24 | 320 | + | + | + |
| 28 | ≥640 | + | + | + |
| 32 | ≥640 | + | + | + |
| 36 | ≥640 | + | + | + |
| 40 | ≥640 | + | + | + |
| 44 | ≥640 | + | + | + |
| 48 | ≥640 | + | + | + |

EP 0 577 243 A2

Table 11 (cont.)

B. **Post-exposure antibody-treated chimpanzee (x299)**

| weeks. p.c. | virus-neutralizing antibody titer- | anti-HIV 1 ELISA and western blot- | virus isolation- | virus specific PCR- |
|---|---|---|---|---|
| 0 | 640[b] | +/-[b] | - | - |
| (1 day) | 320 | +/- | ND | ND |
| 1 | 320 | +/- | - | - |
| 2 | 160 | +/- | - | - |
| 3 | 80 | +/- | - | - |
| 4 | 20 | - | - | - |
| 6 | 10 | - | - | - |
| 8 | <10 | - | - | - |
| 10 | 10 | - | - | - |
| 12 | <10 | - | - | - |
| 14 | <10 | - | - | - |
| 16 | <10 | - | - | - |
| 18 | <10 | - | - | - |
| 20 | <10 | - | - | - |
| 24 | <10 | - | - | - |
| 28 | <10 | - | - | - |
| 32 | <10 | - | - | - |
| 36 | <10 | - | - | - |
| 40 | <10 | - | - | - |
| 44 | <10 | - | - | - |
| 48 | <10 | - | - | - |

Footnotes to Table 11

a

See footnotes to Table 1 for technique descriptions.

b

Antibody titers were determined using serum obtained immediately after Cβ1 adminstration (see text). The virus-neutralizing antibody titer prior to Cβ1 administration was <10.

EXAMPLE 14

HIV Isolation

Virus isolations were performed by in vitro culture of chimpanzee PBMCs using four methods. (1) Chimpanzee PBMCs were co-cultured with an equal number of mitogen-activated human PMBCs in medium containing IL-2 and DEAE-dextran. (2) Chimpanzee PBMCs were activated by phytohemaglutinin (PHA) and then co-cultured per method 1. (3) Chimpanzee PBMCs were stimulated with PHA and were then cultured alone in IL-2 containing medium. (4) Chimpanzee PBMCs were co-cultured with an equal number of activated human PBMCs in medium supplemented with PHA and IL-2. In each case, cultures contained $1x10^7$ chimpanzee cells in a volume of 10 ml. The cultures were incubated at 37°C in a 5% $CO_2$ atmosphere for a period of 4 weeks. Medium supernatants were assayed for HIV-1 p24 antigen production at weekly intervals. Virus growth by any of the methods was considered to be a positive virus isolation.

41

EXAMPLE 15

Detection of HIV Nucleic Acid by PCR

The presence of HIV-1 specific nucleic acid in chimpanzee PBMCs was assayed by polymerase chain reaction (PCR). DNA was obtained from PBMCs by incubation of cell samples in 10mM TRIS HCl, pH 8.3, 1.0 mM EDTA, pH 8.0, 0.5% SDS, 150 μg/ml proteinase K at 60° for 60 minutes. After phenol/chloroform extraction, the DNA was precipitated by ethanol. 1-3 μg of DNA was used for the PCR. Each reaction mixture contained, in addition to the DNA, 1.5 mM $MgCL_2$, 10 mM TRIS HCl, pH 8.3, 50 mM KCl, 0.032 mM of each dNTP, 50 ng of each primer and 1.0 unit Taq polymerase. The HIV-1 gag specific primer pair SK38/39 was used. 35 reaction cycles were performed; each cycle consisted of a 94°C incubation for 1.5 minutes. and a 56°C incubation for 3.0 minutes. Following the reaction, the amplified product was detected by hybridization with the SK19 probe. Sensitivity of the PCR assay was determined to be 10 HIV-1 specific copies per DNA sample.

EXAMPLE 16

Neutralization of HIV-1 Infectivity in-vitro by Human Monoclonal Antibody

For neutralization tests in MT-4 cells of lymphotrophic and lymphocytolytic isolates, 2-fold serial dilutions of antibodies were made and 100 μL volumes were used in each test well. 100 μL of virus stock dilution was added to each test well and the virus-antibody mixtures were incubated at 37°C for 1 hour after which $1 \times 10^4$ MT-4 cells in 50 μL of culture medium were added to each well. The cultures were incubated for 7 days when the antibody neutralization endpoint was determined. The neutralization endpoints were determined as the last dilution of the antibody preparation that prevented MT-4 cell killing. Uninfected MT-4 cells were cultured with each test and a virus stock retitration was performed with each analysis. Virus neutralization tests of the macrophage-monocytropic isolate SF-162 were performed in primary macrophage-monocyte cultures. A 1/10 dilution of a SF-162 stock, was mixed with 2-fold serial dilutions of antibodies, incubated for 1 hr at 37°C, and each mixture added to a microtiter plate well containing $2.0 \times 10^5$ cells. A human positive sera, with a significant neutralization titer was run with each test as well as a non-neutralizing negative human sera and cells infected in the absence of antibody or serum. All macrophage/monocyte cultures were fed with fresh medium at days 5, 8, 12, 14 and 21, post infection. Medium was collected at days 18, 21 and 24 for p24 ELISA (Coulter Immunology) in order to determine the production of virus. In addition, the infected macrophage/monocyte cells were lysed at day 24 for the determination of intracellular virus by p24 ELISA. The neutralization endpoint was determined as the last dilution of the antibody preparation that prevented macrophage/monocyte infection in vitro as evidenced by the absence of p24 production. The results are shown in Table 12.

Table 12:

| HIV-1 Isolates | SEQ. ID. NO.: | V3 Loop Sequence | Neutralization Endpoint (ug/ml), Gemetric Mean and (range) In Vitro MT-4 Cell Killing Assay |
|---|---|---|---|
| IIIB | 27 | TRKSIRIQRGPGRAFVTIGKIG | 1.29 (0.39-1.56) |
| MN | 28 | YNKRKRIHIGPGRAFYTTKNII | 0.37 (0.04-0.78) |
| AL-1 | 29 | IYRKGRIHIGPGRAFHTTRQII | 0.15 (0.09-0.19) |
| SF-2 | 30 | NNTRKSIYIGPGRAFHTTGRII | 0.04 (0.04) |
| DU 6587-5 | 31 | SNVRNRIHIGPGRAFHTTKRIT | 0.62 (0.39-0.78) |
| WMJ-2 | 32 | NNVRRSLSIGPGRAFRTREIIG | 1.35 (0.78-1.56) |
| DU 7887-7 | 33 | NNTSRGIRIGPGRAILATERII | 0.78 (0.78) |
| RF | 34 | NNTRKSITKGPGRVIYATGQII | 0.62 (0.39-0.78) |

| | | | Neutralization Endpoint in Monocyte/Macrophage Culture |
|---|---|---|---|
| SF-162 | 35 | NNTRKSITIGPGRAFYATGDII | 1.98 (1.25-2.50) |

SEQUENCE LISTING

(1) GENERAL INFORMATION:

    (i) APPLICANT:
        (A) NAME: Merck & Co., Inc.
        (B) STREET: 126 E. Lincoln Ave
        (C) CITY: Rahway
        (D) STATE: New Jersey
        (E) COUNTRY: USA
        (F) ZIP: 07065

    (ii) TITLE OF INVENTION: Recombinant Human HIV-Neutralizing Monoclonal Antibodies for Prevention and Treatment of HIV Infection

    (iii) NUMBER OF SEQUENCES: 59

    (iv) CORRESPONDENCE ADDRESS:

    (v) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25

    (vi) CURRENT APPLICATION DATA:
        (A) APPLICATION NUMBER:
        (B) FILING DATE:
        (C) CLASSIFICATION:

(2) INFORMATION FOR SEQ ID NO:1:

    (i) SEQUENCE CHARACTERISTICS:
        (A) LENGTH: 24 amino acids
        (B) TYPE: amino acid
        (C) STRANDEDNESS: single
        (D) TOPOLOGY: linear

    (ii) MOLECULE TYPE: peptide

    (ix) FEATURE:
        (B) LOCATION: 1
        (D) OTHER INFORMATION: Leu is Nle

    (xi) SEQUENCE DESCRIPTION: SEQ ID NO:1:

Leu Cys Tyr Asn Lys Arg Lys Arg Ile Gly Pro Gly Arg Ala Phe Tyr
1               5                   10                  15
Thr Thr Lys Asn Ile Ile Gly Cys
                20

(2) INFORMATION FOR SEQ ID NO:2:

    (i) SEQUENCE CHARACTERISTICS:
        (A) LENGTH: 23 amino acids
        (B) TYPE: amino acid
        (C) STRANDEDNESS: single
        (D) TOPOLOGY: linear

(xi) SEQUENCE DESCRIPTION: SEQ ID NO:2:

Tyr Asn Lys Arg Lys Arg Ile His Ile Gly Pro Gly Arg Ala Phe Tyr
1            5                  10                 15

Thr Thr Lys Asn Ile Ile Gly
            20

(2)  INFORMATION FOR SEQ ID NO:3:

  (i)  SEQUENCE CHARACTERISTICS:
      (A)  LENGTH: 8 amino acids
      (B)  TYPE: amino acid
      (C)  STRANDEDNESS: single
      (D)  TOPOLOGY: linear

(ii) MOLECULE TYPE: peptide

(xi) SEQUENCE DESCRIPTION: SEQ ID NO:3:

Pro Gly Arg Ala Phe Tyr Thr Thr
1               5

(2) INFORMATION FOR SEQ ID NO:4:

    (i) SEQUENCE CHARACTERISTICS:
        (A) LENGTH: 7 amino acids
        (B) TYPE: amino acid
        (C) STRANDEDNESS: single
        (D) TOPOLOGY: linear

    (ii) MOLECULE TYPE: peptide

(xi) SEQUENCE DESCRIPTION: SEQ ID NO:4:

Arg Lys Arg Ile His Ile Gly
1               5

(2) INFORMATION FOR SEQ ID NO:5:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide

(xi) SEQUENCE DESCRIPTION: SEQ ID NO:5:

His Ile Gly Pro Gly Arg
1               5

(2) INFORMATION FOR SEQ ID NO:6:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:6:

Tyr Asn Lys Arg Lys Arg
1               5

(2) INFORMATION FOR SEQ ID NO:7:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:7:

Asn Lys Arg Lys Arg Ile
1               5

(2) INFORMATION FOR SEQ ID NO:8:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide

(xi) SEQUENCE DESCRIPTION: SEQ ID NO:8:

Lys Arg Lys Arg Ile His
1               5

(2) INFORMATION FOR SEQ ID NO:9:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:9:

Arg Lys Arg Ile His Ile
1               5

(2) INFORMATION FOR SEQ ID NO:10:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:10:

Lys Arg Ile His Ile Gly
1               5

(2) INFORMATION FOR SEQ ID NO:11:

    (i) SEQUENCE CHARACTERISTICS:
        (A) LENGTH: 6 amino acids
        (B) TYPE: amino acid
        (C) STRANDEDNESS: single
        (D) TOPOLOGY: linear

(ii) MOLECULE TYPE: peptide

(xi) SEQUENCE DESCRIPTION: SEQ ID NO:11:

Arg Ile His Ile Gly Pro
1               5

(2) INFORMATION FOR SEQ ID NO:12:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:12:

    Ile His Ile Gly Pro Gly
    1               5

(2) INFORMATION FOR SEQ ID NO:13:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:13:

    His Ile Gly Pro Gly Arg
    1               5

(2) INFORMATION FOR SEQ ID NO:14:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:14:

    Ile Gly Pro Gly Arg Ala
    1            5

(2) INFORMATION FOR SEQ ID NO:15:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:15:

    Gly Pro Gly Arg Ala Phe
    1            5

(2) INFORMATION FOR SEQ ID NO:16:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide

51

(xi) SEQUENCE DESCRIPTION: SEQ ID NO:16:

Pro Gly Arg Ala Phe Tyr
1               5


(2) INFORMATION FOR SEQ ID NO:17:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide


(xi) SEQUENCE DESCRIPTION: SEQ ID NO:17:

Gly Arg Ala Phe Tyr Thr
1               5

(2) INFORMATION FOR SEQ ID NO:18:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide


(xi) SEQUENCE DESCRIPTION: SEQ ID NO:18:

Arg Ala Phe Tyr Thr Thr
1               5

(2) INFORMATION FOR SEQ ID NO:19:

    (i) SEQUENCE CHARACTERISTICS:
        (A) LENGTH: 6 amino acids
        (B) TYPE: amino acid
        (C) STRANDEDNESS: single
        (D) TOPOLOGY: linear

(ii) MOLECULE TYPE: peptide


(xi) SEQUENCE DESCRIPTION: SEQ ID NO:19:

Ala Phe Tyr Thr Thr Lys
1               5

(2) INFORMATION FOR SEQ ID NO:20:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide


    (xi) SEQUENCE DESCRIPTION: SEQ ID NO:20:

    Phe Tyr Thr Thr Lys Asn
    1               5

(2) INFORMATION FOR SEQ ID NO:21:

    (i) SEQUENCE CHARACTERISTICS:
        (A) LENGTH: 6 amino acids
        (B) TYPE: amino acid
        (C) STRANDEDNESS: single
        (D) TOPOLOGY: linear

    (ii) MOLECULE TYPE: peptide


    (xi) SEQUENCE DESCRIPTION: SEQ ID NO:21:

    Tyr Thr Thr Lys Asn Ile
    1               5

(2) INFORMATION FOR SEQ ID NO:22:

    (i) SEQUENCE CHARACTERISTICS:
       (A) LENGTH: 6 amino acids
       (B) TYPE: amino acid
       (C) STRANDEDNESS: single
       (D) TOPOLOGY: linear

    (ii) MOLECULE TYPE: peptide

    (xi) SEQUENCE DESCRIPTION: SEQ ID NO:22:

    Thr Thr Lys Asn Ile Ile
    1            5

(2) INFORMATION FOR SEQ ID NO:23:

    (i) SEQUENCE CHARACTERISTICS:
       (A) LENGTH: 6 amino acids
       (B) TYPE: amino acid
       (C) STRANDEDNESS: single
       (D) TOPOLOGY: linear

    (ii) MOLECULE TYPE: peptide

    (xi) SEQUENCE DESCRIPTION: SEQ ID NO:23:

    Thr Lys Asn Ile Ile Gly
    1            5

(2) INFORMATION FOR SEQ ID NO:24:

    (i) SEQUENCE CHARACTERISTICS:
       (A) LENGTH: 5 amino acids
       (B) TYPE: amino acid
       (C) STRANDEDNESS: single
       (D) TOPOLOGY: linear

    (ii) MOLECULE TYPE: peptide

(xi) SEQUENCE DESCRIPTION: SEQ ID NO:24:

Lys Arg Ile His Ile
1               5

(2) INFORMATION FOR SEQ ID NO:25:

    (i) SEQUENCE CHARACTERISTICS:
        (A) LENGTH: 4 amino acids
        (B) TYPE: amino acid
        (C) STRANDEDNESS: single
        (D) TOPOLOGY: linear

    (ii) MOLECULE TYPE: peptide

(xi) SEQUENCE DESCRIPTION: SEQ ID NO:25:

Gly Pro Gly Arg
1

(2) INFORMATION FOR SEQ ID NO:26:

    (i) SEQUENCE CHARACTERISTICS:
        (A) LENGTH: 15 amino acids
        (B) TYPE: amino acid
        (C) STRANDEDNESS: single
        (D) TOPOLOGY: linear

    (ii) MOLECULE TYPE: peptide

(xi) SEQUENCE DESCRIPTION: SEQ ID NO:26:

Arg Lys Arg Ile His Ile Gly Pro Gly Arg Ala Phe Tyr Thr Thr
1               5                   10                  15

(2) INFORMATION FOR SEQ ID NO:27:

    (i) SEQUENCE CHARACTERISTICS:
        (A) LENGTH: 22 amino acids
        (B) TYPE: amino acid
        (C) STRANDEDNESS: single
        (D) TOPOLOGY: linear

(ii) MOLECULE TYPE: peptide

(xi) SEQUENCE DESCRIPTION: SEQ ID NO:27:

Thr Arg Lys Ser Ile Arg Ile Gln Arg Gly Pro Gly Arg Ala Phe Val
1               5                   10                  15

Thr Ile Gly Lys Ile Gly
            20

(2) INFORMATION FOR SEQ ID NO:28:

    (i) SEQUENCE CHARACTERISTICS:
        (A) LENGTH: 22 amino acids
        (B) TYPE: amino acid
        (C) STRANDEDNESS: single
        (D) TOPOLOGY: linear

    (ii) MOLECULE TYPE: peptide

(xi) SEQUENCE DESCRIPTION: SEQ ID NO:28:

Tyr Asn Lys Arg Lys Arg Ile His Ile Gly Pro Gly Arg Ala Phe Tyr
1               5                   10                  15

Thr Thr Lys Asn Ile Ile
            20

(2) INFORMATION FOR SEQ ID NO:29:

    (i) SEQUENCE CHARACTERISTICS:
        (A) LENGTH: 22 amino acids
        (B) TYPE: amino acid
        (C) STRANDEDNESS: single
        (D) TOPOLOGY: linear

    (ii) MOLECULE TYPE: peptide

(xi) SEQUENCE DESCRIPTION: SEQ ID NO:29:

```
Ile Tyr Arg Lys Gly Arg Ile His Ile Gly Pro Gly Arg Ala Phe His
1               5                   10                  15

Thr Thr Arg Gln Ile Ile
                20
```

(2) INFORMATION FOR SEQ ID NO:30:

    (i) SEQUENCE CHARACTERISTICS:
        (A) LENGTH: 22 amino acids
        (B) TYPE: amino acid
        (C) STRANDEDNESS: single
        (D) TOPOLOGY: linear

    (ii) MOLECULE TYPE: peptide

(xi) SEQUENCE DESCRIPTION: SEQ ID NO:30:

```
Asn Asn Thr Arg Lys Ser Ile Tyr Ile Gly Pro Gly Arg Ala Phe His
1               5                   10                  15

Thr Thr Gly Arg Ile Ile
                20
```

(2) INFORMATION FOR SEQ ID NO:31:

    (i) SEQUENCE CHARACTERISTICS:
        (A) LENGTH: 22 amino acids
        (B) TYPE: amino acid
        (C) STRANDEDNESS: single
        (D) TOPOLOGY: linear

    (ii) MOLECULE TYPE: peptide

(xi) SEQUENCE DESCRIPTION: SEQ ID NO:31:

```
Ser Asn Val Arg Asn Arg Ile His Ile Gly Pro Gly Arg Ala Phe His
1               5                   10                  15

Thr Thr Lys Arg Ile Thr
                20
```

(2) INFORMATION FOR SEQ ID NO:32:

    (i) SEQUENCE CHARACTERISTICS:
       (A) LENGTH: 22 amino acids
       (B) TYPE: amino acid
       (C) STRANDEDNESS: single
       (D) TOPOLOGY: linear

    (ii) MOLECULE TYPE: peptide

    (xi) SEQUENCE DESCRIPTION: SEQ ID NO:32:

Asn Asn Val Arg Arg Ser Leu Ser Ile Gly Pro Gly Arg Ala Phe Arg
1            5                10               15

Thr Arg Glu Ile Ile Gly
          20

(2) INFORMATION FOR SEQ ID NO:33:

    (i) SEQUENCE CHARACTERISTICS:
       (A) LENGTH: 22 amino acids
       (B) TYPE: amino acid
       (C) STRANDEDNESS: single
       (D) TOPOLOGY: linear

    (ii) MOLECULE TYPE: peptide

    (xi) SEQUENCE DESCRIPTION: SEQ ID NO:33:

Asn Asn Thr Ser Arg Gly Ile Arg Ile Gly Pro Gly Arg Ala Ile Leu
1            5                10               15

Ala Thr Glu Arg Ile Ile
          20

(2) INFORMATION FOR SEQ ID NO:34:

    (i) SEQUENCE CHARACTERISTICS:
        (A) LENGTH: 22 amino acids
        (B) TYPE: amino acid
        (C) STRANDEDNESS: single
        (D) TOPOLOGY: linear

    (ii) MOLECULE TYPE: peptide


    (xi) SEQUENCE DESCRIPTION: SEQ ID NO:34:

Asn Asn Thr Arg Lys Ser Ile Thr Lys Gly Pro Gly Arg Val Ile Tyr
1               5                   10                  15

Ala Thr Gly Gln Ile Ile
                20

(2) INFORMATION FOR SEQ ID NO:35:

    (i) SEQUENCE CHARACTERISTICS:
        (A) LENGTH: 22 amino acids
        (B) TYPE: amino acid
        (C) STRANDEDNESS: single
        (D) TOPOLOGY: linear

    (ii) MOLECULE TYPE: peptide


    (xi) SEQUENCE DESCRIPTION: SEQ ID NO:35:

Asn Asn Thr Arg Lys Ser Ile Thr Ile Gly Pro Gly Arg Ala Phe Tyr
1               5                   10                  15

Ala Thr Gly Asp IIe Ile
                20

(2) INFORMATION FOR SEQ ID NO:36:

    (i) SEQUENCE CHARACTERISTICS:
        (A) LENGTH: 30 base pairs
        (B) TYPE: nucleic acid
        (C) STRANDEDNESS: single
        (D) TOPOLOGY: linear

(ii) MOLECULE TYPE: cDNA

(xi) SEQUENCE DESCRIPTION: SEQ ID NO:36:

CGGAATTCAG GTGCAGCTGC AGCAGTCTGG 30

(2) INFORMATION FOR SEQ ID NO:37:

    (i) SEQUENCE CHARACTERISTICS:
        (A) LENGTH: 37 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:37:

CCGAATTCGC GGCCGCACTC ATTTACCCGG AGACAGG 37

(2) INFORMATION FOR SEQ ID NO:38:

    (i) SEQUENCE CHARACTERISTICS:
        (A) LENGTH: 42 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:38:

GGCGTCGACT CACCATGGCC GGCTCCCCTC TCYTCCTCACC C 42

(2) INFORMATION FOR SEQ ID NO:39:

    (i) SEQUENCE CHARACTERISTICS:
        (A) LENGTH: 34 base pairs
        (B) TYPE: nucleic acid
        (C) STRANDEDNESS: single
        (D) TOPOLOGY: linear

(ii) MOLECULE TYPE: cDNA

(xi) SEQUENCE DESCRIPTION: SEQ ID NO:39:

CCGAATTCGC GGCCGCCTAT GAACATTCTG TAGG 34

(2) INFORMATION FOR SEQ ID NO:40:

    (i) SEQUENCE CHARACTERISTICS:
        (A) LENGTH: 39 base pairs
        (B) TYPE: nucleic acid
        (C) STRANDEDNESS: single
        (D) TOPOLOGY: linear

    (ii) MOLECULE TYPE: cDNA

(xi) SEQUENCE DESCRIPTION: SEQ ID NO:40:

GATCCTCGAG GATTCTAGAA GGGTCAGATG TCGGTGTTG 39

(2) INFORMATION FOR SEQ ID NO:41:

    (i) SEQUENCE CHARACTERISTICS:
        (A) LENGTH: 30 base pairs
        (B) TYPE: nucleic acid
        (C) STRANDEDNESS: single
        (D) TOPOLOGY: linear

    (ii) MOLECULE TYPE: cDNA

(xi) SEQUENCE DESCRIPTION: SEQ ID NO:41:

GATCGAATTC CTGGGATCCT GCAGCTCTAG 30

(2) INFORMATION FOR SEQ ID NO:42:

    (i) SEQUENCE CHARACTERISTICS:
        (A) LENGTH: 43 base pairs
        (B) TYPE: nucleic acid
        (C) STRANDEDNESS: single
        (D) TOPOLOGY: linear

(ii) MOLECULE TYPE: cDNA


(xi) SEQUENCE DESCRIPTION: SEQ ID NO:42:

TTACTCGAGA CGCGTACTAG TGAGCTTGCT ACAAGGGACT TTC                  43

(2) INFORMATION FOR SEQ ID NO:43:

    (i) SEQUENCE CHARACTERISTICS:
        (A) LENGTH: 30 base pairs
        (B) TYPE: nucleic acid
        (C) STRANDEDNESS: single
        (D) TOPOLOGY: linear

    (ii) MOLECULE TYPE: cDNA


(xi) SEQUENCE DESCRIPTION: SEQ ID NO:43:

ATTTCTAGAA AGCTTTATTG AGGCTTAAGC                                 30

(2) INFORMATION FOR SEQ ID NO:44:

    (i) SEQUENCE CHARACTERISTICS:
        (A) LENGTH: 30 base pairs
        (B) TYPE: nucleic acid
        (C) STRANDEDNESS: single
        (D) TOPOLOGY: linear

    (ii) MOLECULE TYPE: cDNA


(xi) SEQUENCE DESCRIPTION: SEQ ID NO:44:

TATACTCGAG CAGACACTGG ACGCTGAACC                                 30

(2) INFORMATION FOR SEQ ID NO:45:

    (i) SEQUENCE CHARACTERISTICS:
        (A) LENGTH: 35 base pairs
        (B) TYPE: nucleic acid
        (C) STRANDEDNESS: single
        (D) TOPOLOGY: linear

(ii) MOLECULE TYPE: cDNA

(xi) SEQUENCE DESCRIPTION: SEQ ID NO:45:

TATATGATCA GAATTCGCCC GGGAAGTATG TACAG                                    35

(2) INFORMATION FOR SEQ ID NO:46:

    (i) SEQUENCE CHARACTERISTICS:
       (A) LENGTH: 1380 base pairs
       (B) TYPE: nucleic acid
       (C) STRANDEDNESS: single
       (D) TOPOLOGY: linear

    (ii) MOLECULE TYPE: cDNA

    (xi) SEQUENCE DESCRIPTION: SEQ ID NO:46:

GAGGTGCAGC TGGTGGAGTC TGGGGGAGGC TTGGTAAAGC CTGGGGGGTC CCTCAGACTC          60

ACCTGTGTAG CCTCTGGTTT CACGTTCAGT GATGTCTGGC TGAACTGGGT CCGCCAGGCT         120

CCAGGGAAGG GGCTGGAGTG GGTCGGCCGT ATTAAAAGCA GAACTGATGG TGGGACAACA         180

GACTACGCTG CATCCGTGAA AGGCAGATTC ACCATCTCAA GAGATGACTC AAAAAACACG         240

CTATATCTGC AAATGAATAG CCTGAAAACA GAGGACACAG CCGTTTATTC CTGCACCACA         300

GATGGTTTTA TTATGATTCG GGGAGTCTCC GAGGACTACT ACTACTACTA CATGGACGTT         360

TGGGGCAAAG GGACCACGGT CACCGTGAGC TCAGCCTCCA CCAAGGGCCC ATCGGTCTTC         420

CCCCTGGCAC CCTCCTCCAA GAGCACCTCT GGGGGCACAG CGGCCCTGGG CTGCCTGGTC         480

AAGGACTACT TCCCCGAACC GGTGACGGTG TCGTGGAACT CAGGCGCCCT GACCAGCGGC         540

GTGCACACCT TCCCGGCTGT CCTACAGTCC TCAGGACTCT ACTCCCTCAG CAGCGTGGTG         600

ACCGTGCCCT CCAGCAGCTT GGGCACCCAG ACCTACATCT GCAACGTGAA TCACAAGCCC         660

AGCAACACCA AGGTGGACAA GAAAGTTGAG CCCAAATCTT GTGACAAAAC TCACACATGC         720

```
CCACCGTGCC CAGCACCTGA ACTCCTGGGG GGACCGTCAG TCTTCCTCTT CCCCCCAAAA    780

CCCAAGGACA CCCTCATGAT CTCCCGGACC CCTGAGGTCA CATGCGTGGT GGTGGACGTG    840

AGCCACGAAG ACCCTGAGGT CAAGTTCAAC TGGTACGTGG ACGGCGTGGA GGTGCATAAT    900

GCCAAGACAA AGCCGCGGGA GGAGCAGTAC AACAGCACGT ACCGTGTGGT CAGCGTCCTC    960

ACCGTCCTGC ACCAGGACTG GCTGAATGGC AAGGAGTACA AGTGCAAGGT CTCCAACAAA   1020

GCCCTCCCAG CCCCCATCGA GAAAACCATC TCCAAAGCCA AAGGGCAGCC CCGAGAACCA   1080

CAGGTGTACA CCCTGCCCCC ATCCCGGGAT GAGCTGACCA AGAACCAGGT CAGCCTGACC   1140

TGCCTGGTCA AAGGCTTCTA TCCCAGCGAC ATCGCCGTGG AGTGGGAGAG CAATGGGCAG   1200

CCGGAGAACA ACTACAAGAC CACGCCTCCC GTGCTGGACT CCGACGGCTC CTTCTTCCTC   1260

TACAGCAAGC TCACCGTGGA CAAGAGCAGG TGGCAGCAGG GGAACGTCTT CTCATGCTCC   1320

GTGATGCATG AGGCTCTGCA CAACCACTAC ACGCAGAAGA GCCTCTCCCT GTCTCCGGGT   1380
```

(2) INFORMATION FOR SEQ ID NO:47:

    (i) SEQUENCE CHARACTERISTICS:
        (A) LENGTH: 654 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:47:

```
CAGTCTGTGT TGACGCAGCC GCCCTCAGTG TCTGCGGCCC CAGGACAGAA GGTCACCATC     60

TCCTGCTCTG GAAGCAGCTC CAACATTGGG AATAATTATG TATTGTGGTA CCAGCAGTTC    120

CCAGGAACAG CCCCCAAACT CCTCATTTAT GGCAATAATA AGCGACCCTC AGGGATTCCT    180

GACCGATTCT CTGGCTCCAA GTCTGGCACG TCAGCCACCC TGGGCATCAC CGGACTCCAG    240

ACTGGGGACG AGGCCGATTA TTTCTGCGCA ACATGGGATA GCGGCCTGAG TGCTGATTGG    300
```

GTGTTCGGCG GAGGGACCAA GCTGACCGTC CTAAGTCAGC CCAAGGCTGC CCCCTCGGTC          360

ACTCTGTTCC CGCCCTCCTC TGAGGAGCTT CAAGCCAACA AGGCCACACT GGTGTGTCTC          420

ATAAGTGACT TCTACCCGGG AGCCGTGACA GTGGCCTGGA AGGCAGATAG CAGCCCCGTC          480

AAGGCGGGAG TGGAGACCAC CACACCCTCC AAACAAAGCA ACAACAAGTA CGCGGCCAGC          540

AGCTATCTGA GCCTGACGCC TGAGCAGTGG AAGTCCCACA GAAGCTACAG CTGCCAGGTC          600

ACGCATGAAG GGAGCACCGT GGAGAAGACA GTGGCCCCTA CAGAATGTTC ATAG               654

(2) INFORMATION FOR SEQ ID NO:48:

    (i) SEQUENCE CHARACTERISTICS:
       (A) LENGTH: 54 base pairs
       (B) TYPE: nucleic acid
       (C) STRANDEDNESS: single
       (D) TOPOLOGY: linear

   (ii) MOLECULE TYPE: cDNA

   (xi) SEQUENCE DESCRIPTION: SEQ ID NO:48:

CATTCGCTTA CCCTGCAGAA GCTTGTTGAC TAGTGAGATC ACAGTTCTCT CTAC               54

(2) INFORMATION FOR SEQ ID NO:49:

    (i) SEQUENCE CHARACTERISTICS:
       (A) LENGTH: 19 base pairs
       (B) TYPE: nucleic acid
       (C) STRANDEDNESS: single
       (D) TOPOLOGY: linear

   (ii) MOLECULE TYPE: cDNA

   (xi) SEQUENCE DESCRIPTION: SEQ ID NO:49:

GGAGTGGACA CCTGTGGAG                                                      19

(2) INFORMATION FOR SEQ ID NO:50:

    (i) SEQUENCE CHARACTERISTICS:
       (A) LENGTH: 19 base pairs
       (B) TYPE: nucleic acid
       (C) STRANDEDNESS: single
       (D) TOPOLOGY: linear

    (ii) MOLECULE TYPE: cDNA


    (xi) SEQUENCE DESCRIPTION: SEQ ID NO:50:

GGTGAGTCCT TACAACCTC                             19

(2) INFORMATION FOR SEQ ID NO:51:

    (i) SEQUENCE CHARACTERISTICS:
       (A) LENGTH: 44 base pairs
       (B) TYPE: nucleic acid
       (C) STRANDEDNESS: single
       (D) TOPOLOGY: linear

    (ii) MOLECULE TYPE: cDNA


    (xi) SEQUENCE DESCRIPTION: SEQ ID NO:51:

GAATGTGCCT ACTTTCTAGA CTCGAGTATA AATCTCTGGC CATG            44

(2) INFORMATION FOR SEQ ID NO:52:

    (i) SEQUENCE CHARACTERISTICS:
       (A) LENGTH: 39 base pairs
       (B) TYPE: nucleic acid
       (C) STRANDEDNESS: single
       (D) TOPOLOGY: linear

    (ii) MOLECULE TYPE: cDNA

(xi) SEQUENCE DESCRIPTION: SEQ ID NO:52:

CTCCACAGGT GTCCACTCCG AGGTGCAGCT GGTGGAGTC　　　　　　　　　39

(2) INFORMATION FOR SEQ ID NO:53:

    (i) SEQUENCE CHARACTERISTICS:
        (A) LENGTH: 39 base pairs
        (B) TYPE: nucleic acid
        (C) STRANDEDNESS: single
        (D) TOPOLOGY: linear

    (ii) MOLECULE TYPE: cDNA

(xi) SEQUENCE DESCRIPTION: SEQ ID NO:53:

GAGGTTGTAA GGACTCACCT GAGCTCACGG TGACCGTGG　　　　　　　　　39

(2) INFORMATION FOR SEQ ID NO:54:

    (i) SEQUENCE CHARACTERISTICS:
        (A) LENGTH: 54 base pairs
        (B) TYPE: nucleic acid
        (C) STRANDEDNESS: single
        (D) TOPOLOGY: linear

    (ii) MOLECULE TYPE: cDNA

(xi) SEQUENCE DESCRIPTION: SEQ ID NO:54:

CATTCGCTTA CCCTGCAGAA GCTTGTTGAC TAGTGAGATC ACAGTTCTCT CTAC　　　54

(2) INFORMATION FOR SEQ ID NO:55:

    (i) SEQUENCE CHARACTERISTICS:
        (A) LENGTH: 19 base pairs
        (B) TYPE: nucleic acid
        (C) STRANDEDNESS: single
        (D) TOPOLOGY: linear

    (ii) MOLECULE TYPE: cDNA

(xi) SEQUENCE DESCRIPTION: SEQ ID NO:55:

GGAGTGGACA CCTGTGGAG                                             19

(2) INFORMATION FOR SEQ ID NO:56:

    (i) SEQUENCE CHARACTERISTICS:
        (A) LENGTH: 39 base pairs
        (B) TYPE: nucleic acid
        (C) STRANDEDNESS: single
        (D) TOPOLOGY: linear

    (ii) MOLECULE TYPE: cDNA


    (xi) SEQUENCE DESCRIPTION: SEQ ID NO:56:

CTCCACAGGT GTCCACTCCC AGTCTGTGTT GACGCAGCC                       39

(2) INFORMATION FOR SEQ ID NO:57:

    (i) SEQUENCE CHARACTERISTICS:
        (A) LENGTH: 79 base pairs
        (B) TYPE: nucleic acid
        (C) STRANDEDNESS: single
        (D) TOPOLOGY: linear

    (ii) MOLECULE TYPE: cDNA


    (xi) SEQUENCE DESCRIPTION: SEQ ID NO:57:

GAATGTGCCT ACTTTCTAGA CTCGAGAACT GAGGAAGCAA AGTTTAAATT CTACTCACGA    60

CTTAGGACGG TCAGCTTGG                                             79

(2) INFORMATION FOR SEQ ID NO:58:

    (i) SEQUENCE CHARACTERISTICS:
        (A) LENGTH: 18 base pairs
        (B) TYPE: nucleic acid
        (C) STRANDEDNESS: single
        (D) TOPOLOGY: linear

    (ii) MOLECULE TYPE: cDNA

(xi) SEQUENCE DESCRIPTION: SEQ ID NO:58:

CATTCGCTTA CCCTGCAG                                                          18

(2) INFORMATION FOR SEQ ID NO:59:

    (i) SEQUENCE CHARACTERISTICS:
        (A) LENGTH: 19 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:59:

GAATGTGCCT ACTTTCTAG                                                         19

## Claims

1. A recombinant human anti-HIV antibody or fragment thereof, which is HIV-neutralizing for 2 or more HIV serotype isolates.

2. The recombinant human anti-HIV antibody or fragment thereof according to Claim 1, wherein the antibody or fragment is neutralizing for 2 or more HIV-I isolates selected from the group consisting of IIIB, MN, AL-1, SF-2, WMJ-2, R-, DU 6587-5 and DU 7887-7.

3. A recombinant human anti-HIV antibody or fragment thereof which is neutralizing for HIV-1 isolates IIIB, MN, AL-1, SF-2, WMJ-2, RF, DU 6587-5, and DU 7887-7.

4. The recombinant human anti-HIV antibody or fragment thereof according to Claim 1 or Claim 3, wherein the neutralizing antibody or fragment binds to an HIV-1-neutralizing epitope of gp120.

5. The recombinant human anti-HIV-1 antibody or fragment thereof according to claim 4, wherein the neutralizing epitope of HIV-1 gp120 is within the V3 loop of gp120.

6. The recombinant human anti-HIV-1 antibody or fragment thereof according to Claim 5, wherein the neutralizing epitope of HIV gp120 V3 loop comprises the amino acid sequence GPXR, and X is any amino acid.

7. The recombinant human anti-HIV-1 antibody or fragment thereof according to Claim 6, wherein the neutralizing epitope of HIV gp120 V3 loop comprises the amino acid sequence GPGR.

8. A recombinant human anti-HIV antibody or fragment thereof, wherein the antibody heavy chain class is IgG, and the antibody is neutralizing for HIV-1 isolates IIIB, MN, AL-1, SF-2, WMJ-2, RF, DU 6587-5, and DU 7887-7.

9. The recombinant human anti-HIV antibody or fragment thereof according to Claim 8, wherein the antibody heavy chain subclass is $IgG_3$, and the antibody is neutralizing for HIV-1 serotype isolates IIIB, MN, AL-1, SF-2, WMJ-2, DU6587-5, and DU 7887-7.

10. The recombinant human anti-HIV antibody or fragment thereof according to Claim 9, wherein the heavy chain IgG subclass region is genetically modified to an $IgG_1$ subclass region, and the antibody is neutralizing for HIV-1 serotype isolates IIIB, MN, AL-1, SF-2, WMJ-2, DU6587-5, and DU 7887-7.

11. An expression cassette comprising a promoter sequence, an ORF encoding all or part of the variable domain of an HIV-neutralizing antibody light chain or heavy chain, said antibody being neutralizing for 2 or more HIV serotypes, and a suitable transcription termination sequence.

12. The expression cassette according to Claim 11 wherein the ORF encodes the variable domain of an HIV-1-neutralizing antibody light chain or heavy chain, said antibody being neutralizing for 2 or more HIV-1 serotypes.

13. The expression cassette according to Claim 12 wherein the ORF encodes the variable domain of the antibody 447-52D light chain or heavy chain.

14. An expression cassette comprising at least one promoter sequence, a first ORF encoding the light chain and a second ORF encoding the heavy chain of an HIV-1-neutralizing antibody, and a suitable transcription terminator sequence.

15. The expression cassette according to Claim 14 wherein the first and second ORFs encode the light chain and heavy chain of an HIV-1-neutralizing antibody, said antibody being neutralizing for 2 or more HIV-1 isolates.

16. The expression cassette according to Claim 15 wherein the second ORF encodes an IgG heavy chain.

17. The expression cassette according to Claim 16 wherein the second ORF encodes an IgG1 heavy chain.

18. The expression cassette according to Claim 15 wherein the first and second ORFs encode the light chain and heavy chain of the antibody 447-52D.

19. The expression cassette according to Claim 16 wherein the IgG heavy chain subclass region is genetically modified to an $IgG_1$ subclass region.

20. A host cell containing an expression cassette wherein said expression cassette comprises a promoter sequence, an ORF encoding all or part of the variable domain of an HIV neutralizing antibody light chain or heavy chain, and a suitable transcription termination sequence.

21. A host cell containing two expression cassettes wherein said expression cassettes each comprise a promoter sequence, an ORF encoding all or part of the variable domain of an HIV neutralizing antibody light chain or heavy chain, and a suitable transcription termination sequence.

22. The host cell according to Claim 20 having the American Type Culture Collection Accession Number 68945.

23. The host cell according to Claim 20 having the American Type Culture Collection Accession Number 68943.

24. The host cell according to Claim 21 having the American Type Culture Collection Accession Number 68944.

25. A pharmaceutical composition comprising a recombinant human anti-HIV antibody or fragment thereof as claimed in any of Claims 1 to 3 and a physiologically acceptable diluent or carrier.

26. The use of a recombinant human anti-HIV antibody or fragment thereof as claimed in any of claims 1 to 3 for the manufacture of a medicament for passively protecting a member of an HIV-susceptible speices against HIV infection.

27. A phramaceutical composition comprising a first and second anti-HIV agent, wherein said first agent is a recombinant anti-HIV antibody neutralizing for 2 or more HIV isolates, and said second agent is a non-antibody HIV inhibitor.

28. The pharmaceutical composition according to Claim 27 wherein the non-antibody HIV inhibitor is from a class of HIV inhibitors selected from the group consisting of reverse transcriptase inhibitors, proteinase inhibitors, transcription trans-activation inhibitors, antisense oligonucleotides, and viral attachment inhibitors.

29. The pharmaceutical composition according to Claim 38 wherein the non-antibody HIV inhibitor is a reverse transcriptase inhibitor.

30. The phramceutical composition of Claim 29 wherein the reverse transcriptase inhibitor is selected from the group consisting of:
3-{[(4, 7-dichlorobenzoaxazol-2-yl)methyl]amino-5-ethyl-6-methyl-2-(1H)-pyridinone,
3-{[(4,7-dimethylbenzoxazol-2-yl)methyl]amino}-5-ethyl-6-methyl-2-(1H)-pyridinone,
3-{[(7-chlorobenzoxazol-2-yl)methyl]amino}-5-ethyl-6-methyl-2-(1H)-pyridinone,
3-{[(7-methylbenzoxazol-2-yl)methyl]amnio}-5-ethyl-6-methyl-2-(1H)-pyridinone,
3-{[(4-fluorobenzoxazol-2-yl)methyl]amino}-5-ethyl-6-methyl-2-(1H)-pyridinone,
3-{[(7-fluorobenzoxazol-2-yl)methyl]amino}-5-ethyl-6-methyl-2-(1H)-pyridinone,
3-{[(benzoxazol-2-yl)methyl]amino}5-ethyl-6-methyl-2-(1H)-pyridinone,
3-{[(4-chlorobenzoxazol-2-yl)methyl]amino}-5-ethyl-6-methyl-2-(1H)-pyridinone,
3-{[(4-fluoro-7-chlorobenzoxazol-2-yl)methyl]amino}-5-ethyl-6-methyl-2-(1H)-pyridinone, and
3-[2-(benzoxazol-2-yl)ethyl]-5-ethyl-6-methyl-2-(1H)-pyridinone).

31. A process for the preparation of the recombinant human anti-HIV antibody as claimed in claim 1, which process comprises the steps of:
1) identifying human antibody-producing cells which produce an antibody molecule capable of neutralizing two or more different HIV-1 serotype isolates;
2) isolating the DNA which encodes the light chain and the heavy chain of the antibody molecule;
3) altering the DNA sequence encoding the heavy and/or light chain to a different immunoglobulin subclass, if necessary or desired;
4) inserting the DNA sequences into expression vectors and expressing the recombinant antibody molecules in appropriate host cells; and
5) purifying the recombinant antibody from host cell cultures.

AMPLIFICATION OF HEAVY CHAIN cDNA FROM HETEROHYBRIDOMA 447

```
            EcoRI        PvuII
       5'-CGGAATTC AGGTGCAGCTGCAGCAGTCTGG-3'   SEQ.ID.NO.: 36
            EcoRI    NotI
       5'-CCGAATTC GCGGCCGCACTCATTTACCCGGAGACAGG-3'
                                                  SEQ.ID.NO.: 37
```

AMPLIFICATION OF LIGHT CHAIN cDNA FROM HETEROHYBRIDOMA 447

```
            SalI     NcoI   NaeI
       5'-GGCGTCGAC TCACCATG GCCGGCTCCCCTCTC(C/T)TCCTCACCC-3'
            EcoRI    NotI                    SEQ.ID.NO.: 38
       5'-CCGAATTC GCGGCCGC CTATGAACATTCTGTAGG-3'   SEQ.ID.NO.: 39
```

AMPLIFICATION OF THE LAMBDA CONSTANT REGION

```
            XhoI        XbaI
     C1  5'-GATCCTCGAG GATTCTAGA AGGGTCAGATGTCGGTGTTG-3'
            EcoRI     BamHI               SEQ.ID.NO.: 40
     C2  5'-GATCGAATTC CTGGGATCC TGCAGCTCTAG-3'     SEQ.ID.NO.: 41
```

AMPLIFICATION OF THE HIV-1 LTR FRAGMENT -117 TO +80

```
            XhoI MluI SpeI
       5'-TTACTCGAG ACGCGT ACTAGT GAGCTTGCTACAAGGGACTTTC-3'
            XbaI  HindIII        AflII       SEQ.ID.NO.: 42
       5'-ATTTCTAGA AAGCTT TATTGAGGCTTAAG C-3'     SEQ.ID.NO.: 43
```

AMPLIFICATION OF GAMMA 1 CONSTANT REGION

```
            XhoI
     G1  5'-TATACTCGAG CAGACACTGGACGCTGAACC-3'     SEQ.ID.NO.: 44
            BclI   EcoRI  XmaI
     G2  5'-TATATGATCA GAATTC GCCCGGG AAGTATGTACAG-3'    SEQ.ID.NO.: 45
```

# FIG.1

10                          30                          50

GAGGTGCAGCTGGTGGAGTCTGGGGGAGGCTTGGTAAAGCCTGGGGGGTCCCTCAGACTC

GluValGlnLeuValGluSerGlyGlyGlyLeuValLysProGlyGlySerLeuArgLeu

70                          90                          110

ACCTGTGTAGCCTCTGGTTTCACGTTCAGTGATGTCTGGCTGAACTGGGTCCGCCAGGCT

ThrCysValAlaSerGlyPheThrPheSerAspValTrpLeuAsnTrpValArgGlnAla

130                         150                         170

CCAGGGAAGGGGCTGGAGTGGGTCGGCCGTATTAAAAGCAGAACTGATGGTGGGACAACA

ProGlyLysGlyLeuGluTrpValGlyArgIleLysSerArgThrAspGlyGlyThrThr

190                         210                         230

GACTACGCTGCATCCGTGAAAGGCAGATTCACCATCTCAAGAGATGACTCAAAAAACACG

AspTyrAlaAlaSerValLysGlyArgPheThrIleSerArgAspAspSerLysAsnThr

# FIG. 2A₁

EP 0 577 243 A2

250                           270                           290

CTATATCTGCAAATGAATAGCCTGAAAACAGAGGACACAGCCGTTTATTCCTGCACCACA

LeuTyrLeuGlnMetAsnSerLeuLysThrGluAspThrAlaValTyrSerCysThrThr

310                           330                           350

GATGGTTTTATTATGATTCGGGGAGTCTCCGAGGACTACTACTACTACATGGACGTT

AspGlyPheIleMetIleArgGlyValSerGluAspTyrTyrTyrTyrTyrMetAspVal

370                           390                           410

TGGGGCAAAGGGACCACGGTCACCGTGAGCTCAGCCTCCACCAAGGGCCCATCGGTCTTC

TrpGlyLysGlyThrThrValThrValSerSerAlaSerThrLysGlyProSerValPhe

430                           450                           470

CCCCTGGCACCCTCCTCCAAGAGCACCTCTGGGGGCACAGCGGCCCTGGGCTGCCTGGTC

ProLeuAlaProSerSerLysSerThrSerGlyGlyThrAlaAlaLeuGlyCysLeuVal

# FIG.2A2

490  510  530

AAGGACTACTTCCCCGAACCGGTGACGGTGTCGTGGAACTCAGGCGCCCTGACCAGCGGC

LysAspTyrPheProGluProValThrValSerTrpAsnSerGlyAlaLeuThrSerGly

550  570  590

GTGCACACCTTCCCGGCTGTCCTACAGTCCTCAGGACTCTACTCCCTCAGCAGCGTGGTG

ValHisThrPheProAlaValLeuGlnSerSerGlyLeuTyrSerLeuSerSerValVal

610  630  650

ACCGTGCCCTCCAGCAGCTTGGGCACCCAGACCTACATCTGCAACGTGAATCACAAGCCC

ThrValProSerSerSerLeuGlyThrGlnThrTyrIleCysAsnValAsnHisLysPro

670  690  710

AGCAACACCAAGGTGGACAAGAAAGTTGAGCCCAAATCTTGTGACAAAACTCACACATGC

SerAsnThrLysValAspLysLysValGluProLysSerCysAspLysThrHisThrCys

730  750  770

CCACCGTGCCCAGCACCTGAACTCCTGGGGGGACCGTCAGTCTTCCTCTTCCCCCCAAAA

ProProCysProAlaProGluLeuLeuGlyGlyProSerValPheLeuPheProProLys

FIG.2A3

EP 0 577 243 A2

790                    810                    830

CCCAAGGACACCCTCATGATCTCCCGGACCCCTGAGGTCACATGCGTGGTGGTGGACGTG

ProLysAspThrLeuMetIleSerArgThrProGluValThrCysValValValAspVal

850                    870                    890

AGCCACGAAGACCCTGAGGTCAAGTTCAACTGGTACGTGGACGGCGTGGAGGTGCATAAT

SerHisGluAspProGluValLysPheAsnTrpTyrValAspGlyValGluValHisAsn

910                    930                    950

GCCAAGACAAAGCCGCGGGAGGAGCAGTACAACAGCACGTACCGTGTGGTCAGCGTCCTC

AlaLysThrLysProArgGluGluGlnTyrAsnSerThrTyrArgValValSerValLeu

970                    990                    1010

ACCGTCCTGCACCAGGACTGGCTGAATGGCAAGGAGTACAAGTGCAAGGTCTCCAACAAA

ThrValLeuHisGlnAspTrpLeuAsnGlyLysGluTyrLysCysLysValSerAsnLys

# FIG.2A4

EP 0 577 243 A2

1030                          1050                          1070

GCCCTCCCAGCCCCCATCGAGAAAACCATCTCCAAAGCCAAAGGGCAGCCCCGAGAACCA

AlaLeuProAlaProIleGluLysThrIleSerLysAlaLysGlyGlnProArgGluPro

1090                          1110                          1130

CAGGTGTACACCCTGCCCCCATCCCGGGATGAGCTGACCAAGAACCAGGTCAGCCTGACC

GlnValTyrThrLeuProProSerArgAspGluLeuThrLysAsnGlnValSerLeuThr

1150                          1170                          1190

TGCCTGGTCAAAGGCTTCTATCCCAGCGACATCGCCGTGGAGTGGGAGAGCAATGGGCAG

CysLeuValLysGlyPheTyrProSerAspIleAlaValGluTrpGluSerAsnGlyGln

1210                          1230                          1250

CCGGAGAACAACTACAAGACCACGCCTCCCGTGCTGGACTCCGACGGCTCCTTCTTCCTC

ProGluAsnAsnTyrLysThrThrProProValLeuAspSerAspGlySerPhePheLeu

# FIG.2A5

1270     1290     1310

TACAGCAAGCTCACCGTGGACAAGAGCAGGTGGCAGCAGGGGAACGTCTTCTCATGCTCC
TyrSerLysLeuThrValAspLysSerArgTrpGlnGlnGlyAsnValPheSerCysSer

1330     1350     1370

GTGATGCATGAGGCTCTGCACAACCACTACACGCAGAAGAGCCTCTCCCTGTCTCCGGGT
ValMetHisGluAlaLeuHisAsnHisTyrThrGlnLysSerLeuSerLeuSerProGly

AAATGA
LysEnd

FIG.2A6

EP 0 577 243 A2

10                          30                          50

CAGTCTGTGTTGACGCAGCCGCCCTCAGTGTCTGCGGCCCCAGGACAGAAGGTCACCATC

GlnSerValLeuThrGlnProProSerValSerAlaAlaProGlyGlnLysValThrIle

70                          90                          110

TCCTGCTCTGGAAGCAGCTCCAACATTGGGAATAATTATGTATTGTGGTACCAGCAGTTC

SerCysSerGlySerSerSerAsnIleGlyAsnAsnTyrValLeuTrpTyrGlnGlnPhe

130                         150                         170

CCAGGAACAGCCCCCAAACTCCTCATTTATGGCAATAATAAGCGACCCTCAGGGATTCCT

ProGlyThrAlaProLysLeuLeuIleTyrGlyAsnAsnLysArgProSerGlyIlePro

190                         210                         230

GACCGATTCTCTGGCTCCAAGTCTGGCACGTCAGCCACCCTGGGCATCACCGGACTCCAG

AspArgPheSerGlySerLysSerGlyThrSerAlaThrLeuGlyIleThrGlyLeuGln

FIG.2B1

EP 0 577 243 A2

250           270           290

ACTGGGGACGAGGCCGATTATTTCTGCGCAACATGGGATAGCGGCCTGAGTGCTGATTGG

ThrGlyAspGluAlaAspTyrPheCysAlaThrTrpAspSerGlyLeuSerAlaAspTrp

310           330           350

GTGTTCGGCGGAGGGACCAAGCTGACCGTCCTAAGTCAGCCCAAGGCTGCCCCCTCGGTC

ValPheGlyGlyGlyThrLysLeuThrValLeuSerGlnProLysAlaAlaProSerVal

370           390           410

ACTCTGTTCCCGCCCTCCTCTGAGGAGCTTCAAGCCAACAAGGCCACACTGGTGTGTCTC

ThrLeuPheProProSerSerGluGluLeuGlnAlaAsnLysAlaThrLeuValCysLeu

430           450           470

ATAAGTGACTTCTACCCGGGAGCCGTGACAGTGGCCTGGAAGGCAGATAGCAGCCCCGTC

IleSerAspPheTyrProGlyAlaValThrValAlaTrpLysAlaAspSerSerProVal

## FIG.2B2

490                          510                          530

AAGGCGGGAGTGGAGACCACCACACCCTCCAAACAAAGCAACAACAAGTACGCGGCCAGC

LysAlaGlyValGluThrThrThrProSerLysGlnSerAsnAsnLysTyrAlaAlaSer

550                          570                          590

AGCTATCTGAGCCTGACGCCTGAGCAGTGGAAGTCCCACAGAAGCTACAGCTGCCAGGTC

SerTyrLeuSerLeuThrProGluGlnTrpLysSerHisArgSerTyrSerCysGlnVal

610                          630                          650

ACGCATGAAGGGAGCACCGTGGAGAAGACAGTGGCCCCTACAGAATGTTCATAG

ThrHisGluGlySerThrValGluLysThrValAlaProThrGluCysSerEnd

# FIG. 2B3

VH                 PstI   HindIII   HincII/SpeI

S1   5'CATTCGCTTACCCTGCAG AAGCTT GTTG ACTAGTGAGATCACAGTTCTCTCTAC-3'    SEQ.ID.NO.:48
S2   5'-GGAGTGGACACCTGTGGAG-3'     SEQ.ID.NO.: 49
I1   5'-GGTGAGTCCTTACAACCTC-3'      SEQ.ID.NO.: 50

                    XbaI   XhoI

I2   5'-GAATGTGCCTACTT TCTAGA CTCGAG TATAAATCTCTGGCCATG-3'    SEQ.ID.NO.: 51
H1   5'-CTCCACAGGTGTCCACTCCGAGGTGCAGCTGGTGGAGTC-3'    SEQ.ID.NO.: 52
H2   5'-GAGGTTGTAAGGACTCACCTGAGCTCACGGTGACCGTGG-3'    SEQ.ID.NO.: 53

VL                 PstI   HindIII   HincII/SpeI

S1   5'-CATTCGCTTACCCTGCAG AAGCTT GTTG ACTAGTGAGATCACAGTTCTCTCTAC-3'    SEQ.ID.NO.:54
S2   5'-GGAGTGGACACCTGTGGAG-3'    SEQ.ID.NO.: 55
L1   5'-CTCCACAGGTGTCCACTCCCAGTCTGTGTTGACGCAGCC-3'    SEQ.ID.NO.: 56

                   XbaI   XhoI

L2   5'-GAATGTGCCTACTTTCTAGA CTCGAGAACTGAGGAAGCAAAGTTTAAATTCTACT
            CACGACTTAGGACGGTCAGCTTGG-3'    SEQ.ID.NO.: 57

TERMINAL AMPLIFICATION PRIMERS

A1   5'-CATTCGCTTACCCTGCAG-3'   SEQ.ID.NO.: 58
A2   5'-GAATGTGCCTACTTTCTAG-3'   SEQ.ID.NO.: 59

# FIG.3

EP 0 577 243 A2

FIG.4

EcoRI(11203)
BamHI(1)
SalI(277)

SV pA

HUMAN
C–GAMMA1

Amp R

SVE Pm

XbaI(9108)
XhoI(9102)

SfiI(2577)

447VH

pHIV/447H/Cγ1
(11.5kb)

HindIII(8267)
HIV LT

SpeI(7963)
Mlu(7957)

SVt INS+pA

BglII(7047)

GS MINIGENE

FIG.5

FIG.6

EP 0 577 243 A2

FIG.7

FIG.8